(19)

Europäisches Patentamt
European Patent Office
Office européen des brevets

(11) **EP 4 293 043 A1**

(12) **EUROPEAN PATENT APPLICATION**
published in accordance with Art. 153(4) EPC

(43) Date of publication:
**20.12.2023 Bulletin 2023/51**

(21) Application number: **22752193.7**

(22) Date of filing: **29.01.2022**

(51) International Patent Classification (IPC):
**C07K 14/82** (2006.01)   **C07K 14/725** (2006.01)
**C12N 5/10** (2006.01)   **C12N 15/12** (2006.01)
**A61K 38/17** (2006.01)   **A61K 39/00** (2006.01)
**A61P 35/00** (2006.01)

(52) Cooperative Patent Classification (CPC):
**A61K 38/17; A61K 39/00; A61P 35/00;**
**C07K 14/435; C07K 14/705; C07K 14/82;**
**C12N 5/10**

(86) International application number:
**PCT/CN2022/075005**

(87) International publication number:
**WO 2022/171032 (18.08.2022 Gazette 2022/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **10.02.2021  PCT/CN2021/076432**

(71) Applicant: **Shanghai Genbase Biotechnology Co.,
Ltd.**
**China (Shanghai) Pilot Free Trade Zone
Shanghai 201203 (CN)**

(72) Inventors:
• **MOU, Nan**
  **Shanghai 201203 (CN)**
• **YU, Yue**
  **Shanghai 201203 (CN)**
• **YUAN, Jijun**
  **Shanghai 201203 (CN)**

(74) Representative: **Mewburn Ellis LLP**
**Aurora Building**
**Counterslip**
**Bristol BS1 6BX (GB)**

Remarks:
The complete document including Reference
Table(s) and the Sequence Listing(s) can be
downloaded from the EPO website

(54) **EPITOPE PEPTIDE OF RAS G13D MUTANT AND T CELL RECEPTOR RECOGNIZING RAS G13D MUTANT**

(57)    The present invention provides an epitope peptide of a RAS G13D mutant, an antigen presenting cell expressing the epitope peptide, a tumor vaccine containing the antigen presenting cell, and a use of the tumor vaccine in the prevention or treatment of a tumor having RAS G13D mutation. The present invention also provides a T cell receptor (TCR) specifically recognizing a RAS G13D mutant, a conjugate and a fusion protein containing the TCR, an immune cell expressing the TCR, a T cell drug containing the immune cell, and a use of the T cell drug in the prevention or treatment of a tumor having RAS G13D mutation.

Fig. 1

EP 4 293 043 A1

**Description**

**Technical Field**

The present invention relates to the fields of immunology and tumor therapy. Specifically, the present invention relates to a RAS G13D mutant epitope peptide, an antigen-presenting cell expressing the epitope peptide, a tumor vaccine containing the same, and uses thereof for preventing or treating a tumor with RAS G13D mutation. The present invention also relates to a T cell receptor (TCR) specifically recognizing a RAS G13D mutant, a conjugate and fusion protein comprising the TCR, an immune cell expressing the TCR, and a T cell drug comprising the same, and uses thereof for preventing or treating a tumor with RAS G13D mutation.

**Background Art**

RAS is a proto-oncogene that has GTPase activity and participates in many signaling pathways that regulate cell proliferation, differentiation and apoptosis, such as MAPK, PI3K, and STAT signaling pathways, etc. In 1982, Der CJ et al first confirmed that RAS gene mutation is the driving factor of cancer (Der CJ et. Al, 1982). There are three RAS-encoding genes in human genes, namely HRAS (GeneID: 3265), NRAS (GeneID: 4893) and KRAS (GeneID: 3845). The three RAS genes have a high degree of sequence homology (>90%). About 33% of human tumors carry RAS gene mutations, and RAS gene mutations have become the most frequent proto-oncogene mutations (Karnoub AE, 2008). Among the RAS gene mutations in human tumors, KRAS gene mutation has the highest incidence and accounts for about 22%, NRAS gene mutation accounts for about 8.0%, and HRAS gene mutation accounts for about 3.3%.

KRAS gene mutations have the highest incidence in solid tumors (about 86% of the three RAS mutations), such as colorectal cancer (30% to 50%), pancreatic cancer (about 85%) and non-small cell lung cancer (15% to 25%); more than 97% of KRAS mutations are concentrated in Exon2 and Exon3, in which Exon2 has the highest mutation frequency (e.g., G12C, G12V, G12D, G13D, etc.), G12D and G13D mutations account for about 20% to 30% of colorectal cancer, 60% to 70% of pancreatic cancer and 38% of non-small cell lung cancer. KRAS mutation is the driving gene of tumor drug resistance. For example, as for drugs such as EGFR TKI and EGFR monoclonal antibody drugs (Cetuximab, etc.), it is necessary to detect the KRAS mutation status of tumor patients, and the patients with KRAS mutation have a very low response to EGFR inhibitors, that is about 0~5% (Jackman DM et. Al, 2009). Tumor patients with KRAS mutations have shorter progression-free survival and overall survival than patients with KRAS wild-type tumors; meanwhile, patients with KRAS mutations also have a higher possibility of postoperative recurrence and metastasis.

In recent decades, scientists' studies on the structure and biology of KRAS have shown that GTP binds KRAS protein with an extremely high affinity (pM level), and it is difficult for small molecule inhibitors to inhibit KRAS activity by competing with GTP; other proteins that interact with KRAS in cells are involved in signaling, and small molecular compounds are also difficult to inhibit KRAS downstream signaling through competitive inhibition of protein-protein interactions; at the same time, the structure of KRAS protein shows that it has a relatively smooth structure and lacks a "pocket" for small molecule inhibitors to bind, so that it is extremely difficult to develop a small molecule inhibitor for KRAS protein itself and related proteins thereof. The development of KRAS inhibitors mainly focuses on the interference of KRAS modification and synthetic lethality for the treatment of tumors with KRAS mutant, for example, the development of Farnesyl transferase inhibitors, but all of them ended in failure (Heidi Ledford, 2015). In recent years, the drug development for KRAS mutants has mainly focused on the KRAS G12C mutant. By designing compounds that can irreversibly bind to the cysteine residue in the G12C mutation, the KRAS G12C mutant is locked in an inactive state, thereby inhibiting KRAS mutant G12C activity; currently, no drugs are in development for other KRAS mutants (e.g., G12V and G13D).

Statistics from the World Health Organization (WHO) show that among the cancers with high-frequency RAS mutations in China (e.g., pancreatic cancer, colorectal cancer, lung cancer, endometrial cancer, ovarian cancer, and prostate cancer), the number of annual incidences reaches 1.6 million, in which 449,000 have RAS mutations (including those at sites G12, G13, Q61, etc.). The patients with RAS mutations have higher tumor recurrence, drug resistance, poorer prognosis and shorter overall survival. These patient groups urgently need new treatment methods.

T cell receptor (TCR) recognizes a peptide sequence of viral protein and mutant gene transcription product presented by HLA, and TCR can specifically recognize a mutated peptide, so KRAS gene mutation is an ideal TCR target. T cell receptors are generated from VDJ gene rearrangement, and the naturally occurring T cell receptor repertoire capacity is about $10^{16-10}$ (Harlan S. Robins, 2009); the T cell receptor repertoire capacity is about 1000 to 10,000 times that of B cell receptor, such a huge repertoire capacity corresponds to the human leukocyte antigen system. HLA is divided into types I (A, B, C, etc.) and II (DP, DR, DQ, etc.), respectively presenting peptides of different lengths (8~16 mer). The discovery of anti-KRAS mutant TCR was mainly carried out by the National Cancer Research Center of the United States. The currently discovered RAS mutant TCRs are of HLA-A*11:01-restricted recognition for KRAS G12V and HLA-C*08:02-restricted recognition for KRAS G12D, and there is no TCR for KRAS G13D; and because the HLA allele is half-inherited, with strong regional genetic distribution. Therefore, it is urgent to discover T cell receptors that recognize KRAS G13D and cover patient populations in more regions.

## Contents of the present invention

The present invention provides an epitope peptide of RAS G13D mutant and a T cell receptor (TCR) that specifically recognizes the epitope peptide, a cell and pharmaceutical composition comprising the epitope peptide or TCR, and a nucleic acid encoding the epitope peptide or TCR, a vector and host cell for preparing the epitope peptide or TCR, and a method for treating a subject with the epitope peptide or TCR. The epitope peptide and TCR provided by the present invention can be used to induce an immune response against a tumor comprising a RAS G13D mutation and thus treat the above-mentioned tumor in a subject. In addition, the epitope peptide and TCR provided by the present invention are of MHC-II restriction, and the MHC-II restriction is an allele showing predominantly high frequency in the Asia-Pacific populations, so it is suitable for patients in the Asia-Pacific region. In addition, the MHC-II restriction is also widely distributed in European, American, and Oceanian populations, so it has broad application prospects.

### Epitope peptide

Therefore, in a first aspect, the present invention provides an isolated epitope peptide or variant thereof, the epitope peptide consisting of 11-30 (e.g., 30, 29, 28, 27, 26, 25, 24, 23, 22, 21, 20, 19, 18, 17, 16, 15, 14, 13, 12, or 11) contiguous amino acid residues of a RAS G13D mutant, and containing the amino acid residues at positions 7 to 17 of the RAS G13D mutant;

the variant differs from the epitope peptide from which it is derived only by substitution of one or several (e.g., 1, 2 or 3) amino acid residues, and does not comprise amino acid substitution at positions corresponding to the amino acid positions 8, 10, 13, 14 and 16 of the RAS G13D mutant, and retains a biological function of the epitope peptide from which it is derived. In certain embodiments, the variant differs from the epitope peptide from which it is derived only by substitution of one or several (e.g., 1, 2 or 3) amino acid residues, and does not comprise amino acid substitution at positions corresponding to the amino acid positions 8, 9, 10, 11, 13, 14 and 16 of the RAS G13D mutant, and retains a biological function of the epitope peptide from which it is derived. The biological function comprises an ability to be presented by a MHC-II molecule and subsequently recognized by a T cell, for example, recognized by an antigen-specific T cell receptor on the T cell.

In certain embodiments, the epitope peptide or variant thereof of the present invention is a MHC-II restricted antigen, that is, the epitope peptide or variant thereof of the present invention can exhibit or present or form a complex with the background of an MHC-II molecule expressed on the surface of a cell. In certain embodiments, the epitope peptide or variant thereof of the present invention is capable of being presented by an MHC-II molecule, and the epitope peptide or variant thereof associated with an MHC-II molecule is capable of being recognized by a T cell, for example recognized by an antigen-specific T cell receptor on the T cell.

In certain embodiments, the MHC-II molecule is HLA-DQ.

In certain embodiments, the HLA-DQ comprises one selected from the group consisting of HLA-DQB1*0301, HLA-DQB1*0303, HLA-DQB1*0319, HLA-DQB1*0201, HLA-DQB1*0603, HLA-DQB1*0604, and HLA-DQB1*0302. Preferably, the HLA-DQ comprises one selected from the group consisting of HLA-DQB1*0301, HLA-DQB1*0303, and HLA-DQB1*0319. More preferably, the HLA-DQ comprises HLA-DQB1*0301.

In some embodiments, the HLA-DQ comprises one selected from the group consisting of HLA-DQA1*0501, HLA-DQA1*0505, HLA-DQA1*0102, HLA-DQA1*0103, and HLA-DQA1*0301. Preferably, the HLA-DQ comprises one selected from the group consisting of HLA-DQA1*0501 and HLA-DQA1*0505.

In certain embodiments, the HLA-DQ comprises one selected from the group consisting of HLA-DQB1*0301, HLA-DQB1*0319 or HLA-DQB1*0303 (preferably HLA-DQB1*0301), and further comprises one selected from the group consisting of HLA-DQA1*0501 or DQA1*0505.

In certain embodiments, the HLA-DQ comprises HLA-DQB1*0301 and HLA-DQA1*0501. In certain embodiments, the HLA-DQ comprises HLA-DQB1*0301 and HLA-DQA1*0505. In certain embodiments, the HLA-DQ comprises HLA-DQB1*0303 and HLA-DQA1*0501. In certain embodiments, the HLA-DQ comprises HLA-DQB1*0303 and HLA-DQA1*0505. In certain embodiments, the HLA-DQ comprises HLA-DQB1*0319 and HLA-DQA1*0501. In certain embodiments, the HLA-DQ comprises HLA-DQB1*0319 and HLA-DQA1*0505.

In certain embodiments, the epitope peptide consists of 11-25 (e.g., 11-20, 11-19, or 11-16) contiguous amino acid residues of the RAS G13D mutant.

In certain embodiments, the RAS G13D mutant is a KRAS G13D mutant. In certain embodiments, the RAS G13D mutant has a sequence set forth in SEQ ID NO: 51 or has a sequence identity of at least 80%, at least 85%, at least 90%, at least 91%, at least 92%, at least 93%, at least 94%, at least 95%, at least 96%, at least 97%, at least 98%, at least 99%, or 100% as compared thereto.

In certain embodiments, the amino acid residues at positions 7-17 of the RAS G13D mutant have a sequence set forth in SEQ ID NO: 14.

In some embodiments, the epitope peptide comprises or consists of the amino acid residues at positions 7-17 of the RAS G13D mutant (e.g., as set forth in SEQ ID NO: 14). In certain embodiments, the epitope peptide comprises or consists of the amino acid residues at positions 5-23 of the RAS G13D mutant (e.g., as set forth in SEQ ID NO: 15). In certain embodiments, the epitope peptide comprises or consists of the amino acid residues at positions 5-22 of the RAS G13D mutant (e.g., as set forth in SEQ ID NO: 16). In certain embodiments, the epitope peptide comprises or consists of the amino acid residues at positions 5-21 of the RAS G13D mutant (e.g., as set forth in SEQ ID NO: 17). In certain embodiments, the epitope peptide comprises or consists of the amino acid residues at positions 4-20 of the RAS G13D mutant (e.g., as set forth in SEQ ID NO: 18). In some embodiments, the epitope peptide comprises or consists of the amino acid residues at positions 5-20 of the RAS G13D mutant (e.g., as set forth in SEQ ID NO: 19). In certain embodiments, the epitope peptide comprises or consists of the amino acid residues at positions 4-19 of the RAS G13D mutant (e.g., as set forth in SEQ ID NO: 20). In some embodiments, the epitope peptide comprises or consists of the amino acid residues at positions 5-19 of the RAS G13D mutant (e.g., as set forth in SEQ ID NO: 21). In some embodiments, the epitope peptide comprises or consists of the amino acid residues at positions 6-19 of the RAS G13D mutant (e.g., as set forth in SEQ ID NO: 22). In certain embodiments, the epitope peptide comprises or consists of the amino acid residues at positions 7-19 of the RAS G13D mutant (e.g., as set forth in SEQ ID NO: 23). In certain embodiments, the epitope peptide comprises or consists of the amino acid residues at positions 5-18 of the RAS G13D mutant (e.g., as set forth in SEQ ID NO: 26). In certain embodiments, the epitope peptide comprises or consists of the amino acid residues at positions 5-17 of the RAS G13D mutant (e.g., as set forth in SEQ ID NO: 27).

In certain embodiments, the epitope peptide consists of the amino acid residues at positions 4-19 of the RAS G13D mutant (e.g., as set forth in SEQ ID NO: 20).

In some embodiments, the epitope peptide comprises or consists of the sequence set forth in any one of SEQ ID NOs: 14-23, 26-27. In certain embodiments, the variant comprises or consists of a sequence selected from the group consisting of: (i) a sequence having a substitution, deletion or addition of one or several amino acids (e.g., a substitution, deletion or addition of 1, 2, 3, 4 or 5 amino acids) as compared to the sequence set forth in any one of SEQ ID NOs: 14-23, 26-27; (ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in any one of SEQ ID NOs: 14-23, 26-27.

In another aspect, the present invention provides an MHC-peptide complex, which comprises the epitope peptide or variant thereof of the present invention and an MHC-II molecule bound thereto. In certain embodiments, the MHC-II molecule is HLA-DQ.

In certain embodiments, the HLA-DQ comprises one selected from the group consisting of HLA-DQB1*0301, HLA-DQB1*0303, HLA-DQB1*0319, HLA-DQB1*0201, HLA-DQB 1 *0603, HLA-DQB1 *0604, and HLA-DQB1 *0302. Preferably, the HLA-DQ comprises one selected from the group consisting of HLA-DQB1*0301, HLA-DQB1*0303, and HLA-DQB1*0319. More preferably, the HLA-DQ comprises HLA-DQB1*0301.

In some embodiments, the HLA-DQ comprises one selected from the group consisting of HLA-DQA1*0501, HLA-DQA1*0505, HLA-DQA1*0102, HLA-DQA1*0103, and HLA-DQA1*0301. Preferably, the HLA-DQ comprises one selected from the group consisting of HLA-DQA1*0501 and HLA-DQA1*0505.

In certain embodiments, the HLA-DQ comprises one selected from the group consisting of HLA-DQB1*0301, HLA-DQB1*0319 or HLA-DQB1*0303 (preferably HLA-DQB1*0301), and further comprises one selected from the group consisting of HLA-DQA1*0501 or DQA1 *0505.

In certain embodiments, the HLA-DQ comprises HLA-DQB1*0301 and HLA-DQA1*0501. In certain embodiments, the HLA-DQ comprises HLA-DQB1*0301 and HLA-DQA1*0505. In certain embodiments, the HLA-DQ comprises HLA-DQB1*0303 and HLA-DQA1*0501. In certain embodiments, the HLA-DQ comprises HLA-DQB1*0303 and HLA-DQA1*0505. In certain embodiments, the HLA-DQ comprises HLA-DQB1*0319 and HLA-DQA1*0501. In certain embodiments, the HLA-DQ comprises HLA-DQB1*0319 and HLA-DQA1*0505.

In certain embodiments, the MHC-peptide complex is present on a surface of a cell. Thus, the present invention also encompasses a cell expressing the MHC-peptide complex.

T cell receptor

In a second aspect, the present invention provides an isolated T cell receptor or antigen-binding fragment thereof capable of specifically recognizing the epitope peptide or variant thereof or the MHC-peptide complex of the present invention. In certain embodiments, the epitope peptide or variant thereof is presented by an MHC-II molecule. In certain embodiments, the MHC-II molecule is HLA-DQ.

In certain embodiments, the HLA-DQ comprises one selected from the group consisting of HLA-DQB1*0301, HLA-DQB1*0303, HLA-DQB1*0319, HLA-DQB1*0201, HLA-DQB 1 *0603, HLA-DQB 1 *0604, and HLA-DQB 1 *0302.

Preferably, the HLA-DQ comprises one selected from the group consisting of HLA-DQB1*0301, HLA-DQB1*0303, and HLA-DQB1*0319. More preferably, the HLA-DQ comprises HLA-DQB1*0301.

In some embodiments, the HLA-DQ comprises one selected from the group consisting of HLA-DQA1*0501, HLA-DQA1*0505, HLA-DQA1*0102, HLA-DQA1*0103, and HLA-DQA1*0301. Preferably, the HLA-DQ comprises one selected from the group consisting ofHLA-DQA1*0501 and HLA-DQA1*0505.

In certain embodiments, the HLA-DQ comprises one selected from the group consisting of HLA-DQB1 *0301, HLA-DQB1*0319 or HLA-DQB1 *0303 (preferably HLA-DQB1 *0301), and further comprises one selected from the group consisting of HLA-DQA1*0501 or DQA1 *0505.

In certain embodiments, the HLA-DQ comprises HLA-DQB1*0301 and HLA-DQA1*0501. In certain embodiments, the HLA-DQ comprises HLA-DQB1*0301 and HLA-DQA1*0505. In certain embodiments, the HLA-DQ comprises HLA-DQB1*0303 and HLA-DQA1*0501. In certain embodiments, the HLA-DQ comprises HLA-DQB1*0303 and HLA-DQA1*0505. In certain embodiments, the HLA-DQ comprises HLA-DQB1*0319 and HLA-DQA1*0501. In certain embodiments, the HLA-DQ comprises HLA-DQB1*0319 and HLA-DQA1*0505.

In a third aspect, the present invention provides an isolated T cell receptor or antigen-binding fragment thereof capable of specifically recognizing a RAS G13D mutant, the TCR or antigen-binding fragment thereof comprising an $\alpha$ chain variable region (V$\alpha$) and/or a $\beta$ chain variable region (V$\beta$), wherein,

> (a) the V$\alpha$ comprises CDR1$\alpha$, CDR2$\alpha$ and CDR3$\alpha$, wherein the CDR3$\alpha$ has a sequence set forth in SEQ ID NO: 8 or a sequence having a substitution, deletion or addition of one or several amino acids (e.g., a substitution, deletion or addition of 1, 2 or 3 amino acids) compared thereto; and/or,

> (b) the V$\beta$ comprises CDR1$\beta$, CDR2$\beta$ and CDR3$\beta$, wherein the CDR3$\beta$ has a sequence set forth in any one of SEQ ID NOs: 11, 54-91 or a sequence having a substitution, deletion or addition of one or several amino acids (e.g., a substitution, deletion or addition of 1, 2 or 3 amino acids) compared thereto.

In certain embodiments, the CDR3$\alpha$ does not comprise an amino acid substitution and deletion at amino acid position corresponding to the amino acid position 5 of SEQ ID NO:8.

In some embodiments, the CDR3$\beta$ has a sequence as set forth in ASSX$_1$X$_2$X$_3$X$_4$PQH (SEQ ID NO: 92); wherein, X$_1$ is selected from the group consisting of Q, A, C, D, E, G, H, I, L, M, N, S, T, V, W or Y; X$_2$ is selected from the group consisting of T, A, C, H, K, N, S, V or W; X$_3$ is selected from the group consisting of V, I, S or T; X$_4$ is selected from the group consisting of P, C, D, E, F, G, H, L, M, R, S, V or W. In certain embodiments, X$_1$ is selected from the group consisting of Q, A, C, E, G, M, W or Y; X$_2$ is selected from the group consisting of T, H, K, N, S or V; X$_3$ is selected from the group consisting of V or T; X$_4$ is selected from the group consisting of P, C, D, E, F, M, S or W.

In certain embodiments, the CDR3$\beta$ has a sequence set forth in any one of SEQ ID NOs: 11, 54-91. In some embodiments, the CDR3$\beta$ has a sequence set forth in any one of SEQ ID NOs: 11, 54-55, 57-58, 62, 67-68, 71-75, 79-83, 87, 89, 91.

In some preferred embodiments, the CDR1$\alpha$ has a sequence set forth in SEQ ID NO: 6 or a sequence having a substitution, deletion or addition of one or several amino acids (e.g., a substitution, deletion or addition of 1, 2 or 3 amino acids) as compared thereto.

In some preferred embodiments, the CDR2$\alpha$ has a sequence set forth in SEQ ID NO: 7 or a sequence having a substitution, deletion or addition of one or several amino acids (e.g., a substitution, deletion or addition of 1, 2 or 3 amino acids) as compared thereto.

In certain preferred embodiments, the CDR1$\beta$ has a sequence set forth in SEQ ID NO: 9 or a sequence having a substitution, deletion or addition of one or several amino acids (e.g., a substitution, deletion or addition of 1, 2 or 3 amino acids) as compared thereto.

In some preferred embodiments, the CDR2$\beta$ has a sequence set forth in SEQ ID NO: 10 or a sequence having a substitution, deletion or addition of one or several amino acids (e.g., a substitution, deletion or addition of 1, 2 or 3 amino acids) as compared thereto.

In certain embodiments, the TCR or antigen-binding fragment thereof comprises an $\alpha$ chain variable region (V$\alpha$) and/or a $\beta$ chain variable region (V$\beta$), wherein,

> (a) the V$\alpha$ comprises the following three complementarity determining regions (CDRs):

>> (i) CDR1$\alpha$, which has a sequence set forth in SEQ ID NO: 6 or a sequence having a substitution, deletion or addition of one or several amino acids (e.g., a substitution, deletion or addition of 1, 2 or 3 amino acids) as compared thereto,

>> (ii) CDR2$\alpha$, which has a sequence set forth in SEQ ID NO: 7 or a sequence having a substitution, deletion or addition of one or several amino acids (e.g., a substitution, deletion or addition of 1, 2 or 3 amino acids) as compared thereto,

(iii) CDR3α, which has a sequence set forth in SEQ ID NO: 8 or a sequence having a substitution, deletion or addition of one or several amino acids (e.g., a substitution, deletion or addition of 1, 2 or 3 amino acids) as compared thereto;

and/or,

(b) the Vβ comprises the following three complementarity determining regions (CDRs):

(iv) CDR1β, which has a sequence set forth in SEQ ID NO: 9 or a sequence having a substitution, deletion or addition of one or several amino acids (e.g., a substitution, deletion or addition of 1, 2 or 3 amino acids) as compared thereto,

(v) CDR2β, which has a sequence set forth in SEQ ID NO: 10 or a sequence having a substitution, deletion or addition of one or several amino acids (e.g., a substitution, deletion or addition of 1, 2 or 3 amino acids) as compared thereto, and

(vi) CDR3β, which has a sequence set forth in any one of SEQ ID NOs: 11, 54-91 or a sequence having a substitution, deletion or addition of one or several amino acids (e.g., a substitution, deletion or addition of 1, 2 or 3 amino acids) as compared thereto.

In certain embodiments, the CDR3α does not comprise an amino acid substitution and deletion at amino acid position corresponding to amino acid position 5 of SEQ ID NO:8.

In some embodiments, the CDR3β has a sequence set forth in $ASSX_1X_2X_3X_4PQH$ (SEQ ID NO: 92); wherein, $X_1$ is selected from the group consisting of Q, A, C, D, E, G, H, I, L, M, N, S, T, V, W or Y; $X_2$ is selected from the group consisting of T, A, C, H, K, N, S, V or W; $X_3$ is selected from the group consisting of V, I, S or T; $X_4$ is selected from the group consisting of P, C, D, E, F, G, H, L, M, R, S, V or W. In certain embodiments, $X_1$ is selected from the group consisting of Q, A, C, E, G, M, W or Y; $X_2$ is selected from the group consisting of T, H, K, N, S or V; $X_3$ is selected from the group consisting of V or T; $X_4$ is selected from the group consisting of P, C, D, E, F, M, S or W.

In certain embodiments, the CDR3β has a sequence set forth in any one of SEQ ID NOs: 11, 54-91. In some embodiments, the CDR3β has a sequence set forth in any one of SEQ ID NOs: 11, 54-55, 57-58, 62, 67-68, 71-75, 79-83, 87, 89, 91.

In certain embodiments, the substitution described in any of the above embodiments is a conservative substitution.

In certain embodiments, the CDR described in any of the above embodiments is defined according to the IMGT numbering system.

The TCR of the second or third aspect may be used in any TCR structure.

In certain embodiments, the TCR may be a full length TCR comprising a full length α chain and a full length β chain.

In certain embodiments, the TCR is a soluble TCR lacking one or more transmembrane and/or cytoplasmic regions. In certain embodiments, the soluble TCR is produced by fusing the extracellular domain of the TCR of the present invention to other protein domains (e.g., maltose-binding protein, thioredoxin, human constant κ domain, or leucine zipper), see, for example, et al., Front Oncol., 2014;4:378, which is hereby incorporated by reference in its entirety.

In certain embodiments, the TCR of the present invention may also be a single-chain TCR (scTCR) comprising Vα and Vβ linked by a peptide linker. Such scTCR may comprise Vα and Vβ, each of the Vα and Vβ is linked to a TCR constant region. Alternatively, the scTCR may comprise Vα and Vβ, wherein Vα, Vβ, or both Vα and Vβ are not linked to a TCR constant region. Exemplary scTCRs are described in PCT Publication Nos. WO 2003/020763, WO 2004/033685, and WO 2011/044186, the disclosures of each of which are incorporated herein by reference in their entirety.

In certain embodiments, the TCR of the present invention may comprise two polypeptide chains (e.g., an α chain and a β chain), wherein each of the chains has been engineered to have a cysteine residue that can form an interchain disulfide bond. Thus, the TCR of the present invention may comprise two polypeptide chains linked by an engineered disulfide bond. Exemplary TCRs with engineered disulfide bonds are described in US Patent Nos. 8,361,794 and 8,906,383, each of which is incorporated herein by reference in its entirety.

In certain embodiments, the T cell receptor of the second or third aspect is a membrane-bound or soluble T cell receptor. In certain embodiments, the T cell receptor of the second or third aspect is a full-length TCR, a soluble TCR or a single-chain TCR.

In certain embodiments, the TCR or antigen-binding fragment thereof comprises an α chain variable region (Vα) and/or a β chain variable region (Vβ), the Vα comprises a CDR3α as set forth in SEQ ID NO: 8, and the Vβ comprises a CDR3β as set forth in ASSX$_1$X$_2$X$_3$X$_4$PQH (SEQ ID NO: 92), wherein, X$_1$ is selected from the group consisting of Q, A, C, D, E, G, H, I, L, M, N, S, T, V, W or Y; X$_2$ is selected from the group consisting of T, A, C, H, K, N, S, V or W; X$_3$ is selected from the group consisting of V, I, S or T; X$_4$ is selected from the group consisting of P, C, D, E, F, G, H, L, M, R, S, V or W. In certain embodiments, X$_1$ is selected from the group consisting of Q, A, C, E, G, M, W or Y; X$_2$ is selected from the group consisting of T, H, K, N, S or V; X$_3$ is selected from the group consisting of V or T; X$_4$ is selected from the group consisting of P, C, D, E, F, M, S or W. In certain embodiments, the TCR or antigen-binding fragment thereof comprises an α chain variable region (Vα) and/or a β chain variable region (Vβ), the Vα comprising CDR3α as set forth in SEQ ID NO: 8, the Vβ comprises CDR3β as set forth in any one of SEQ ID NOs: 11, 54-92.

In some embodiments, the Vα comprises CDR1α, CDR2α and CDR3α as set forth in SEQ ID NOs: 6-8, respectively, and the Vβ comprises CDR1β as set forth in SEQ ID NO: 9, CDR2β as set forth in SEQ ID NO: 10, and CDR3β as set forth in ASSX$_1$X$_2$X$_3$X$_4$PQH (SEQ ID NO: 92), wherein X$_1$ is selected from the group consisting of Q, A, C, D, E, G, H, I, L, M, N, S, T, V, W or Y; X$_2$ is selected from the group consisting of T, A, C, H, K, N, S, V or W; X$_3$ is selected from the group consisting of V, I, S or T; X$_4$ is selected from the group consisting of P, C, D, E, F, G, H, L, M, R, S, V or W. In certain embodiments, X$_1$ is selected from the group consisting of Q, A, C, E, G, M, W or Y; X$_2$ is selected from the group consisting of T, H, K, N, S or V; X$_3$ is selected from the group consisting of V or T; X$_4$ is selected from the group consisting of P, C, D, E, F, M, S or W.

In some embodiments, the Vα comprises CDR1α, CDR2α and CDR3α as set forth in SEQ ID NOs: 6-8, respectively, and the Vβ comprises CDR1β as set forth in SEQ ID NO: 9, CDR2β as set forth in SEQ ID NO: 10, and CDR3β as set forth in any one of SEQ ID NOs: 11, 54-92. In some embodiments, the Vα comprises CDR1α, CDR2α and CDR3α as set forth in SEQ ID NOs: 6-8, respectively, and the Vβ comprises CDR1β as set forth in SEQ ID NO: 9, CDR2β as set forth in SEQ ID NO: 10, and CDR3β as set forth in any one of SEQ ID NOs: 11, 54-55, 57-58, 62, 67-68, 71-75, 79-83, 87, 89, 91.

In certain embodiments, the Vα and/or Vβ in any one of the above embodiments has the following characteristics:

(a) the Vα also comprises FR1α, FR2α, FR3α and FR4α, wherein:

the FR1α has a sequence set forth in SEQ ID NO: 93 or a sequence having a substitution, deletion or addition of one or several amino acids (e.g., a substitution, deletion or addition of 1, 2 or 3 amino acids) as compared thereto;

the FR2α has a sequence set forth in SEQ ID NO: 94 or a sequence having a substitution, deletion or addition of one or several amino acids (e.g., a substitution, deletion or addition of 1, 2 or 3 amino acids) as compared thereto;

the FR3α has a sequence set forth in SEQ ID NO: 95 or a sequence having a substitution, deletion or addition of one or several amino acids (e.g., a substitution, deletion or addition of 1, 2 or 3 amino acids) as compared thereto;

the FR4α has a sequence set forth in SEQ ID NO: 96 or a sequence having a substitution, deletion or addition of one or several amino acids (e.g., a substitution, deletion or addition of 1, 2 or 3 amino acids) as compared thereto;
and/or,

(b) the Vβ also comprises FR1β, FR2β, FR3β and FR4β, wherein:

the FR1β has a sequence set forth in SEQ ID NO: 97 or a sequence having a substitution, deletion or addition of one or several amino acids (e.g., a substitution, deletion or addition of 1, 2 or 3 amino acids) as compared thereto;

the FR2β has a sequence set forth in SEQ ID NO: 98 or a sequence having a substitution, deletion or addition of one or several amino acids (e.g., a substitution, deletion or addition of 1, 2 or 3 amino acids) as compared thereto;

the FR3β has a sequence set forth in SEQ ID NO: 99 or a sequence having a substitution, deletion or addition of one or several amino acids (e.g., a substitution, deletion or addition of 1, 2 or 3 amino acids) as compared thereto;

the FR4β has a sequence set forth in SEQ ID NO: 100 or a sequence having a substitution, deletion or addition of one or several amino acids (e.g., a substitution, deletion or addition of 1, 2 or 3 amino acids) as compared thereto.

In certain embodiments, the substitution is a conservative substitution.

In certain embodiments, the Vα of the TCR or antigen-binding fragment thereof comprises a sequence set forth in SEQ ID NO: 4 or variant thereof, wherein the variant is selected from the group consisting of the following amino acid sequences:

(i) a sequence having a substitution, deletion or addition of one or several amino acids (e.g., a substitution, deletion or addition of 1, 2, 3, 4 or 5 amino acids) as compared to the sequence set forth in SEQ ID NO: 4; or

(ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in SEQ ID NO: 4.

In certain embodiments, the substitution described in (i) is a conservative substitution.

In certain embodiments, the Vα of the TCR or antigen-binding fragment thereof comprises a sequence set forth in SEQ ID NO: 4 or variant thereof, wherein the variant does not comprise an amino acid substitution or deletion at position 97, and the amino acid position is determined according to the IMGT TCR numbering system.

In certain embodiments, the Vβ of the TCR or antigen-binding fragment thereof comprises a sequence set forth in SEQ ID NO: 5 or variant thereof, wherein the variant is selected from the group consisting of the following amino acid sequences:

(i) a sequence having a substitution, deletion or addition of one or several amino acids (e.g., a substitution, deletion or addition of 1, 2, 3, 4 or 5 amino acids) as compared to the sequence set forth in SEQ ID NO: 5; or
(ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in SEQ ID NO: 5.

In certain embodiments, the substitution described in (i) is a conservative substitution.

In certain embodiments, the Vβ of the TCR or antigen-binding fragment thereof comprises a sequence set forth in SEQ ID NO: 5 or variant thereof, wherein the variant comprises one or more (e.g., 1, 2, 3 or 4) amino acid substitutions selected from the group consisting of the following, and the amino acid positions are determined according to the IMGT TCR numbering system: (1) substitution of amino acid at position 95 with A, C, D, E, G, H, I, L, M, N, S, T, V, W or Y; (2) substitution of amino acid at position 96 with A, C, H, K, N, S, V or W; (3) substitution of amino acid at position 97 with I, S or T; (4) substitution of amino acid at position 98 with C, D, E, F, G, H, L, M, R, S, V or W.

In certain embodiments, the variant comprises one or more (e.g., 1, 2, 3 or 4) amino acid substitutions selected from the group consisting of the following, and the amino acid positions are determined according to the IMGT TCR numbering system: (1) substitution of amino acid at position 95 with A, C, E, G, M, W or Y; (2) substitution of amino acid at position 96 with H, K, N, S or V; (3) substitution of amino acid at position 97 with T; (4) substitution of amino acid at position 98 with C, D, E, F, M, S or W.

In certain embodiments, the TCR or antigen-binding fragment thereof of the present invention comprises Vα represented by SEQ ID NO: 4 and/or Vβ represented by SEQ ID NO: 5.

In some embodiments, the TCR or antigen-binding fragment thereof can specifically recognize the epitope peptide or variant thereof (e.g., the sequence set forth in any one of SEQ ID NOs: 14-23, 26-27) or MHC-peptide complex of the present invention. In certain embodiments, the epitope peptide or variant thereof is presented by MHC-II molecules.

In certain embodiments, the MHC-II molecule is HLA-DQ.

In certain embodiments, the HLA-DQ comprises one selected from the group consisting of HLA-DQB1*0301, HLA-DQB1*0303, HLA-DQB1*0319, HLA-DQB1*0201, HLA-DQB 1 *0603, HLA-DQB 1 *0604, and HLA-DQB 1 *0302. Preferably, the HLA-DQ comprises one selected from the group consisting of HLA-DQB1*0301, HLA-DQB1*0303, and HLA-DQB1*0319. More preferably, the HLA-DQ comprises HLA-DQB1*0301.

In some embodiments, the HLA-DQ comprises one selected from the group consisting of HLA-DQA1*0501, HLA-DQA1*0505, HLA-DQA1*0102, HLA-DQA1*0103, and HLA-DQA1*0301. Preferably, the HLA-DQ comprises one selected from the group consisting of HLA-DQA1*0501 and HLA-DQA1*0505.

In certain embodiments, the HLA-DQ comprises one selected from the group consisting of HLA-DQB1*0301, HLA-DQB1*0319 or HLA-DQB1*0303 (preferably HLA-DQB1*0301), and further comprises one selected from the group consisting of HLA-DQA1*0501 or DQA1 *0505.

In certain embodiments, the HLA-DQ comprises HLA-DQB1*0301 and HLA-DQA1*0501. In certain embodiments, the HLA-DQ comprises HLA-DQB1*0301 and HLA-DQA1*0505. In certain embodiments, the HLA-DQ comprises HLA-DQB1*0303 and HLA-DQA1*0501. In certain embodiments, the HLA-DQ comprises HLA-DQB1*0303 and HLA-DQA1*0505. In certain embodiments, the HLA-DQ comprises HLA-DQB1*0319 and HLA-DQA1*0501. In certain embodiments, the HLA-DQ comprises HLA-DQB1*0319 and HLA-DQA1*0505.

In certain embodiments, a T cell expressing on its surface the TCR or antigen-binding fragment thereof is activated under co-cultivation with a second cell displaying (e.g., in the context of MHC-II) the epitope peptide or variant thereof of the present invention. In certain embodiments, the activation of the T cell can be measured using any suitable indicator known in the art. Non-limiting examples of such suitable indicator include: increased secretion level of cytokine (e.g., IL-2, IFN-$\gamma$, etc.), increased proliferative activity, and/or increased expression level of activation marker (e.g., CD25, CD69, CD107a, etc.). In certain embodiments, the activation of the T cell also comprises the apoptosis or death of the second cell displaying (e.g., in the context of MHC-II) the epitope peptide or variant thereof of the present invention induced with the T cell.

## Conjugate and fusion protein

In a fourth aspect, the present invention provides a conjugate comprising the TCR or antigen-binding fragment thereof described in the second or third aspect and an effector moiety conjugated thereto.

In this context, the term "effector moiety" refers to a component or functional group that is capable of modulating (e.g., increasing or decreasing) a natural activity of a molecule linked thereto or conferring a novel activity on the molecule. In some embodiments, the effector moiety is a compound that has an effect on a cell targeted by the TCR.

In this context, the term "conjugation" refers to any method known in the art for functionally linking a protein domain, including but not limited to: recombinant fusion with or without a linker, intein-mediated fusion, non-covalent bonding and covalent bonding, such as disulfide bonding, peptide bonding, hydrogen bonding, electrostatic bonding, and conformational bonding, such as biotin-avidin bonding. In certain embodiments, the conjugation can be performed by chemical or recombinant manner, and the chemical manner comprises forming a covalent bond between two molecules to form one molecule.

In certain embodiments, the effector moiety may be a therapeutic moiety. The therapeutic moiety refers to a compound that can be used as a therapeutic agent. The conjugate takes advantage of the targeting property of the TCR to allow the therapeutic moiety to exert a therapeutic effect on a cell targeted by the TCR.

In certain embodiments, the therapeutic moiety is selected from the group consisting of immunopotentiators, such as immunostimulatory cytokines or immunostimulatory antibodies. In certain exemplary embodiments, the immunostimulatory cytokine is selected from, for example, IL-2, IL-3, IL-12, IL-15, IL-18, IFN-$\gamma$, IL-10, TGF-$\beta$, GM-CSF, or any combination thereof. The various cytokines listed refer to polypeptides with the natural biological activity of the cytokines, including, for example, full-length proteins, active fragments or mutants thereof. For example, IL-2 refers to a polypeptide having IL-2 activity, which may be a full-length IL-2, an active fragment or mutant of IL-2. In certain exemplary embodiments, the immunostimulatory antibody is selected from, for example, anti-CD3 antibody, anti-CD28 antibody, anti-CD40L (CD154) antibody, anti-41BB (CD137) antibody, anti-OX40 antibody, anti-GITR antibody, or any combination thereof. In some embodiments, the immunopotentiator is selected from the group consisting of anti-CD3 antibody, anti-CD28 antibody, IL-2, IL-15 or any combination thereof.

In certain embodiments, the therapeutic moiety is selected from the group consisting of cytotoxic agents. Herein, the cytotoxic agent includes any agent that is detrimental to (e.g., kills) a cell.

In certain embodiments, the cytotoxic agent is selected from the group consisting of alkylating agent, microtubule inhibitor or mitotic inhibitor, antitumor antibiotic, antimetabolite, topoisomerase inhibitor, tyrosine kinase inhibitor, radionuclide agent, and any combination thereof.

Examples of alkylating agent useful in the conjugate of the present invention include, but are not limited to, nitrogen mustards (e.g., mechlorethamine, chlorambucil, melphalan, cyclophosphamide, etc.), ethyleneimines (e.g., thiotepa, etc.), sulfate esters and polyols (e.g., busulfan, dibromomannitol), nitrosoureas (e.g., carmustine, lomustine, etc.), platinum antineoplastic agents (e.g., cisplatin, oxaliplatin, carboplatin, etc.) and so on.

Examples of mitotic inhibitor or microtubule inhibitor useful in the conjugate of the present invention include, but are not limited to, maytansinoids (e.g., maytansine, maytansinol, C-3 esters of maytansinol, etc.), taxanes (e.g., docetaxel, paclitaxel, or nanoparticle paclitaxel, etc.), vinca alkaloids (e.g., vindesine sulfate, vincristine, vinblastine, or vinorelbine, etc.)

Examples of antitumor antibiotic useful in the conjugate of the present invention include, but are not limited to, actinomycins, anthracyclines (e.g., daunorubicin, doxorubicin, epirubicin, idarubicin, etc.), calicheamicin, duocarmycin, etc.

Examples of antimetabolite useful in the conjugate of the present invention include, but are not limited to, folate antagonists (e.g., methotrexate, etc.), pyrimidine antagonists (e.g., 5-fluorouracil, floxuridine, cytarabine, capecitabine, gemcitabine, etc.), purine antagonists (e.g., 6-mercaptopurine, 6-thioguanine, etc.), adenosine deaminase inhibitors (e.g., cladribine, fludarabine, nelarabine, pentostatin, etc.).

Examples of topoisomerase inhibitor useful in the conjugate of the present invention include, but are not limited to, camptothecins and derivatives thereof (e.g., irinotecan, topotecan, etc.), amsacrine, daunomycin, adriamycin, epipodophyllotoxins, ellipticines, epirubicin, etoposide, propylimine, teniposide, etc.

Examples of tyrosine kinase inhibitor useful in the conjugate of the present invention include, but are not limited to, axitinib, bosutinib, cediranib, dasatinib, erlotinib, gefitinib, imatinib, lapatinib, neratinib, nilotinib, semaxinib, sunitinib, vandetanib, etc.

Examples of radionuclide agent useful in the conjugate of the present invention include, but are not limited to, $^{131}$I, $^{111}$In, $^{90}$Y, $^{177}$Lu, and the like.

In certain embodiments, the effector moiety is capable of increasing the solubility of the TCR. In certain embodiments, the effector moiety is selected from the group consisting of various portions of heavy or light chain constant regions of various subclasses of immunoglobulins (e.g., IgG, IgM, IgA, IgE). In certain embodiments, the effector moiety is selected from the group consisting of constant regions of human immunoglobulin, for example, heavy chain constant regions or light chain constant regions.

In certain embodiments, the effector moiety is selected from the group consisting of detectable labels. The detectable label of the present invention can be any substance detectable by fluorescent, spectroscopic, photochemical, biochemical, immunological, electrical, optical or chemical means. Such labels are well known in the art, examples of which include, but are not limited to, enzymes (e.g., horseradish peroxidase, alkaline phosphatase, β-galactosidase, urease, glucose oxidase, etc.), radionuclides (e.g., $^{3}$H, $^{125}$I, $^{35}$S, $^{14}$C, or $^{32}$P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750)), luminescent substances (e.g., chemiluminescent substances, such as acridinium ester compounds), magnetic beads (e.g., Dynabeads®), thermometric labels such as colloidal gold or colored glass or plastic (e.g., polystyrene, polypropylene, latex, etc.) beads, and biotin for binding avidin (e.g., streptavidin) modified with the aforementioned labels. The detectable labels as described above can be detected by methods known in the art. For example, radioactive labels can be detected using photographic film or scintillation counters, and fluorescent labels can be detected using photodetectors to detect emitted light. Enzyme labels are generally detected by providing a substrate to the enzyme and detecting the reaction product produced by the action of the enzyme on the substrate, and thermometric labels are detected by simple visualization of a colored label. In certain exemplary embodiments, the detectable label is selected from the group consisting of enzyme, radionuclide, fluorescent dye, luminescent substance (e.g., chemiluminescent substance), or biotin.

In certain embodiments, the TCR or antigen-binding fragment thereof of the present invention is conjugated to an effector moiety optionally via a linker (e.g., a peptide linker). In certain embodiments, the effector moiety is linked to the N- or C-terminus of the TCR or antigen-binding fragment thereof of the present invention.

In certain embodiments, when the effector moiety is a peptide or protein, the conjugate is preferably a fusion protein.

Therefore, in the fifth aspect, the present invention also provides a fusion protein comprising the TCR or antigen-binding fragment thereof as described in the second or third aspect and an additional peptide or protein.

In certain embodiments, the TCR or antigen-binding fragment thereof of the present invention is fused to the additional peptide or protein, optionally via a peptide linker. In certain embodiments, the effector moiety is linked to the N- or C-terminus of the TCR or antigen-binding fragment thereof of the present invention.

In certain embodiments, the additional peptide or protein may be selected from the group consisting of effector moieties of various peptides or proteins described in the fourth aspect.

In certain embodiments, the additional peptide or protein is selected from the group consisting of therapeutic peptide or protein, immunoglobulin constant region (e.g., human immunoglobulin constant region), detectable protein label, or protein tag.

In certain embodiments, the therapeutic peptide or protein is selected from the group consisting of: immunostimulatory antibodies (e.g., anti-CD3 antibodies, anti-CD28 antibodies, anti-CD40L (CD154) antibodies, anti-41BB (CD137) antibodies, anti-OX40 antibodies, anti-GITR antibodies, or any combination thereof), immunostimulatory cytokines (e.g., IL-2, IL-3, IL-12, IL-15, IL-18, IFN-γ, IL-10, TGF-β, GM-CSF, or any combination thereof), or peptides or proteins that are toxic to cells, capable of inhibiting cell proliferation, or inducing apoptosis (e.g., thymidine kinase TK (TK/GCV), TRAIL, or FasL).

In certain embodiments, the detectable protein label is selected from the group consisting of enzymes (e.g., horseradish peroxidase, alkaline phosphatase, β-galactosidase, urease, glucose oxidase, etc.), fluorescent proteins (e.g., green fluorescent protein (GFP), blue fluorescent protein (BFP), yellow fluorescent protein (YFP), red fluorescent protein (RFP)) or biotin, etc.

In some embodiments, the protein tag is selected from the group consisting of His, Flag, GST, MBP, HA, or Myc, etc., and those skilled in the art know how to select a suitable protein tag according to the desired purpose (e.g.,

purification, detection or tracking).

Preparation of epitope peptide, TCR and fusion protein

The epitope peptide, TCR or fusion protein containing TCR of the present invention can be prepared by various methods known in the art, for example, by genetic engineering recombination technology. For example, DNA molecules encoding them are obtained by chemical synthesis or PCR amplification; the resulting DNA molecules are inserted into expression vectors, and then transfected into host cells; then, the transfected host cells are cultivated under specific conditions, and express the epitope peptide, TCR or fusion protein containing TCR of present invention.

Accordingly, in a sixth aspect, the present invention provides an isolated nucleic acid molecule, comprising:

(i) a nucleotide sequence encoding the epitope peptide or variant thereof described in the first aspect;

(ii) a nucleotide sequence encoding the TCR or antigen-binding fragment thereof or α-chain variable region and/or β-chain variable region thereof described in the second or third aspect;

(iii) a nucleotide sequence encoding the fusion protein described in the fifth aspect.

In certain embodiments, the isolated nucleic acid molecule comprises a first nucleotide sequence encoding the α-chain variable region of the TCR or antigen-binding fragment thereof described in the second or third aspect and a second nucleotide sequence encoding the β-chain variable region thereof. In certain embodiments, the first nucleotide sequence and the second nucleotide sequence are linked optionally by a nucleotide sequence encoding a self-cleaving peptide (e.g., P2A, E2A, F2A or T2A). In certain embodiments, the self-cleaving peptide is P2A.

In a seventh aspect, the present invention provides a vector (e.g., a cloning vector or an expression vector) comprising the isolated nucleic acid molecule of the sixth aspect. In certain embodiments, the vector of the present invention is, for example, plasmid, cosmid, phage, and the like.

In an eighth aspect, the present invention provides a host cell comprising the isolated nucleic acid molecule of the sixth aspect or the vector of the seventh aspect. Such host cells include, but are not limited to, prokaryotic cells such as E. coli cells, and eukaryotic cells such as yeast cells, insect cells, plant cells, and animal cells (e.g., mammalian cells, such as mouse cells, human cells, etc.). In certain embodiments, the host cell is a microorganism.

In another aspect, there is also provided a method for preparing the epitope peptide, TCR or fusion protein comprising TCR of the present invention, which comprises: culturing the host cell described in the eighth aspect under conditions that allow protein expression, and recovering the epitope peptide, TCR or fusion protein comprising TCR from a culture of the host cell.

Engineered antigen presenting cell (APC)

The epitope peptide and variant thereof of the present invention can be used in T cell-based immunotherapy. In some cases, the T cell can recognize an MHC-peptide complex presented on the surface of APC through its TCR to induce an MHC-restricted immune response to RAS mutant.

Therefore, in a ninth aspect, the present invention provides an engineered antigen-presenting cell (APC), which presents on its surface the epitope peptide or variant thereof of the first aspect.

In certain embodiments, the epitope peptide or variant thereof is presented by an MHC-II molecule.

In certain embodiments, the MHC-II molecule is HLA-DQ.

In certain embodiments, the HLA-DQ comprises one selected from the group consisting of HLA-DQB1*0301, HLA-DQB1*0303, HLA-DQB1*0319, HLA-DQB1*0201, HLA-DQB 1 *0603, HLA-DQB 1 *0604, and HLA-DQB 1 *0302. Preferably, the HLA-DQ comprises one selected from the group consisting of HLA-DQB1*0301, HLA-DQB1*0303, and HLA-DQB1*0319. More preferably, the HLA-DQ comprises HLA-DQB1*0301.

In some embodiments, the HLA-DQ comprises one selected from the group consisting of HLA-DQA1*0501, HLA-DQA1*0505, HLA-DQA1*0102, HLA-DQA1*0103, and HLA-DQA1*0301. Preferably, the HLA-DQ comprises one selected from the group consisting ofHLA-DQA1*0501 and HLA-DQA1*0505.

In certain embodiments, the HLA-DQ comprises one selected from the group consisting of HLA-DQB1*0301, HLA-DQB1*0319 or HLA-DQB1*0303 (preferably HLA-DQB1*0301), and further comprises one selected from the group consisting of HLA-DQA1*0501 or DQA1 *0505.

In certain embodiments, the HLA-DQ comprises HLA-DQB1*0301 and HLA-DQA1*0501. In certain embodiments, the HLA-DQ comprises HLA-DQB1*0301 and HLA-DQA1*0505. In certain embodiments, the HLA-DQ comprises HLA-DQB1*0303 and HLA-DQA1*0501. In certain embodiments, the HLA-DQ comprises HLA-DQB1*0303 and HLA-DQA1*0505. In certain embodiments, the HLA-DQ comprises HLA-DQB1*0319 and HLA-DQA1*0501. In certain embodiments, the HLA-DQ comprises HLA-DQB1*0319 and HLA-DQA1*0505.

In certain embodiments, the APC is selected from the group consisting of dendritic cell, monocyte, macrophage, lymphoblastoid cell (LCL), or any combination thereof.

In certain embodiments, the APC is positive for HLA-DQB1*0301, positive for HLA-DQB 1*0303, positive for HLA-DQB 1*0319, positive for HLA-DQB 1*0201, positive for HLA-DQB 1*0603, positive for HLA-DQB 1 * 0604 or positive for HLA-DQB 1*0302; preferably, the APC is positive for HLA-DQB1*0301, positive for HLA-DQB1*0303 or positive for HLA-DQB 1*0319; more preferably, the APC is positive for HLA-DQB1* 0301.

In certain embodiments, the APC is positive for HLA-DQA1*0501, positive for HLA-DQA1*0505, positive for HLA-DQA1*0102, positive for HLA-DQA1*0103, or positive for HLA-DQA1*0301; preferably, the APC is positive for HLA-DQA1*0501 or positive for HLA-DQA1*0505.

In certain embodiments, the APC is positive for HLA-DQB1*0301, positive for HLA-DQB1*0319, or positive for HLA-DQB1*0303 (preferably positive for HLA-DQB1*0301), and is also positive for HLA-DQA1*0501 or positive for HLA-DQA1*0505.

In certain embodiments, the APC has an HLA-DP type of HLA-DQB1*0301/HLA-DQA1*0501, HLA-DQB1*0301/HLA-DQA1*0505, HLA-DQB1*0303/HLA-DQA1*0501, HLA-DQB1*0303/HLA-DQA1*0505, HLA-DQB1*0319/HLA-DQA1*0505 or HLA-DQB1*0319/HLA-DQA1*0501.

In certain embodiments, the APC is isolated from a subject positive for HLA-DQB1*0301, positive for HLA-DQB1*0303, positive for HLA-DQB1*0319, positive for HLA-DQB1*0201, positive for HLA-DQB1*0603, positive for HLA-DQB 1 *0604 or positive for HLA-DQB1*0302; preferably, the APC is isolated from a subject positive for HLA-DQB1*0301, positive for HLA-DQB 1 *0303 or positive for HLA-DQB1*0319; more preferably, positive for HLA-DQB1*0301.

In some embodiments, the APC is isolated from a subject positive for HLA-DQA1*0501, positive for HLA-DQA1*0505, positive for HLA-DQA1*0102, positive for HLA-DQA1*0103 or positive for HLA-DQA1*0301; preferably, the APC is isolated from a subject positive for HLA-DQA1*0501 or positive for HLA-DQA1*0505.

In certain embodiments, the APC is isolated from a subject who is positive for HLA-DQB1*0301, positive for HLA-DQB1*0319, or positive for HLA-DQB1*0303 (preferably positive for HLA-DQB1*0301), and is positive for HLA-DQA1*0501 or positive for HLA-DQA1*0505.

In certain embodiments, the APC is isolated from a subject with the following HLA-DP type: HLA-DQB1*0301/HLA-DQA1*0501, HLA-DQB1*0301/HLA-DQA1*0505, HLA-DQB1*0303/HLA-DQA1*0501, HLA-DQB1*0303/HLA-DQA1*0505, HLA-DQB1*0319/HLA-DQA1*0501 or HLA-DQB1*0319/HLA-DQA1*0501.

In certain embodiments, the engineered APC is obtained by contacting the APC with the epitope peptide or variant thereof described in the first aspect in vitro (i.e. exposing the APC to a sufficient amount of the epitope peptide or variant thereof). In some embodiments, the engineered APC is obtained by introducing an expression vector comprising a nucleotide sequence encoding the epitope peptide or variant thereof described in the first aspect into the APC in vitro.

The APC of the ninth aspect may be self/autologous ("self") or non-self ("non-self", e.g., allogeneic). "Autologous" refers to that cells are from the same subject; "allogeneic" refers to that cells are from a subject of the same species that is genetically distinct from that of the cells being compared.

The APC of the ninth aspect may be isolated or obtained from any tissue in which such cell is found, or may be otherwise cultured and provided. For example, the APC can be found in the bone marrow or peripheral blood mononuclear cells (PBMCs) of mammals, in the spleen of mammals, or in the skin of mammals (i.e., Langerhans cells can be found in the skin, which possess some properties similar to those of DCs), and then the APC is obtained by performing culturing in media containing appropriate cytokines followed by sorting.

In another aspect, the present invention provides a method for preparing the above-mentioned engineered APC, which comprises: (1) providing an APC from a subject; (2) contacting the APC with the epitope peptide or variant thereof described in the first aspect in vitro or introducing an expression vector comprising a nucleotide sequence encoding the epitope peptide or variant thereof described in the first aspect into the APC, so as to obtained an APC presenting on its surface the epitope peptide or variant thereof.

Engineered immune cells

The TCR or antigen-binding fragment thereof of the present invention can be used in T cell-based immunotherapy. In some instances, the T cell expressing the TCR of the present invention induces an MHC-restricted immune response to RAS mutant by recognizing an MHC-peptide complex.

Therefore, in the tenth aspect, the present invention provides an engineered immune cell, which expresses on its surface the TCR or antigen-binding fragment thereof described in the second or third aspect. The engineered immune cell of the present invention is antigen specific to the RAS G13D mutant. In certain embodiments, the engineered immune cell of the present invention has one or more characteristics selected from the following:

(i) specifically binding to the RAS G13D mutant, not binding to or binding with a lower affinity to other RAS proteins (including wild-type RAS protein or other mutants);

(ii) specifically binding to the epitope peptide or variant thereof described in the first aspect (e.g., the sequence set forth in any one of SEQ ID NOs: 14-23, 26-27, especially SEQ ID NO: 20);

(iii) being activated upon co-culture with an APC displaying (e.g., in the context of MHC-II) the epitope peptide or variant thereof of the present invention, in which the non-limiting examples of the activation include: increased secretion level of cytokine (e.g., II, -2, IFN-$\gamma$, etc.), increased proliferative activity, and/or increased expression level of activation marker (e.g., CD25, CD69, CD107a, etc.), as well as increased killing activity to a second cell displaying (e.g., in the context of MHC-II) the epitope peptide or variant thereof of the present invention.

In certain embodiments, the engineered immune cell comprises a nucleotide sequence encoding the TCR or antigen-binding fragment thereof as described in the second or third aspect.

The immune cell of the tenth aspect may be isolated or obtained from any tissue in which such cell is found. For example, the APC can be found in mammalian peripheral blood mononuclear cells, bone marrow, lymph node tissue, umbilical cord blood, thymus tissue, ascitic fluid, pleural effusion, spleen tissue or tumors, and then it is optionally cultured and sorted in culture media containing appropriate cytokines so as to obtain the desired immune cell. Alternatively, the immune cell can also be cultured and provided in other ways, for example, obtained from precursor cells of immune cells (e.g., precursors of T lymphocytes) through induction and differentiation, and the precursor cells can be, for example, pluripotent stem cells (e.g., embryonic stem cells, induced pluripotent stem cells), hematopoietic stem cells or lymphocyte progenitor cells, and hematopoietic stem cells or lymphocyte progenitor cells isolated and/or enriched from, for example, bone marrow, umbilical cord blood or peripheral blood.

In certain embodiments, the immune cell is a lymphocyte. In certain embodiments, the immune cell is selected from the group consisting of T cell, tumor infiltrating lymphocyte (TIL), natural killer (NK) cell, natural killer T (NKT) cell, or any combination thereof. Exemplary immune cells that can be used to express the TCR of the present invention include PBMC, TIL and/or T cell. In certain embodiments, the T cell is selected from the group consisting of: $\alpha\beta$ T cell, $\gamma\delta$ T cell, iPSC-derived T cell, CD8+ cytotoxic T cell, CD4+ cytotoxic T cell, CD4+ helper T cell (e.g., Th1 or Th2 cell), CD4/CD8 double positive T cell, tumor infiltrating T cell, thymocyte, memory T cell, natural killer T cell such as invariant natural killer T cell. In certain embodiments, the immune cell comprises CD4+ T cell. Those skilled in the art will understand that the immune cell may also include progenitor cell (precursor cell) of immune cell, wherein the progenitor cell may be induced to differentiate into immune cells in vivo or in vitro. Thus, in certain embodiments, the immune cell comprise immune cell progenitor, such as hematopoietic stem cell (HSC) contained within a population of CD34+ cells derived from cord blood, bone marrow, or flowing peripheral blood, which upon administration to a subject is differentiated into mature immune cells, or which can be induced to differentiate into mature immune cells in vitro.

The immune cell of the tenth aspect may be self/autologous ("self") or non-self ("non-self", e.g. allogeneic). "Autologous" refers to that cells are from the same subject; "allogeneic" refers to that cells are from a subject of the same species that is genetically distinct from that of the cells being compared.

In certain embodiments, the immune cell is isolated from a subject positive for HLA-DQB 1 *0301, positive for HLA-DQB1*0303, positive for HLA-DQB1 *0319, positive for HLA-DQB1*0201, positive for HLA-DQB 1*0603, positive for DQB1*0604 or positive for HLA-DQB 1 *0302; preferably, the immune cell is isolated from a subject positive for HLA-DQB1 *0301, positive for HLA-DQB1*0303 or positive for HLA-DQB1*0319; more preferably positive for HLA-DQB1*0301.

In certain embodiments, the immune cell is isolated from a subject positive for HLA-DQA1*0501, positive for HLA-DQA1*0505, positive for HLA-DQA1*0102, positive for HLA-DQA1*0103, or positive for HLA-DQA1*0301; preferably, the immune cell is isolated from a subject positive for HLA-DQA1*0501 or positive for HLA-DQA1*0505.

In certain embodiments, the immune cell is isolated from a subject that is positive for HLA-DQB 1 *0301, positive for HLA-DQB 1 *0319 or positive for HLA-DQB 1 *0303 (preferably positive for HLA-DQB1*0301), and is positive for HLA-DQA1* 0501 or positive for HLA-DQA1*0505.

In certain embodiments, the immune cell is isolated from a subject with the following HLA-DP type: HLA-DQB1*0301/HLA-DQA1*0501,    HLA-DQB1*0301/HLA-DQA1*0505,    HLA-DQB1*0303/HLA-DQA1*0501,    HLA-DQB1*0303/HLA-DQA1*0505, HLA-DQB1*0319/HLA-DQA1*0505 or HLA-DQB1*0319/HLA-DQA1*0501.

It should be understood that the engineered immune cell of the present invention may be contained in an isolated population of cells. The population of cells may be a heterogeneous population, for example, the population of cells may further comprise, in addition to the engineered immune cell of the present invention, at least one additional cell, and the additional cell is not antigen-specific for the RAS G13D mutant, or for example, the population of cells contains more than one type of immune cells, but these types of immune cells all express the TCR of the present invention so as to have antigen specificity for the RAS G13D mutant. Furthermore, the population of cells can also be a substantially homogeneous population, for example, the population mainly comprises (e.g., consists essentially of) T cells that have antigen specificity for the RAS G13D mutant.

In another aspect, the present invention provides a method for preparing the above-mentioned engineered immune cell, which comprises: (1) providing an immune cell from a subject; (2) introducing a nucleic acid molecule or vector comprising a nucleotide sequence encoding the TCR or antigen-binding fragment thereof of the present invention into the immune cell of step (1), so as to obtain an immune cell expressing the TCR or antigen-binding fragment thereof.

In some embodiments, in step (1), the immune cell is subjected to pretreatment; the pretreatment comprises sorting, activation and/or proliferation of the immune cell. In certain embodiments, the pretreatment comprises contacting the immune cell with one or more selected from the group consisting of anti-CD3 antibody, anti-CD28 antibody, IL-2 and IL-15, thereby stimulating the immune cell and inducing its proliferation, thereby generating a pretreated immune cell.

In some embodiments, in step (2), the nucleic acid molecule or vector can be introduced into the immune cell by various suitable methods, such as calcium phosphate transfection, DEAE-dextran-mediated transfection, microinjection, electroporation, TALEN method, ZFN method, non-viral vector-mediated transfection (e.g. liposome) or viral vector-mediated transfection (e.g. lentiviral infection, retroviral infection, adenoviral infection), and other physical, chemical or biological means for transferring into host cells, such as transposon technology, CRISPR-Cas9 and other technologies.

In some embodiments, after step (2), the method further comprises: expanding the immune cell obtained in step (2).

Epitope peptide-based therapy

The epitope peptide or APC presenting the epitope peptide of the present invention can be used in T cell-based immunotherapy to induce an anti-tumor immune response.

Therefore, in the eleventh aspect, the present invention provides a pharmaceutical composition comprising: the epitope peptide or variant thereof described in the first aspect or the MHC-peptide complex as described above, the nucleic acid molecule or vector or host cell comprising a nucleotide sequence encoding the peptide or variant thereof, or the engineered antigen-presenting cell (APC) described in the ninth aspect; and a pharmaceutically acceptable carrier and/or excipient.

In certain embodiments, the pharmaceutical composition is a tumor vaccine.

In certain embodiments, the pharmaceutical composition comprises an adjuvant. Adjuvants are those substances that can enhance the immune response in a non-specific manner, such as Freund's complete adjuvant, Freund's incomplete adjuvant, Toll receptor ligand, immunostimulatory antibody (e.g., anti-CD3 antibody, anti-CD28 antibody, anti-CD40L (CD154) antibody, anti-41BB (CD137) antibody, anti-OX40 antibody, anti-GITR antibody, or any combination thereof), or immunostimulatory cytokine (e.g., IL-2, IL-3, IL-12, IL-15, IL -18, IFN-$\gamma$, IL-10, TGF-$\beta$, GM-CSF, or any combination thereof), etc.

In certain embodiments, the pharmaceutical composition further comprises an additional therapeutic agent, such as an antineoplastic agent or an immunopotentiator.

In certain embodiments, the antineoplastic agent is selected from the group consisting of alkylating agent, mitotic inhibitor, antineoplastic antibiotic, antimetabolite, topoisomerase inhibitor, tyrosine kinase inhibitor, radionuclide agent, radiation sensitizer (e.g., gemcitabine, 5-fluorouracil, taxane, cisplatin, etc.), antiangiogenic agent, cytokine (e.g., GM-CSF, IL-7, IL-12, IL-15, IL-18, IL-21, etc.), antibody specific to tumor cell (e.g., CD20 antibody such as rituximab, Her2 antibody such as trastuzumab, VEGF antibody such as bevacizumab, EGFR antibody such as cetuximab etc.), immune checkpoint inhibitor (e.g., PD-1 antibody, PD-L1 antibody, CTLA-4 antibody, LAG-3 antibody, or TIM3 antibody).

In certain embodiments, the immunopotentiator is selected from the group consisting of immunostimulatory antibody (e.g., anti-CD3 antibody, anti-CD28 antibody, anti-CD40L (CD154) antibody, anti-41BB (CD137) antibody, anti-OX40 antibody, anti-GITR antibody or any combination thereof) or immunostimulatory cytokine (e.g., IL-2, IL-3, IL-12, IL-15, IL-18, IFN-$\gamma$, IL-10, TGF-$\beta$, GM-CSF, or any combination thereof).

In certain embodiments, in the pharmaceutical composition, the epitope peptide or variant thereof, the MHC-peptide complex, the engineered APC of the present invention and the additional therapeutic agent may be supplied as separate components or as mixed components.

In a twelfth aspect, the present invention provides a method for inducing an immune response against a tumor with a RAS G13D mutation in a subject, and/or preventing or treating a tumor with a RAS G13D mutation in a subject, wherein the method comprises administering to the subject in need thereof an effective amount of the epitope peptide or variant thereof of the first aspect, the MHC-peptide complex as described above, a nucleic acid molecule or vector or host cell comprising a nucleotide sequence encoding the epitope peptide or variant thereof, or the engineered antigen-presenting cell (APC) of the ninth aspect, or the pharmaceutical composition of the eleventh aspect.

In certain embodiments, the tumor with RAS G13D mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia.

In certain embodiments, the subject is a human.

In certain embodiments, the subject is positive for HLA-DQB1*0301, positive for HLA-DQB1*0303, positive for HLA-DQB 1*0319, positive for HLA-DQB 1*0201, positive for HLA-DQB 1 *0603, positive for HLA-DQB1 *0604, positive for HLA-DQB1 *0302; preferably, the subject is positive for HLA-DQB1*0301, positive for HLA-DQB1*0303 or positive for HLA-DQB1*0319; more preferably, the subject is positive for HLA-DQB1 *0301.

In certain embodiments, the subject is positive for HLA-DQA1*0501, positive for HLA-DQA1*0505, positive for HLA-DQA1*0102, positive for HLA-DQA1*0103, or positive for HLA-DQA1*0301; preferably, the subject is positive for HLA-DQA1*0501 or positive for HLA-DQA1*0505.

In certain embodiments, the subject is positive for HLA-DQB1*0301, positive for HLA-DQB1*0319, or positive for HLA-DQB 1 *0303 (preferably positive for HLA-DQB1*0301), and is also positive for HLA-DQA1*0501 or positive for HLA-DQA1*0505.

In certain embodiments, the subject has an HLA-DP type selected from the group consisting of: HLA-DQB1*0301/HLA-DQA1*0501, HLA-DQB1*0301/HLA-DQA1*0505, HLA-DQB1*0303/HLA-DQA1*0501, HLA-DQB1*0303/HLA-DQA1*0505, HLA-DQB1*0319/HLA-DQA1*0505 or HLA-DQB1*0319/HLA-DQA1*0501.

In certain embodiments, the epitope peptide or variant thereof described in the first aspect, the MHC-peptide complex as described above, the nucleic acid molecule or vector or host cell comprising a nucleotide sequence encoding the epitope peptide or variant thereof, or the engineered antigen-presenting cell (APC) described in the ninth aspect, or the pharmaceutical composition described in the eleventh aspect can be administrated in combination with an another therapeutic agent (e.g., an immunopotentiator or antineoplastic agent). Accordingly, in certain embodiments, the method further comprises administering to the subject an additional therapeutic agent (e.g., an immunopotentiator or antineoplastic agent), for example, simultaneously, separately, or sequentially.

In certain embodiments, the epitope peptide or variant thereof described in the first aspect, the MHC-peptide complex as described above, the nucleic acid molecule or vector or host cell comprising a nucleotide sequence encoding the epitope peptide or variant thereof, or the engineered antigen presenting cell (APC) of the ninth aspect, or the pharmaceutical composition of the eleventh aspect may be administered in combination with an additional therapy, for example, simultaneously, separately or sequentially. This additional therapy can be any therapy known to be used on tumor, such as surgery, chemotherapy, radiation therapy, targeted therapy, immunotherapy, hormone therapy, gene therapy or palliative care.

The epitope peptide or variant thereof of the present invention, the MHC-peptide complex as described above, the nucleic acid molecule or vector or host cell comprising a nucleotide sequence encoding the epitope peptide or variant thereof, the engineered antigen presenting cell (APC), or the pharmaceutical composition containing the same, can be formulated into any dosage form known in the medical field, for example, tablet, pill, suspension, emulsion, solution, gel, capsule, powder, granule, elixir, lozenge, suppository, injection (including injection solution, sterile powder for injection, and concentrated solution for injection), inhalant, spray, etc. The preferred dosage form depends on the intended mode of administration and therapeutic use. The pharmaceuticals of the present invention should be sterile and stable under the conditions of manufacture and storage. A preferred dosage form is injection. Such injection can be a sterile solution for injection. For example, the sterile solution for injection can be prepared by the following method: incorporating into an appropriate solvent a necessary amount of the epitope peptide or variant thereof of the present invention, the MHC-peptide complex as described above, the engineered antigen-presenting cell (APC) or the pharmaceutical composition comprising the same, and optionally, incorporating with other desired ingredients (including but not limited to, pH regulator, surfactant, adjuvant, ionic strength enhancer, isotonic agent, preservative, diluent, or any combination thereof), followed by filter sterilization. In addition, the sterile solution for injection can be prepared as sterile lyophilized powder (e.g., by vacuum drying or freeze-drying) for ease of storage and use. Such sterile lyophilized powder can be dispersed in suitable vehicle before use, such as water for injection (WFI), bacteriostatic water for injection (BWFI), sodium chloride solution (e.g. 0.9% (w/v) NaCl), dextrose solution (e.g., 5% dextrose), surfactant-containing solution (e.g., 0.01% polysorbate 20), pH buffer solution (e.g., phosphate buffered saline), Ringer's solution and any combination thereof.

Accordingly, in certain exemplary embodiments, the pharmaceutical composition of the eleventh aspect comprises a sterile injectable liquid (e.g., aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% dextrose), solution-containing surfactant (e.g., 0.01% polysorbate 20), pH buffered solution (e.g., phosphate buffered saline), Ringer's solution, and any combination thereof.

The epitope peptide or variant thereof of the present invention, the MHC-peptide complex as described above, the nucleic acid molecule or vector or host cell comprising a nucleotide sequence encoding the epitope peptide or variant thereof, the engineered antigen presenting cell (APC), or the pharmaceutical composition comprising the same, may be administered by any suitable method known in the art, including, but not limited to, oral, buccal, sublingual, ocular, local, parenteral, rectal, intrathecal, intra-cisterna, inguinal, intravesical, topical (e.g., powder, ointment, or drops), or nasal routes. However, for many therapeutic uses, the preferred route/mode of administration is parenteral (e.g., intravenous or bolus injection, subcutaneous injection, intraperitoneal injection, intramuscular injection). The skilled artisan will understand that the route and/or mode of administration will vary depending on the intended purpose. In certain embodiments, the epitope peptide or variant thereof of the present invention, the MHC-peptide complex as described above, the nucleic acid molecule or vector or host cell comprising a nucleotide sequence encoding the epitope peptide or variant thereof, the engineered antigen presenting cell (APC), or the pharmaceutical composition comprising the same, are administered by intravenous injection or bolus injection.

The pharmaceutical composition described in the eleventh aspect may comprises a "therapeutically effective amount" or "prophylactically effective amount" of the epitope peptide or variant thereof of the present invention, the MHC-peptide complex as described above, the nucleic acid molecule or vector or host cell comprising a nucleotide sequence encoding the peptide or variant thereof, or the engineered antigen-presenting cell (APC). In this context, the "therapeutically effective amount" is an amount capable of generating an immune response in a treated subject, and the immune response is capable of reducing or inhibiting proliferation of tumor cells and/or eliminating tumor cells; the "prophylactically effective amount" refers to an amount capable of generating an immune response against target cells (e.g., tumor cells containing RAS mutation) in the treated subject, and the immune response is capable of preventing the formation of tumors in the subject, or capable of substantially reducing the chance of developing a tumor or continuing to develop a tumor in the subject.

In another aspect, the present invention provides a use of the epitope peptide or variant thereof described in the first aspect, the MHC-peptide complex as described above, the nucleic acid molecule or vector or host cell comprising a nucleotide sequence encoding the epitope peptide or variant thereof, or the engineered antigen-presenting cell (APC) described in the ninth aspect, or the pharmaceutical composition described in the eleventh aspect, in the manufacture of a medicament for inducing an immune response in a subject against a tumor having a RAS G13D mutation, and/or preventing or treating a tumor having a RAS G13D mutation in a subject.

_TCR-based therapy_

The TCR of the present invention or the immune cell expressing the TCR can be used in T cell-based immunotherapy to kill a tumor containing RAS G13D mutation.

Therefore, in the thirteenth aspect, the present invention provides a pharmaceutical composition, which comprises: the TCR or antigen-binding fragment thereof described in the second or third aspect, the conjugate described in the fourth aspect, or the fusion protein described in the fifth aspect, the nucleic acid molecule or vector or host cell comprising a nucleotide sequence encoding the TCR or antigen-binding fragment thereof or conjugate or fusion protein, or the engineered immune cell described in the tenth aspect; and a pharmaceutically acceptable carrier and/or excipient.

In certain embodiments, the pharmaceutical composition further comprises an additional therapeutic agent, such as an antineoplastic agent or an immunopotentiator.

In certain embodiments, the antineoplastic agent is selected from the group consisting of alkylating agent, mitotic inhibitor, antineoplastic antibiotic, antimetabolite, topoisomerase inhibitor, tyrosine kinase inhibitor, radionuclide agent, radiation sensitizer (e.g., gemcitabine, 5-fluorouracil, taxanes, cisplatin, etc.), antiangiogenic agent, cytokine (e.g., GM-CSF, IL-7, IL-12, IL-15, IL-18, IL-21, etc.), antibody specific to tumor cell (e.g., CD20 antibody such as rituximab, Her2 antibody such as trastuzumab, VEGF antibody such as bevacizumab, EGFR antibody such as cetuximab etc.), immune checkpoint inhibitor (e.g., PD-1 antibody, PD-L1 antibody, CTLA-4 antibody, LAG-3 antibody, or TIM3 antibody).

In certain embodiments, the immunopotentiator is selected from the group consisting of immunostimulatory antibody (e.g., anti-CD3 antibody, anti-CD28 antibody, anti-CD40L (CD154) antibody, anti-41BB (CD137) antibody, anti-OX40 antibody, anti-GITR antibody, or any combination thereof) or immunostimulatory cytokine (e.g., IL-2, IL-3, IL-12, IL-15, IL-18, IFN-$\gamma$, IL-10, TGF-$\beta$, GM-CSF, or any combination thereof).

In certain embodiments, in the pharmaceutical composition, the TCR or antigen-binding fragment thereof, conjugate, fusion protein or engineered immune cell of the present invention and the additional therapeutic agent may be used as separate components or as mixed components.

In a fourteenth aspect, the present invention provides a method for inducing an immune response in a subject against a tumor with a RAS G13D mutation, and/or preventing or treating a tumor with a RAS G13D mutation in a subject, wherein the method comprises administering to the subject in need thereof an effective amount of the TCR or antigen-binding fragment thereof of the second or third aspect, the conjugate of the fourth aspect, or the fusion protein of the fifth aspect, the nucleic acid molecule or vector or host cell comprising a nucleotide sequence encoding the TCR or antigen-binding fragment thereof or conjugate or fusion protein, or the engineered immune cell described in the tenth aspect, or the pharmaceutical composition of the thirteenth aspect.

In certain embodiments, the tumor having RAS G13D mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia.

In certain embodiments, the subject is a human.

In certain embodiments, the subject is positive for HLA-DQB1*0301, positive for HLA-DQB1*0303, positive for HLA-DQB 1*0319, positive for HLA-DQB 1*0201, positive for HLA-DQB 1 *0603, positive for HLA- DQB 1 *0604 or positive for HLA-DQB 1 *0302; preferably, the subject is positive for HLA-DQB1*0301, positive for HLA-DQB1*0303 or positive for HLA-DQB1*0319; more preferably, the subject is positive for HLA-DQB1 *0301.

In certain embodiments, the subject is positive for HLA-DQA1*0501, positive for HLA-DQA1*0505, positive for HLA-DQA1*0102, positive for HLA-DQA1*0103, or positive for HLA-DQA1*0301; preferably, the subject is positive for HLA-DQA1*0501 or positive for HLA-DQA1*0505.

In certain embodiments, the subject is positive for HLA-DQB1*0301, positive for HLA-DQB 1*0319, or positive for HLA-DQB 1 *0303 (preferably positive for HLA-DQB1*0301), and is also positive for HLA-DQA1 *0501 or positive for HLA-DQA1*0505.

In certain embodiments, the subject has an HLA-DP type selected from the group consisting of: HLA-DQB1*0301/HLA-DQA1*0501, HLA-DQB1*0301/HLA-DQA1*0505, HLA-DQB1*0303/HLA-DQA1*0501, HLA-DQB1*0303/HLA-DQA1*0505, HLA-DQB1*0319/HLA-DQA1*0505 or HLA-DQB1*0319/HLA-DQA1*0501.

In certain embodiments, the method comprises: (1) providing an immune cell required by the subject; (2) introducing the nucleotide sequence encoding the TCR or antigen-binding fragment thereof described in the second or third aspect into the immune cell described in step (1) to obtain an immune cell expressing on its surface the TCR or antigen-binding fragment thereof; (3) administering the immune cell obtained in step (2) to the subject.

In certain embodiments, a step of obtaining the immune cell from the subject is comprised prior to step (1). The immune cell may be isolated or obtained from any tissue in which such cell is found (e.g., peripheral blood mononuclear cells, bone marrow, lymph node tissue, umbilical cord blood, thymus tissue, ascites, pleural effusion, spleen tissue, and tumors), or may be cultured and provided by other method, for example, obtained by inducing differentiation from a precursor cell of immune cell (e.g., precursor of T lymphocyte).

In certain embodiments, the immune cell is selected from the group consisting of lymphocytes. In some embodiments, the immune cell is selected from the group consisting of T cell (e.g., $\alpha\beta$T cell, $\gamma\delta$T cell or iPSC-derived T cell), tumor infiltrating lymphocyte (TIL), natural killer (NK) cell, natural killer T (NKT) cell, or any combination thereof. In certain embodiments, the immune cell comprises CD4+ T cell.

In certain exemplary embodiments, a peripheral blood mononuclear cell (PBMC) and/or TIL are obtained from the subject and directly genetically engineered to express the TCR.

In certain exemplary embodiments, a T cell is obtained from the subject and genetically engineered to express the TCR. The T cell can be obtained from a variety of sources, for example, the T cell can be obtained from a blood unit collected from the subject using various techniques known to the skilled person (e.g., deposition, for example, FICOLL™ isolation). In one embodiment, the cells from circulating blood of individual are obtained by apheresis. The product of apheresis usually contains lymphocytes, including T cells, monocytes, granulocytes, B cells, other nucleated white blood cells, red blood cells, and platelets. In one embodiment, the cells collected by apheresis can be washed to remove plasma fraction, and the cells are placed in a suitable buffer or medium for subsequent processing. As understood by those of ordinary skill in the art, the washing step can be accomplished by methods known to those of skill in the art, such as by using a semi-automatic flow-through centrifuge. After washing, cells can be resuspended in a variety of biocompatible buffers or other saline solutions with or without buffers. In certain embodiments, unwanted components of apheresis sample can be removed in the medium in which the cells are directly resuspended. In certain embodiments, T cells are isolated from peripheral blood mononuclear cells (PBMCs) by lysing red blood cells and depleting monocytes (e.g., by gradient centrifugation through PERCOLL™). Specific T cell subpopulations expressing one or more of the following markers: CD3, CD28, CD4, CD8, CD45RA and CD45RO can be further isolated by positive or negative selection techniques. In one embodiment, specific T cell subpopulations expressing CD3, CD28, CD4, CD8, CD45RA and CD45RO are further isolated by positive or negative selection techniques. For example, enrichment of a T cell population can be accomplished by negative selection with a combination of antibodies directed against surface markers specific to the negatively selected cells. An exemplary method is to perform cell sorting and/or selection via negative magnetic immunoadhesion or flow cytometry, in which the negative magnetic immunoadhesion or flow cytometry utilizes a mixture of monoclonal antibodies directed against cell surface markers present on the negatively selected cells. For example, to enrich for CD4+ cells by negative selection, a mixture of monoclonal antibodies typically contains antibodies against CD14, CD20, CD11b, CD16, HLA-DR, and CD8. Flow cytometry and cell sorting can also be used to isolate cell populations of interest for use in the present invention.

In certain embodiments, the immune cells (e.g., T cells) can be activated and expanded (or differentiated, in the case of progenitor cells) in vitro prior to the genetic modification of the immune cells.

In some embodiments, the TCR or antigen-binding fragment thereof of the second or third aspect, the conjugate of the fourth aspect, or the fusion protein of the fifth aspect, the nucleic acid molecule or vector or host cell comprising a nucleotide sequence encoding the TCR or antigen-binding fragment thereof or conjugate or fusion protein, or the engineered immune cell described in the tenth aspect, or the pharmaceutical composition described in the thirteenth aspect, can be administrated in combination with an additional therapeutic agent (e.g., immunopotentiator or antineoplastic agent). Accordingly, in certain embodiments, the method further comprises administering to the subject an additional therapeutic agent (e.g., immune potentiating agent or antineoplastic agent), for example, simultaneously, separately, or sequentially.

In some embodiments, the TCR or antigen-binding fragment thereof of the second or third aspect, the conjugate of the fourth aspect, or the fusion protein of the fifth aspect, the nucleic acid molecule or vector or host cell comprising a nucleotide sequence encoding comprising the TCR or antigen-binding fragment thereof or conjugate or fusion protein, or the engineered immune cell described in the tenth aspect, or the pharmaceutical composition described in the thirteenth aspect, can be administrated in combination with an additional therapy, for example, simultaneously, separately or sequentially. The additional therapy can be any therapy known to be used on tumors, such as surgery, chemotherapy, radiation therapy, targeted therapy, immunotherapy, hormone therapy, gene therapy or palliative care.

The TCR or antigen-binding fragment thereof, conjugate, fusion protein, nucleic acid molecule or vector or host cell comprising a nucleotide sequence encoding the TCR or antigen-binding fragment thereof or conjugate or fusion protein, engineered immune cell, or pharmaceutical composition comprising the same according to the present invention, can be formulated into any dosage form known in the medical field, for example, tablet, pill, suspension, emulsion, solution, gel, capsule, powder, granule, elixir, lozenge, suppository, injection (including solution for injection, sterile powder for injection and concentrated solution for injection), inhalant, spray, etc. The preferred dosage form depends on the intended mode of administration and therapeutic use. The pharmaceuticals of the present invention should be sterile and stable under the conditions of manufacture and storage. A preferred dosage form is injection. Such injection can be a sterile solution for injection. For example, the sterile solution for injection can be prepared by incorporating in an appropriate solvent a necessary dose of the TCR or antigen-binding fragment thereof, conjugate, fusion protein, or engineered immune cell according to the present invention, or the pharmaceutical composition comprising the same, and optionally, simultaneously incorporating other desired ingredients (including but not limited to, pH regulator, surfactant, adjuvant, ionic strength enhancer, isotonic agent, preservative, diluent, or any combination thereof), followed by filter sterilization. In addition, the sterile solution for injection can be prepared as a sterile lyophilized powder (e.g., by vacuum drying or freeze-drying) for ease of storage and use. Such sterile lyophilized powder can be dispersed in a suitable vehicle before use, such as water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffer solution (e.g., phosphate buffered saline), Ringer's solution and any combination thereof.

Accordingly, in certain exemplary embodiments, the pharmaceutical composition of the thirteenth aspect comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffered solution (e.g., phosphate buffered saline), Ringer's solution, and any combination thereof.

The TCR or antigen-binding fragment thereof, conjugate, fusion protein, nucleic acid molecule or vector or host cell comprising a nucleotide sequence encoding the TCR or antigen-binding fragment thereof or conjugate or fusion protein, engineered immune cell, or pharmaceutical composition comprising the same according to the present invention, may be administered by any suitable method known in the art, including, but not limited to, oral, buccal, sublingual, ocular, local, parenteral, rectal, intrathecal, intra-cisterna, groin, intravesical, topical (e.g., powder, ointment, or drops), or nasal route. However, for many therapeutic uses, the preferred route/mode of administration is parenteral (e.g., intravenous or bolus injection, subcutaneous injection, intraperitoneal injection, intramuscular injection). The skilled artisan will understand that the route and/or mode of administration will vary depending on the intended purpose. In certain embodiments, the TCR or antigen-binding fragment thereof, conjugate, fusion protein, nucleic acid molecule or vector or host cell comprising a nucleotide sequence encoding the TCR or antigen-binding fragment thereof or conjugate or fusion protein, engineered immune cell, or pharmaceutical compositions comprising the same according to the present invention are administered by intravenous injection or bolus injection.

The pharmaceutical composition described in the thirteenth aspect may comprise a "therapeutically effective amount" or "prophylactically effective amount" of the TCR or antigen-binding fragment thereof, conjugate, fusion protein, nucleic acid molecule or vector or host cell comprising a nucleotide sequence encoding the TCR or antigen-binding fragment thereof or conjugate or fusion protein, engineered immune cell, or pharmaceutical composition comprising the same according to the present invention. In this context, the "therapeutically effective amount" is an amount capable of generating an immune response capable of reducing or inhibiting proliferation of tumor cells and/or eliminating tumor cells in a treated subject; and the "prophylactically effective amount" refers to an amount capable of generating an immune response against target cells (e.g., tumor cells containing RAS mutation) in a treated subject, in which the immune response is capable of preventing the formation of tumors in the subject, or capable of substantially reducing the chance of developing a tumor or continuing to develop a tumor in the subj ect.

In another aspect, the present invention provides a use of the TCR or antigen-binding fragment thereof described in the second or third aspect, the conjugate described in the fourth aspect, or the fusion protein described in the fifth aspect, the nucleic acid molecule or vector or host cell comprising a nucleotide sequence encoding the TCR or antigen-binding fragment thereof or conjugate or fusion protein, or the engineered immune cell described in the tenth aspect, or the pharmaceutical composition described in the thirteenth aspect, in the manufacture of a medicament for inducing an immune response against a tumor with a RAS G13D mutation in a subject, and/or preventing or treating a tumor with a RAS G13D mutation in a subject.

Combination therapy based on epitope peptide and TCR

In some cases, the epitope peptide of the present invention or APC presenting the epitope peptide, and the TCR of the present invention or immune cells expressing the TCR can also be administered in combination to achieve combination therapy for tumors containing RAS G13D mutation. Therefore, another aspect of the present invention also provides a method for inducing an immune response against a tumor with a RAS G13D mutation in a subject, and/or preventing or treating a tumor with a RAS G13D mutation in a subject, the method comprising administering to the subject in need thereof effective amounts of a first therapeutic agent and a second therapeutic agent in combination, wherein the first therapeutic agent is selected from the epitope peptide of the present invention or an APC presenting the epitope peptide, and the second therapeutic agent is selected from the TCR of the present invention or an immune cell expressing the TCR. The first and second therapeutic agents may be administered simultaneously, separately or sequentially. Another aspect of the present invention also provides a use of the epitope peptide of the present invention or the APC presenting the epitope peptide, the TCR of the present invention or the immune cell expressing the TCR in the manufacture of a medicament, and the medicament is used for inducing an immune response against a tumor having a RAS G13D mutation in a subject, and/or preventing or treating a tumor having a RAS G13D mutation in a subject. The dosage forms, administration routes, indications, combination therapy and the like described above for the epitope peptide-based therapy and the TCR-based therapy can be applied to the combination therapy of the epitope peptide and TCR.

Definition of terms

In the present application, unless otherwise stated, scientific and technical terms used herein have the meanings commonly understood by those skilled in the art. Moreover, the nucleic acid chemistry laboratory operation steps used herein are all routine steps widely used in the corresponding field. Meanwhile, in order to better understand the present invention, definitions and explanations of related terms are provided below. Unless specifically defined or described differently elsewhere herein, the following terms and descriptions related to the present invention are to be understood in accordance with the definitions given below.

As used herein, the term "RAS" refers to a proto-oncogene, and a RAS protein encoded by which has GTPase activity and participates in many signaling pathways that regulate cell proliferation, differentiation and apoptosis, such as MAPK, PI3K, STAT signaling pathways, etc. There are three RAS genes in human genes, namely HRAS (GeneID: 3265), NRAS (GeneID: 4893) and KRAS (GeneID: 3845), and the three RAS genes have a high degree of sequence homology (>90%). RAS gene mutation is the driving factor of cancers, and RAS gene mutation is the most frequent proto-oncogene mutation. The sequences of RAS proteins encoded by RAS genes are well known to those skilled in the art and can be found in various public databases. For example, the sequence of KRAS protein can be found in NCBI: NP_001356715.1, the sequence of NRAS protein can be found in NCBI: NP_002515.1, and the sequence of HRAS protein can be found in NCBI: NP_001123914.1.

As used herein, the term "RAS G13D mutant" refers to a RAS mutant in which amino acid residue Gly at position 13 is mutated to Asp. In some embodiments, the RAS G13D mutant refers to a KRAS G13D mutant. Herein, when referring to the amino acid sequence of the RAS G13D mutant, the sequence set forth in SEQ ID NO:51 is used for description. For example, the expression "amino acid residues at positions 7-17 of RAS G13D mutant protein" refers to amino acid residues at positions 7-17 of the sequence set forth in SEQ ID NO: 51, or the corresponding fragment of other RAS G13D mutant amino acid sequences. "Corresponding fragment" refers to a fragment at the same position in sequences being compared when the sequences are optimally aligned, i.e., when the sequences are aligned for the highest percent identity.

As used herein, the terms "major histocompatibility complex" and "MHC" are used interchangeably, which refer to a group of genes that determine whether transplanted tissues are identical, are closely related to immune responses, and are closely linked, and mainly include MHC-I molecules and MHC-II molecules. "MHC-I molecule" refers to a dimer of MHC-I $\alpha$ chain and $\beta$2 microglobulin chain, and "MHC-II molecule" refers to a dimer of MHC-II $\alpha$ chain and MHC-II $\beta$ chain. The human MHC is called the human leukocyte antigen (HLA) complex.

As used herein, the term "MHC-peptide complex" refers to an MHC molecule (MHC-I or MHC-II) comprising a peptide bound in the MHC peptide-binding pocket well known in the art. In some cases, the MHC molecule can be an membrane-bound protein expressed on the cell surface. In other cases, MHC molecules may be soluble proteins lacking transmembrane or cytoplasmic regions.

As used herein, the term "epitope" in reference to TCR refers to a localized region of an antigen (e.g., a peptide or peptide-MHC complex) to which a TCR can bind. In certain embodiments, a TCR-bound epitope can be detected by, for example, NMR spectroscopy, X-ray diffraction crystallographic study, ELISA assay, hydrogen/deuterium exchange mass spectrometry (e.g., liquid chromatography electrospray mass spectrometry), flow cytometry, mutagenesis mapping (e.g., site-directed mutagenesis mapping) and/or structural modeling. In some exemplary embodiments, the epitope of antigen can be determined by using alanine scanning mutation study. In certain embodiments, the antigen is a peptide-MHC complex or a peptide presented by an MHC molecule.

As used herein, the terms "T cell receptor" and "TCR" are used interchangeably and refer to a molecule comprising a CDR or variable region from an αβ or γδ T cell receptor. Examples of TCR include, but are not limited to, full-length TCR, antigen-binding fragments of TCR, soluble TCR lacking transmembrane and cytoplasmic regions, single-chain TCR containing TCR variable regions attached by flexible linker, TCR chains linked via engineered disulfide bonds, etc.

As used herein, the term "full-length TCR" refers to a TCR comprising a dimer of a first polypeptide chain and a second polypeptide chain, each of the polypeptide chains comprises a TCR variable region and a TCR constant region comprising a transmembrane region and a TCR cytoplasmic region. In certain embodiments, the full-length TCR comprises a mature full-length TCR α chain and a mature full-length TCR β chain. In certain embodiments, the full-length TCR comprises a mature full-length TCR γ chain and a mature full-length TCR δ chain.

As used herein, the term "TCR variable region" refers to a portion of a mature TCR polypeptide chain (e.g., TCR α chain or β chain), and the portion is not encoded by the TRAC gene of TCR α chain, the TRBC1 gene or TRBC2 gene of TCR β chain, the TRDC gene of TCR δ chain or TRGC1 gene or TRGC2 gene encoding of TCR γ chain. In certain embodiments, the TCR variable region of TCR α chain encompasses all amino acids of a mature TCR α chain polypeptide encoded by the TRAV gene and/or TRAJ gene, and the TCR variable region of TCR β chain encompasses all amino acids of a mature TCR β chain polypeptide encoded by the TRBV gene, TRBD gene and/or TRBJ gene (see, for example, "T Cell Receptor Facts Book", (2001), LeFranc and LeFranc, Academic Press, ISBN0-12-441352-8, which is incorporated herein by reference in its entirety). The TCR variable region typically comprises framework regions (FRs) 1, 2, 3 and 4 and complementarity determining regions (CDRs) 1, 2 and 3. Herein, the terms "α chain variable region" and "Vα" are used interchangeably and refer to a variable region of TCRα chain. The terms "β chain variable region" and "Vβ" are used interchangeably and refer to a variable region of TCR β chain.

As used herein, the term "CDR" or "complementarity determining region" in reference to TCR refers to a non-contiguous antigen-binding site found within a variable region of TCR chain (e.g., α chain or β chain). These regions have been described in Lefranc, (1999) "The Immunologist", 7:132-136; Lefranc et al., (1999) "Nucleic Acids Res" 27:209-212; LeFranc (2001) "T Cell Receptor Facts Book", Academic Press, ISBN 0-12-441352-8; Lefranc et al., (2003) Dev Comp Immunol 27(1):55-77; and Kabat et al., (1991) "Sequences of proteins of immunological interest", each of which is hereby incorporated by reference in its entirety. In certain embodiments, the CDRs are defined according to the IMGT numbering system described in Lefranc (1999), supra. In certain embodiments, the CDRs are defined according to the Kabat numbering system described in Kabat, supra.

As used herein, the term "FR" or "framework region" in reference to TCR refers to those amino acid residues in a variable region of TCR chain (e.g., α chain or β chain) other than the CDRs as defined above.

As used herein, the term "constant region" in reference to TCR refers to a TCR portion encoded by the TRAC gene (for TCR α chain), the TRBC1 or TRBC2 gene (for TCR β chain), the TRDC gene (for TCR δ chain), or the TRGC1 or TRGC2 gene (for TCR γ chain), and optionally lacks all or a portion of transmembrane region and/or all or a portion of cytoplasmic region. In certain embodiments, the TCR constant region lacks transmembrane and cytoplasmic regions. The TCR constant region does not contain amino acids encoded by the TRAV, TRAJ, TRBV, TRBD, TRBJ, TRDV, TRDD, TRDJ, TRGV, or TRGJ genes (see, for example, T Cell Receptor Facts Book, (2001), LeFranc and LeFranc, Academic Press, ISBN 0-12-441352-8, which is hereby incorporated by reference in its entirety).

In the context of TCR, the term "extracellular" refers to one or more portions of TCR chain located outside a cell, the term "transmembrane" refers to one or more portions of TCR chain embedded in the plasma membrane of a cell, and the term "cytoplasmic " refers to one or more portions of TCR chain located in the cytoplasm of a cell.

As used herein, the term "antigen-binding portion" in reference to TCR refers to any portion or fragment of a TCR, in which the portion or fragment as a part of the TCR retains the biological activity of the TCR (the parental TCR). The biological activity may include: the ability to specifically bind to the same antigen (e.g., RAS G13D mutant) or MHC-antigen complex to which the parental TCR binds.

As used herein, the term "specific binding" refers to a non-random binding reaction between two molecules, such as the reaction between an antibody and its antigen. The strength or affinity of a specific binding interaction can be expressed in terms of the equilibrium dissociation constant ($K_D$) for that interaction. In the present invention, the term "$K_D$" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen. The specific binding properties between two molecules can be determined using methods well known in the art. One method involves measuring the rate of formation and dissociation of antigen binding site/antigen complex. Both "association rate constant" ($k_a$ or $k_{on}$) and "dissociation rate constant" ($k_{dis}$ or $k_{off}$) can be calculated from concentrations and actual rates of association and dissociation (see, Malmqvist M, Nature, 1993, 361:186-187). The ratio of $k_{dis}/k_{on}$ is equal to the dissociation constant $K_D$ (see, Davies et al., Annual Rev Biochem, 1990; 59:439-473). The values of $K_D$, $k_{on}$ and $k_{dis}$ can be measured by any effective method, for example, measured by surface plasmon resonance (SPR) in Biacore, or by bioluminescent interferometry or Kinexa.

In the context of TCR, the term "specific binding" or "specific recognition" refers to the ability of a TCR to preferentially bind a specific antigen (e.g., a specific peptide or a specific peptide-MHC complex). Typically, a TCR that specifically binds an antigen does not bind or binds with lower affinity to other antigens. For example, an antigen-specific TCR binds to a target antigen with an association constant ($K_a$) at least 2 times, 5 times, 10 times, 50 times, 100 times, 500 times, 1,000 times, 5,000 times, or 10,000 times the $K_a$ for a non-specific antigen. In certain embodiments, the TCR or antigen-binding fragment thereof disclosed herein specifically binds to a RAS G13D mutant. In certain embodiments, the TCR or antigen-binding fragment thereof disclosed herein specifically binds to the epitope peptide or variant thereof of the first aspect. In certain embodiments, the TCR or antigen-binding fragment thereof disclosed herein specifically binds to the sequence set forth in any one of SEQ ID NOs: 14-23, 26-17 (especially SEQ ID NO: 20).

As used herein, the term "antigen presenting cell" or "APC" refers to any cell capable of presenting on its cell surface a peptide fragment of protein associated with a major histocompatibility complex (MHC) molecule. Such cell is well known to those skilled in the art and includes, but is not limited to, for example, dendritic cell, monocyte, macrophage, lymphoblastoid cell (LCL), and the like.

As used herein, the term "immune cell" refers to any cell of the immune system that has one or more effector functions. The immune cells typically comprise cells that play a role in the immune response, and they usually are of hematopoietic origin. The term "effector function" refers to a specialized function of an immune cell, such as a function or response that enhances or promotes an immune attack on a target cell (e.g., killing a target cell, or inhibiting its growth or proliferation). For example, an effector function of a T cell may be, for example, cytolytic activity or activity of helping or including secretion of cytokines. Examples of immune cells include T cells (e.g., $\alpha/\beta$ T cells and $\gamma/\delta$ T cells), B cells, natural killer (NK) cells, natural killer T (NKT) cells, mast cells, and bone marrow-derived macrophages, among others.

The immune cell of the present invention may be self/autologous ("self") or non-self ("non-self", e.g., allogeneic, syngeneic or heteroallelic). As used herein, "autologous" refers to that cells are from the same subject; "allogeneic" refers to that cells are from a subject of the same species that is genetically different from that of the cells being compared; "syngeneic" refers to that cells are from a different subject that is genetically identical to that of the cells being compared; "heteroallelic" refers to that cells are from a species different from that of the cells being compared. In certain embodiments, the immune cell of the present invention is autologous or allogeneic.

As used herein, the term "cytotoxic agent" includes any agent that is detrimental to (e.g., kills) a cell, such as chemotherapeutic drug, bacterial toxin, plant toxin, or radioactive isotope, among others.

As used herein, the term "vector" refers to a nucleic acid delivery vehicle into which a polynucleotide can be inserted. When the vector is capable of achieving expression of a protein encoded by the inserted polynucleotide, the vector is called an expression vector. The vector can be introduced into a host cell by transformation, transduction or transfection, so that the genetic material elements it carries can be expressed in the host cell. Vectors are well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); phage such as lambda phage or M13 phage, and animal virus. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector can contain a variety of elements that control expression, including but not limited to, promoter sequence, transcription initiation sequence, enhancer sequence, selection element, and reporter gene. In addition, the vector may also contain a replication origin.

As used herein, the term "host cell" refers to a cell into which a vector can be introduced, which includes, but is not limited to, prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell, immune cell (e.g., T lymphocyte, NK cell, monocyte, macrophage or dendritic cell, etc.). The host cell can comprise a single cell or a population of cells.

As used herein, the term "isolated" means that it has been separated or purified from components (e.g., nucleic acids, proteins, or other naturally occurring biological or organic molecules) that naturally accompany it.

As used herein, the term "identity" is used to refer to the match of sequences between two polypeptides or between two nucleic acids. When a position in both sequences being compared is occupied by the same base or amino acid monomer subunit (e.g., a position in each of the two DNA molecules is occupied by an adenine, or a position in each of the two polypeptides is occupied by a lysine), then the molecules are identical at that position. "Percent identity" between two sequences is a function of the number of matching positions shared by the two sequences divided by the number of positions being compared $\times 100$. For example, two sequences have an identity of 60% if 6 out of 10 positions match. For example, the DNA sequences CTGACT and CAGGTT have an identity of 50% (3 out of a total of 6 positions match). Generally, comparisons are made when two sequences are aligned for maximum identity. Such alignments can be achieved using, for example, the method of Needleman et al. (1970) J. Mol. Biol. 48:443-453 which can be conveniently performed by computer programs such as the Align program (DNAstar, Inc.). The algorithm of E. Meyers and W. Miller (Comput. Appl Biosci., 4:11-17 (1988)) which has been incorporated into the ALIGN program (version 2.0) can also be used to determine the percent identity between two amino acid sequences by using the PAM120 weight residue table, a gap length penalty of 12, and a gap penalty of 4. In addition, the algorithm of Needleman and Wunsch (J Mol Biol. 48:444-453 (1970)) that has been integrated into the GAP program of the GCG software package (available at www.gcg.com) can be used to determine the percent identity between two amino acid sequences by using the Blossum 62 matrix or PAM250 matrix with a gap weight of 16, 14, 12, 10, 8, 6 or 4 and a length weight of 1, 2, 3, 4, 5 or 6.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions comprise substitutions for amino acid residues with amino acid residues that have similar side chains, e.g., substitutions with residues physically or functionally similar (e.g., having similar size, shape, charge, chemical properties, including the ability to form covalent bonds or hydrogen bonds, etc.) to the corresponding amino acid residues. Families of amino acid residues having similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), $\beta$ branched side chains (e.g. threonine, valine, isoleucine) and aromatic side chains (e.g. tyrosine, phenylalanine, tryptophan, histidine). Therefore, it is preferred to replace the corresponding amino acid residue with another amino acid residue from the same side chain family. Methods for identifying amino acid conservative substitutions are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The writing of the twenty conventional amino acids referred to herein follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient pharmacologically and/or physiologically compatible with the subject and the active ingredient, which are well known in the art (see, for example, Remington's Pharmaceutical Sciences. Edited by Gennaro AR, 19th ed. Pennsylvania: Mack Publishing Company, 1995), and include, but are not limited to: pH adjusting agent, surfactant, adjuvant, ionic strength enhancing agent, diluent, agent for maintaining osmotic pressure, agent for delaying absorption, preservative. For example, the pH adjusting agent includes, but is not limited to, phosphate buffer. The surfactant includes but is not limited to cationic, anionic or nonionic surfactant, such as Tween-80. The ionic strength enhancing agent includes, but is not limited to, sodium chloride. The preservative includes, but is not limited to, various antibacterial and antifungal agents, such as paraben, chlorobutanol, phenol, sorbic acid, and the like. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, and the like. The stabilizing agent has the meaning generally understood by those skilled in the art, and can stabilize the desired activity of the active ingredient in the medicine, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation products thereof (e.g., lactalbumin hydrolyzate), etc. In certain exemplary embodiments, the pharmaceutically acceptable carrier or excipient comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffered solution (e.g., phosphate buffered saline), Ringer's solution, and any combination thereof.

As used herein, the term "prevention" refers to a method performed to prevent or delay the occurrence of a disease or disorder or symptom (e.g., a tumor) in a subject. As used herein, the term "treatment" refers to a method performed to obtain a beneficial or desired clinical outcome. For the purposes of the present invention, a beneficial or desired clinical outcome includes, but is not limited to, relief of symptom, reduction of disease extent, stabilization (i.e., no longer worsening) of disease state, delay or slowing of disease progression, amelioration or palliation of disease status, and relief of symptom (whether partial or total), whether detectable or not. Additionally, "treatment" can also refer to prolonging survival as compared to expected survival if not receiving treatment.

As used herein, the term "effective amount" refers to an amount sufficient to achieve, or at least partially achieve, the desired effect. For example, an effective amount for preventing a disease (e.g., tumor) refers to an amount sufficient to prevent, arrest, or delay the occurrence of the disease (e.g., tumor); an effective amount for treating a disease refers to an amount sufficient to cure, or at least partially prevent, an existing disease or complication thereof in a patient. Determining such an effective amount is well within the capability of those skilled in the art. For example, an amount effective for therapeutic use will depend on the severity of the disease to be treated, the general state of the patient's own immune system, the general condition such as age, weight and sex of the patient, the mode of administration of drug, and other treatments administered concomitantly, and so on.

As used herein, the term "subject" refers to a mammal, such as a primate mammal, such as a human. In certain embodiments, the term "subject" refers to a living organism in which an immune response can be elicited. In certain embodiments, the subject (e.g., human) has a RAS G13D mutation-positive tumor, or is at risk of suffering from the above-mentioned disease.

When the terms "for example", "such as", "e.g.", "including", "comprising" or similar expressions are used herein, these terms will not be regarded as terms of limitation, but will be construed to mean "not limiting to" or "and without limitation".

Beneficial effects of the present invention

The present invention provides an epitope peptide of RAS G13D mutant and a T cell receptor (TCR) that specifically recognizes the epitope peptide, a cell and pharmaceutical composition comprising the epitope peptide or TCR, a nucleic acid encoding the epitope peptide or TCR, a vector and host cell for preparing the epitope peptide or TCR, and a method of using the epitope peptide or TCR to treat a subject. The epitope peptide and TCR provided by the present invention can induce an immune response against a tumor containing a RAS G13D mutation and thus treat the above-mentioned tumor in a subject. In addition, the epitope peptide and TCR provided by the present invention are MHC-II-restricted, and the MHC-II restriction is an allele showing predominantly high frequency in the Asia-Pacific population, so it is especially suitable for patients in the Asia-Pacific region. In addition, the MHC-II restriction is also widely distributed in European, American, and Oceanian populations, so it has broad application prospects. Therefore, the present invention provides a novel T cell-based immunotherapy for the treatment of RAS G13D mutation-positive tumors, which has great clinical value.

Embodiments of the present invention will be described in detail below with reference to the drawings and examples, but those skilled in the art will understand that the following drawings and examples are only for illustrating the present invention, rather than limiting the scope of the present invention. Various objects and advantages of the present invention will become apparent to those skilled in the art from the accompanying drawings and the following detailed description of the preferred embodiment.

**Brief Description of the Drawings**

Fig. 1 shows the results of specific release of IFNγ of different TIL clones in Example 5.

Fig. 2 shows the detection results of the B8.2.4 TCR-T specific release of IL2 induced by the G13D peptide presented by HCT116 cells with different HLA-DQ types.

Fig. 3 shows the detection results of the B8.2.4 TCR-T specific release of IL2 induced by the G13D peptide presented by Lovo cells with different HLA-DQ types.

Fig. 4 shows the detection results of the B8.2.4 TCR-T specific release of IL2 induced by the G13D peptide presented by NCI-H1944 cells.

Fig. 5 shows the detection results of the B8.2.4 TCR-T specific release of IL2 induced by the G13D peptide presented by NCI-H1944 cells.

Fig. 6 shows the results of IL2 release after co-culture of antigen-presenting cells loaded with G13D peptides of different lengths and B8.2.4 TCR-T cells in Example 7.

Fig. 7 shows the results of IL2 release after co-culture of antigen-presenting cells loaded with G13D peptides comprising alanine substitutions at different sites and B8.2.4 TCR-T cells in Example 8.

Fig. 8 shows the results of selective killing of Lovo-CIITA-DQA1*05:01/DQB1*0301-Luc cells by B8.2.4TCR-T in Example 9.

Figs. 9A to 9B show the detection results of affinity of B8.2.4TCR-T to RAS G13D mutant in Example 10.

Sequence information

The information on the sequences involved in the present invention is provided in Table 1 below.

Table 1: Sequence information

| SEQ ID NO | Description | Sequence information |
|---|---|---|
| 1 | Nucleotide sequence of RAS G13D-mRNA | CTTGTTCTTTTTGCAGAAGCTCAGAATAAACGCTCAACTTTG GGCCACCATGCCCCGGCAGCTCAGCGCGGCGGCCGCGCTCT TCGCGTCCCTGGCCGTAATTTTGCACGATGGCAGTCAAATG AGAGCAAAAGCATTTCCAGAAACCAGAGATTATTCTCAACC TACTGCAGCAGCAACAGTACAGGACATAAAAAAACCTGTCC AGCAACCAGCTAAGCAAGCACCTCACCAAACTTTAGCAGCA |

(continued)

| SEQ ID NO | Description | Sequence information |
|---|---|---|
| | | AGATTCATGGATGGTCATATCACCTTTCAAACAGCGGCCAC AGTAAAAATTCCAACAACTACCCCAGCGACTACAAAAAACA CTGCAACCACCAGCCCAATTACCTACACCCTGGTCACAACC CAGGCCACACCCAACAACTCACACACAGCTCCTCCAGTTAC TGAAGTTACAGTCGGCCCTAGCTTAGCCCCTTATTCACTGCC ACCCACCATCACCCCACCAGCTCATACAACTGGAACCAGTT CATCAACCGTCAGCCACACAACTGGGAACACCACTCAACCC AGTAACCAGACCACCCTTCCAGCAACTTTATCGATAGCACT GCACAAAGCACAACCGGTCAGAAGCCTGTTCAACCCACCC ATGCCCCAGGAACAACGGCAGCTGCCCACAATACCACCCGC ACAGCTGCACCTGCCTCCACGGTTCCTGGGCCCACCCTTGCA CCTCAGCCATCGTCAGTCAAGACTGGAATTTATCAGGTTCTA AACGGAAGCAGACTCTGTATAAAAGCAGAGATGGGGATAC AGCTGATTGTTCAAGACAAGGAGTCGGTTTTTTCACCTCGGA GATACTTCAACATCGACCCCAACGCAACGCAAGCCTCTGGG AACTGTGGCACCCGAAAATCCAACCTTCTGTTGAATTTTCAG GGCGGATTTGTGAATCTCACATTTACCAAGGATGAAGAATC ATATTATATCAGTGAAGTGGGAGCCTATTTGACCGTCTCAG ATCCAGAGACAATTTACCAAGGAATCAAACATGCGGTGGTG ATGTTCCAGACAGCAGTCGGGCATTCCTTCAAGTGCGTGAG TGAACAGAGCCTCCAGTTGTCAGCCCACCTGCAGGTGAAAA CAACCGATGTCCAACTTCAAGCCTTTGATTTTGAAGATGACC ACTTTGGAAATGTGGATGAGTGCTCGTCTGACTACACA<u>ACT GAATATAAACTTGTGGTAGTTGGAGCTGGC</u>G<u>ATGTAGGCAA GAGTGCCTTGACGATACAGCTAATTCAGAATCATTTTGTGG ACGAATATGATCCAACAATAGAGGATTCCTACAGGAAGCAA GTAGTAATTGATGGAGAAACCTGTCTCTTGGATATTCTCGAC ACAGCAGGTCAAGAGGAGTACAGTGCAATGAGGGACCAGT ACATGAGGACTGGGGAGGGCTTTCTTTGTGTATTTGCCATAA ATAATACTAAATCATTTGAAGATATTCACCATTATAGAGAA CAAATTAAAAGAGTTAAGGACTCTGAAGATGTACCTATGGT CCTAGTAGGAAATAAATGTGATTTGCCTTCTAGAACAGTAG ACACAAAACAGGCTCAGGACTTAGCAAGAAGTTATGGAATT CCTTTTATTGAAACATCAGCAAAGACAAGACAGGGTGTTGA TGATGCCTTCTATACATTAGTTCGAGAAATTCGAAAACATA AAGAAAGATGAGCAAAGATGGTAAAAGAAGAAAAAGA AGTCAAAGACAAAGTGTGTAATTATG</u>ATTGTGCTTCCTGTG ATTGGGGCCATCGTGGTTGGTCTCTGCCTTATGGGTATGGGT GTCTATAAAATCCGCCTAAGGTGTCAATCATCTGGATACCA GAGAATCTAATCCTCTAGAGGATCTGGTTACCACTAAACCA GCCTCAAGAACACCCGAATGGAGTCTCTAAGCTACATAATA CCAACTTACACTTACAAAATGTTGTCCCCCAAAATGTAGCC ATTCGTATCTGCTCCTAATAAAAGAAAGTTTCTTCAC |

(continued)

| SEQ ID NO | Description | Sequence information |
|---|---|---|
| 2 | Nucleotide sequence of TCR Vα | ATGTCACTTTCTAGCCTGCTGAAGGTGGTCACAGCTTCACTG TGGCTAGGACCTGGCATTGCCCAGAAGATAACTCAAACCCA ACCAGGAATGTTCGTGCAGGAAAAGGAGGCTGTGACTCTGG ACTGCACATATGACACCAGTGATCCAAGTTATGGTCTATTCT GGTACAAGCAGCCCAGCAGTGGGGAAATGATTTTTCTTATT TATCAGGGGTCTTATGACCAGCAAAATGCAACAGAAGGTCG CTACTCATTGAATTTCCAGAAGGCAAGAAAATCCGCCAACC TTGTCATCTCCGCTTCACAACTGGGGGACTCAGCAATGTACT TCTGTGCAATGAGAGAGAGGACCGGCACTGCCAGTAAACTC ACCTTTGGGACTGGAACAAGACTTCAGGTCACGCTC |
| 3 | Nucleotide sequence of TCR Vβ | ATGGGAATCAGGCTCCTCTGTCGTGTGGCCTTTTGTTTCCTG GCTGTAGGCCTCGTAGATGTGAAAGTAACCCAGAGCTCGAG ATATCTAGTCAAAAGGACGGGAGAGAAAGTTTTTCTGGAAT GTGTCCAGGATATGGACCATGAAAATATGTTCTGGTATCGA CAAGACCCAGGTCTGGGGCTACGGCTGATCTATTTCTCATAT GATGTTAAAATGAAAGAAAAAGGAGATATTCCTGAGGGGT ACAGTGTCTCTAGAGAGAAGAAGGAGCGCTTCTCCCTGATT CTGGAGTCCGCCAGCACCAACCAGACATCTATGTACCTCTG TGCCAGCAGCCAGACGGTGCCGCCCCAGCATTTTGGTGATG GGACTCGACTCTCCATCCTA |
| 4 | Amino acid sequence of TCRVa | MSLSSLLKVVTASLWLGPGIAQKITQTQPGMFVQEKEAVTLDC TYD<u>TSDPSYG</u>LFWYKQPSSGEMIFLIY<u>QGSYDQQN</u>ATEGRYSL NFQKARKSANLVISASQLGDSAMYFC<u>AMRERTGTASKLT</u>FGT GTRLQVTL |
| 5 | Amino acid sequence of TCR Vβ | MGIRLLCRVAFCFLAVGLVDVKVTQSSRYLVKRTGEKVFLEC VQD<u>MDHEN</u>MFWYRQDPGLGLRLIYF<u>SYDVKM</u>KEKGDIPEGY SVSREKKERFSLILESASTNQTSMYLC<u>ASSQTVPPQH</u>FGDGTRL SIL |
| 6 | Amino acid sequence of TCR CDR1α | TSDPSYG |
| 7 | Amino acid sequence of TCR CDR2α | QGSYDQQN |
| 8 | Amino acid sequence of TCR CDR3α | AMRERTGTASKLT |
| 9 | Amino acid sequence of TCR CDR1β | MDHEN |
| 10 | Amino acid sequence of TCR CDR2β | SYDVKM |
| 11 | Amino acid sequence of TCR CDR3β | ASSQTVPPQH |
| 12 | Amino acid sequence of RAS WT23mer | TEYKLVVVGAGGVGKSALTIQLI |
| 13 | Amino acid sequence of G13D 23mer | TEYKLVVVGAGDVGKSALTIQLI |

(continued)

| SEQ ID NO | Description | Sequence information |
|---|---|---|
| 14 | Epitope peptide core amino acid sequence | VVVGAGDVGKS |
| 15 | Amino acid sequence of G13D-RT1 | KLVVVGAGDVGKSALTIQL |
| 16 | Amino acid sequence of G13D-RT2 | KLVVVGAGDVGKSALTIQ |
| 17 | Amino acid sequence of G13D-RT3 | KLVVVGAGDVGKSALTI |
| 18 | Amino acid sequence of G13D-RT4 | YKLVVVGAGDVGKSALT |
| 19 | Amino acid sequence of G13D-RT5 | KLVVVGAGDVGKSALT |
| 20 | Amino acid sequence of G13D-RT6 | YKLVVVGAGDVGKSAL |
| 21 | Amino acid sequence of G13D-RT7 | KLVVVGAGDVGKSAL |
| 22 | Amino acid sequence of G13D-RT8 | LVVVGAGDVGKSAL |
| 23 | Amino acid sequence of G13D-RT9 | VVVGAGDVGKSAL |
| 24 | Amino acid sequence of G13D-RT10 | VVGAGDVGKSAL |
| 25 | Amino acid sequence of G13D-RT11 | VGAGDVGKSAL |
| 26 | Amino acid sequence of G13D-RT12 | KLVVVGAGDVGKSA |
| 27 | Amino acid sequence of G13D-RT13 | KLVVVGAGDVGKS |
| 28 | Amino acid sequence of G13D-RT14 | KLVVVGAGDVGK |
| 29 | Amino acid sequence of G13D-RT15 | KLVVVGAGDVG |
| 30 | Amino acid sequence of G13D-RT6-A1 | AKLVWGAGDVGKSAL |
| 31 | Amino acid sequence of G 13 D-RT6-A2 | YALV W GA GDV GKSAL |
| 32 | Amino acid sequence of G13D-RT6-A3 | YKAWVGAGDVGKSAL |
| 33 | Amino acid sequence of G13D-RT6-A4 | YKLAWGAGDVGKSAL |
| 34 | Amino acid sequence of G13D-RT6-A5 | YKLVAVGAGDVGKSAL |

(continued)

| SEQ ID NO | Description | Sequence information |
|---|---|---|
| 35 | Amino acid sequence of G13D-RT6-A6 | YKLVVAGAGDVGKSAL |
| 36 | Amino acid sequence of G13D-RT6-A7 | YKLVWAAGDVGKSAL |
| 37 | Amino acid sequence of G13D-RT6-G8 | YKLVVVGGGDVGKSAL |
| 38 | Amino acid sequence of G13D-RT6-A9 | YKLVVVGAADVGKSAL |
| 39 | Amino acid sequence of G13D-RT6-A11 | YKLVWGAGDAGKSAL |
| 40 | Amino acid sequence of G13D-RT6-A12 | YKLVWGAGDVAKSAL |
| 41 | Amino acid sequence of G13D-RT6-A13 | YKLVWGAGDVGASAL |
| 42 | Amino acid sequence of G13D-RT6-A14 | YKLVVVGAGDVGKAAL |
| 43 | Amino acid sequence of G13D-RT6-G15 | YKLVWGAGDVGKSGL |
| 44 | Amino acid sequence of G13D-RT6-A16 | YKLVVVGAGDVGKSAA |
| 45 | Amino acid sequence of murine TCRα constant region | DIQNPEPAVYQLKDPRSQDSTLCLFTDFDSQINVPKTMESGTFIT DKTVLDMKAMDSKSNGAIAWSNQTSFTCQDIFKETNATYPSSD VPCDATLTEKSFETDMNLNFQNLSVMGLRILLLKVAGFNLLMT LRLWSS |
| 46 | Nucleotide sequence of murine TCRα constant region | GACATCCAGAACCCAGAACCTGCTGTGTACCAGTTAAAAGAT CCTCGGTCTCAGGACAGCACCCTCTGCCTGTTCACCGACTTT GACTCCCAAATCAATGTGCCGAAAACCATGGAATCTGGAAC GTTCATCACTGACAAAACTGTGCTGGACATGAAAGCTATGGA TTCCAAGAGCAATGGGGCCATTGCCTGGAGCAACCAGACAA GCTTCACCTGCCAAGATATCTTCAAAGAGACCAACGCCACCT ACCCCAGTTCAGACGTTCCCTGTGATGCCACGTTGACTGAGA AAAGCTTTGAAACAGATATGAACCTAAACTTTCAAAACCTGT CAGTTATGGGACTCCGAATCCTCCTGCTGAAAGTAGCCGGAT TTAACCTGCTCATGACGCTGAGGCTGTGGTCCAGT |
| 47 | Amino acid sequence of murine TCRβ constant region | EDLRNVTPPKVSLFEPSKAEIANKQKATLVCLARGFFPDHVELS WWVNGKEVHSGVSTDPQAYKESNYSYCLSSRLRVSATFWHNP RNHFRCQVQFHGLSEEDKWPEGSPKPVTQNISAEAWGRADCGI TSASYQQGVLSATILYEILLGKATLYAVLVSTLVVMAMVKRKNS |

(continued)

| SEQ ID NO | Description | Sequence information |
|---|---|---|
| 48 | Nucleotide sequence of murine TCRβ constant region | GAGGACCTGCGCAACGTCACCCCACCAAAGGTCAGTTTGTT TGAGCCATCAAAGGCGGAGATCGCCAACAAACAGAAAGCTA CGCTCGTGTGTTTGGCTCGGGGCTTCTTCCCAGACCACGTAG AACTTTCCTGGTGGGTCAATGGAAAGGAGGTTCATTCCGGA GTGTCCACTGATCCCCAAGCGTACAAGGAATCCAACTATAGC TACTGTCTCTCATCTCGGCTCCGGGTGAGTGCGACATTCTGG CATAATCCTCGGAACCACTTTCGATGCCAAGTGCAGTTTCAT GGGTTGAGCGAGGAAGACAAGTGGCCCGAGGGCAGTCCTA AACCAGTCACTCAAAACATAAGCGCCGAGGCATGGGGTAGA GCCGATTGTGGGATTACTAGCGCTTCATACCAACAAGGGGTA TTGAGCGCTACAATTCTTTACGAAATTCTCCTCGGCAAGGCG ACGCTCTACGCCGTACTGGTGTCTACTCTCGTGGTTATGGCA ATGGTGAAACGGAAAAACAGC |
| 49 | Nucleotide sequence of B8.2.4/TRAV+mCa | ATGTCACTTTCTAGCCTGCTGAAGGTGGTCACAGCTTCACTG TGGCTAGGACCTGGCATTGCCCAGAAGATAACTCAAACCCA ACCAGGAATGTTCGTGCAGGAAAAGGAGGCTGTGACTCTGG ACTGCACATATGACACCAGTGATCCAAGTTATGGTCTATTCT GGTACAAGCAGCCCAGCAGTGGGGAAATGATTTTTCTTATT TATCAGGGGTCTTATGACCAGCAAAATGCAACAGAAGGTCG CTACTCATTGAATTTCCAGAAGGCAAGAAAATCCGCCAACC TTGTCATCTCCGCTTCACAACTGGGGGACTCAGCAATGTACT TCTGTGCAATGAGAGAGAGGACCGGCACTGCCAGTAAACTC ACCTTTGGGACTGGAACAAGACTTCAGGTCACGCTCGACAT CCAGAACCCAGAACCTGCTGTGTACCAGTTAAAAGATCCTCG GTCTCAGGACAGCACCCTCTGCCTGTTCACCGACTTTGACTC CCAAATCAATGTGCCGAAAACCATGGAATCTGGAACGTTCAT CACTGACAAAACTGTGCTGGACATGAAAGCTATGGATTCCAA GAGCAATGGGGCCATTGCCTGGAGCAACCAGACAAGCTTCA CCTGCCAAGATATCTTCAAAGAGACCAACGCCACCTACCCCA GTTCAGACGTTCCCTGTGATGCCACGTTGACTGAGAAAAGCT TTGAAACAGATATGAACCTAAACTTTCAAAACCTGTCAGTTA TGGGACTCCGAATCCTCCTGCTGAAAGTAGCCGGATTTAACC TGCTCATGACGCTGAGGCTGTGGTCCAGT |
| 50 | Nucleotide sequence of B8.2.4/TRBV+mCb | ATGGGAATCAGGCTCCTCTGTCGTGTGGCCTTTTGTTTCCTG GCTGTAGGCCTCGTAGATGTGAAAGTAACCCAGAGCTCGAG ATATCTAGTCAAAAGGACGGGAGAGAAAGTTTTTCTGGAAT GTGTCCAGGATATGGACCATGAAAATATGTTCTGGTATCGA CAAGACCCAGGTCTGGGGCTACGGCTGATCTATTTCTCATAT GATGTTAAAATGAAAGAAAAAGGAGATATTCCTGAGGGGT ACAGTGTCTCTAGAGAGAAGAAGGAGCGCTTCTCCCTGATT CTGGAGTCCGCCAGCACCAACCAGACATCTATGTACCTCTG TGCCAGCAGCCAGACGGTGCCGCCCCAGCATTTTGGTGATG GGACTCGACTCTCCATCCTAGAGGACCTGCGCAACGTCACC CCACCAAAGGTCAGTTTGTTTGAGCCATCAAAGGCGGAGAT CGCCAACAAACAGAAAGCTACGCTCGTGTGTTTGGCTCGGG GCTTCTTCCCAGACCACGTAGAACTTTCCTGGTGGGTCAATG GAAAGGAGGTTCATTCCGGAGTGTCCACTGATCCCCAAGCG TACAAGGAATCCAACTATAGCTACTGTCTCTCATCTCGGCTCC |

(continued)

| SEQ ID NO | Description | Sequence information |
|---|---|---|
| | | GGGTGAGTGCGACATTCTGGCATAATCCTCGGAACCACTTTC GATGCCAAGTGCAGTTTCATGGGTTGAGCGAGGAAGACAAG TGGCCCGAGGGCAGTCCTAAACCAGTCACTCAAAACATAAG CGCCGAGGCATGGGGTAGAGCCGATTGTGGGATTACTAGCGC TTCATACCAACAAGGGGTATTGAGCGCTACAATTCTTTACGA AATTCTCCTCGGCAAGGCGACGCTCTACGCCGTACTGGTGTC TACTCTCGTGGTTATGGCAATGGTGAAACGGAAAAACAGC |
| 51 | Amino acid sequence of RAS G13D mutant | MTEYKLVVVGAGDVGKSALTIQLIQNHFVDEYDPTIEDSYRKQ VVIDGETCLLDILDTAGQEEYSAMRDQYMRTGEGFLCVFAINN TKSFEDIHHYREQIKRVKDSEDVPMVLVGNKCDLPSRTVDTKQ AQDLARSYGIPFIETSAKTRQGVDDAFYTLVREIRKHKEKMSK DGKKKKKKSKTKCVIM |
| 52 | Amino acid sequence of WT-RT6 | YKLVWGAGGVGKSAL |
| 53 | Nucleotide sequence of P2A | GCCACCAACTTCAGCCTGCTGAAGCAGGCCGGCGACGTGGA GGAGAACCCCGGCCCC |
| 54 | Amino acid sequence of TCR CDR3β95Q-A | ASSATVPPQH |
| 55 | Amino acid sequence of TCR CDR3β95Q-C | ASSCTVPPQH |
| 56 | Amino acid sequence of TCR CDR3β95Q-D | ASSDTVPPQH |
| 57 | Amino acid sequence of TCR CDR3β95Q-E | ASSETVPPQH |
| 58 | Amino acid sequence of TCR CDR3β95Q-G | ASSGTVPPQH |
| 59 | Amino acid sequence of TCR CDR3β95Q-H | ASSHTVPPQH |
| 60 | Amino acid sequence of TCR CDR3β95Q-I | ASSITVPPQH |
| 61 | Amino acid sequence of TCR CDR3β95Q-L | ASSLTVPPQH |
| 62 | Amino acid sequence of TCR CDR3β95Q-M | ASSMTVPPQH |
| 63 | Amino acid sequence of TCR CDR3β95Q-N | ASSNTVPPQH |
| 64 | Amino acid sequence of TCR CDR3β95Q-S | ASSSTVPPQH |
| 65 | Amino acid sequence of TCR CDR3β95Q-T | ASSTTVPPQH |
| 66 | Amino acid sequence of TCR CDR3β95Q-V | ASSVTVPPQH |
| 67 | Amino acid sequence of TCR CDR3β95Q-W | ASSWTVPPQH |

(continued)

| SEQ ID NO | Description | Sequence information |
|---|---|---|
| 68 | Amino acid sequence of TCR CDR3β95Q-Y | ASSYVPPQH |
| 69 | Amino acid sequence of TCR CDR3β96T-A | ASSQAVPPQH |
| 70 | Amino acid sequence of TCR CDR3β96T-C | ASSQCVPPQH |
| 71 | Amino acid sequence of TCR CDR3β96T-H | ASSQHVPPQH |
| 72 | Amino acid sequence of TCR CDR3β96T-K | ASSQKVPPQH |
| 73 | Amino acid sequence of TCR CDR3β96T-N | ASSQNVPPQH |
| 74 | Amino acid sequence of TCR CDR3β96T-S | ASSQSVPPQH |
| 75 | Amino acid sequence of TCR CDR3β96T-V | ASSQVVPPQH |
| 76 | Amino acid sequence of TCR CDR3β96T-W | ASSQWVPPQH |
| 77 | Amino acid sequence of TCR CDR3β97V-I | ASSQTIPPQH |
| 78 | Amino acid sequence of TCR CDR3β97V-S | ASSQTSPPQH |
| 79 | Amino acid sequence of TCR CDR3β97V-T | ASSQTTPPQH |
| 80 | Amino acid sequence of TCR CDR3β98P-C | ASSQTVCPQH |
| 81 | Amino acid sequence of TCR CDR3β98P-D | ASSQTVDPQH |
| 82 | Amino acid sequence of TCR CDR3β98P-E | ASSQTVEPQH |
| 83 | Amino acid sequence of TCR CDR3β98P-F | ASSQTVFPQH |
| 84 | Amino acid sequence of TCR CDR3β98P-G | ASSQTVGPQH |
| 85 | Amino acid sequence of TCR CDR3β98P-H | ASSQTVHPQH |
| 86 | Amino acid sequence of TCR CDR3β98P-L | ASSQTVLPQH |
| 87 | Amino acid sequence of TCR CDR3β98P-M | ASSQTVMPQH |
| 88 | Amino acid sequence of TCR CDR3β98P-R | ASSQTVRPQH |

(continued)

| SEQ ID NO | Description | Sequence information |
|---|---|---|
| 89 | Amino acid sequence of TCR CDR3β98P-S | ASSQTVSPQH |
| 90 | Amino acid sequence of TCR CDR3β98P-V | ASSQTWPQH |
| 91 | Amino acid sequence of TCR CDR3β98P-W | ASSQTVWPQH |
| 92 | General formula of TCR CDR3β | ASSX$_1$X$_2$X$_3$X$_4$PQH |
| 93 | Amino acid sequence of FR1α | MSLSSLLKVVTASLWLGPGIAQKITQTQPGMFVQEKEAVTLDCTYD |
| 94 | Amino acid sequence of FR2α | LFWYKQPSSGEMIFLIY |
| 95 | Amino acid sequence of FR3α | ATEGRYSLNFQKARKSANLVISASQLGDSAMYFC |
| 96 | Amino acid sequence of FR4α | FGTGTRLQVTL |
| 97 | Amino acid sequence of FR1β | MGIRLLCRVAFCFLAVGLVDVKVTQSSRYLVKRTGEKVFLECVQD |
| 98 | Amino acid sequence of FR2β | MFWYRQDPGLGLRLIYF |
| 99 | Amino acid sequence of FR3β | KEKGDIPEGYSVSREKKERFSLILESASTNQTSMYLC |
| 100 | Amino acid sequence of FR4β | FGDGTRLSIL |

## EXAMPLES

The present invention will now be described with reference to the following examples, which are intended to illustrate the present invention, but not to limit it.

Unless otherwise specified, the molecular biology experiment methods and immunoassay methods used in the present invention are basically referred to the methods described in J. Sambrook et al., Molecular Cloning: A Laboratory Manual, 2nd Edition, Cold Spring Harbor Laboratory Press, 1989, and F. M. Ausubel et al., Short protocols in Molecular Biology, 3rd Edition, John Wiley & Sons, Inc., 1995; restriction enzymes were used in accordance with the conditions recommended by the product manufacturer. Those skilled in the art understand that the examples describe the present invention by way of example and are not intended to limit the scope of the claimed invention.

Example 1: Culture and expansion of tumor-infiltrating T cells

Surgically resected tumor samples from patients with colorectal cancer containing RAS mutation G13D were minced with a shell scalpel to 2mm to 4mm tumor mass, washed twice with DPBS solution, cultured in 24-well plates, cultured in TIL medium containing IL2 (6000IU/ml), human AB serum (2%), Hepes (25mM), Xvivo15. Every 2-3 days, half of the medium was replaced, and when the tumor-infiltrating T lymphocytes (TIL) grow to cover 60-80% of the plate (containing about 0.5-3.0×10$^6$ TILs), the TILs were harvested and stored in CS10 freezing solution.

The TIL cells can be expanded by co-culturing with peripheral blood mononuclear cells derived from different donors (pooled PBMC, donors > 3) irradiated by γ-ray at a ratio of 1:30-1:200, cultured in T175 culture flasks, and each of the culture flasks contained no more than 1×10$^8$ cells (the culture medium was TIL medium supplemented with 10ng/ml OKT3). After 3 days of culture, half of the medium was replaced (Xvivo15 medium containing 3000IU/ml IL2, 2% human AB serum); after 7 days of culture, the cells were washed once, then the medium was replaced, and passaged at $10\times10^6$/ml; and the cells were harvested and frozen after 10-14 days of culture.

Example 2: Preparation of RAS G13D antigen mRNA

For the sequence containing RAS G13D mutation, the vector UTR-LAMP3 Lumenal-KRAS^G13D-LMP3 Sorting-UTR was constructed according to the following design, the sequence was set forth in SEQ ID NO: 1 in Table 1, in which KRAS was marked with single underline, and G13D mutation was marked with double underline. The above sequence was synthesized and cloned into pcDNA3.1 vector, and the mRNA transcription was prepared in vitro using T7 promoter (mMESSAGE mMACHINE T7 Transcription Kit, Thermofisher), and the mRNA was stored at -80°C.

Example 3: Screening by antigen-presenting cell

DC (dendritic cell) maturation: autologous peripheral blood CD14-positive cells from patients were isolated with MACS CD14 Isolation Kit, and cultured in AIM-V medium containing IL4 (1000IU/ml), GM-CSF (1000IU/ml) and 1% human AB serum. The culture medium was replaced with fresh medium on the third day, and cryopreservation in CS10 freezing solution was carried out 5-6 days after culture.

LCL (lymphoblastoid cell line) induction: $5\times10^6$ peripheral blood mononuclear cells from patients were resuspended in RPMI1640 medium containing 10% fetal bovine serum, and added with B95.8 supernatant of cell culture medium which contains EBV, the induction was generally completed within 14-30 days, half of the medium was replaced every 7 days during the induction period, and the induced LCL cells were expanded and cryopreserved.

After recovery, the TIL cells were cultured for at least 48 hours for screening The DC cells or LCL cells, referred to as antigen-presenting cells (APCs), were transfected with KRAS G13D mRNA using a Neon electroporator, in which APCs were resuspended in electroporation solution to $1\times10^7$/ml, 100 $\mu$l of the cells was added with 5-8$\mu$g of mRNA for each electroporation (1500V, 30ms, 1pulse). Transfected APCs can be used next day after culturing. In a 96-well U-bottom plate, 1-2$\times10^5$ TIL cells and $0.5\times10^5$ DC cells or $4\times10^5$ LCL cells electroporated were added and cultured in Xvivo15 medium, the cell culture supernatant was collected after 16 hours of culture, and IFN$\gamma$ release in the supernatant was determined by Human IFN$\gamma$ Flex Set. Patient B8 had metastatic colorectal cancer, the tumor had KRAS^G13D mutation, and the TIL screening results were as follows:

Table 2: TIL screening results

| TIL Fraction | IFN$\gamma$-Ctrl | IFN$\gamma$-G13D | Relative change |
|---|---|---|---|
| B8.1 | 726 | 755 | 1.0 |
| B8.2 | 744 | 1877 | 2.5 |
| B8.3 | 1086 | 3559 | 3.3 |
| B8.4 | 14464 | 15413 | 1.1 |
| B8.5 | 776 | 837 | 1.1 |
| B8.6 | 1188 | 2172 | 1.8 |
| B8.7 | 3146 | 3197 | 1.0 |
| B8.8 | 1226 | 1198 | 0.9 |
| Ctrl: TIL co-cultured with non-electroporated APC<br>G13D: TIL co-cultured with G13D mRNA-electroporated APC | | | |

Example 4: Sorting and expansion of TIL

The TIL cells stimulated by APCs were sorted by flow cytometry, and $1\times10^6$ TIL cells were resuspended in the flow buffer (1% human AB serum, 2mM EDTA in DPBS solution), added with CD3/CD137 antibody and PI (propidium iodide solution), incubated at 4°C for 1 hour, then washed twice with flow buffer, and sorted with BD FACSAiraII flow sorter. The sorted population was PI-negative, CD3-positive and CD137-positive cell population. The sorted cells were stored in RPMI1640 medium containing 10% human AB serum, and placed on ice. The collected sorted cells (CD3 and CD137+) were centrifuged at 300g for 10 minutes at 4°C to remove 80% of the preservation solution, washed twice with DPBS solution, then resuspended in DPBS, and subjected to $10\times$Genomics single-cell sequencing.

Example 5: Screening of TCR that can recognize KRAS$^{G13D}$

The TCR clone in the single-cell sequencing was subjected to gene synthesis according to the sequence of TRAVmCa-P2A-TRBVmCb, wherein TRAV was the α chain variable region of TCR, mCa was the murine TCRα constant region (its amino acid sequence and nucleotide sequence were set forth in SEQ ID NO:45 and 46, respectively), TRBV was the β chain variable region of TCR, mCb was the murine TCRβ constant region (its amino acid sequence and nucleotide sequence were set forth in SEQ ID NO: 47 and 48, respectively), and P2A was a self-cleaving peptide (its nucleotide sequence was set forth in SEQ ID NO: 53); the above sequence was cloned into a lentiviral shuttle vector (GV401); the transfer and package vectors were transiently transfected into 293T cells according to the standard lentiviral vector packaging method, and the culture supernatant was collected, then the culture supernatant contained the lentiviral vector expressing the TCR. T cells from healthy donors were activated in OKT3/15E8 antibody-coated 6-well plates for 24 hours, transduced with TCR-containing lentiviral vector and cultured for 6-8 days for TCR screening (the transduced T cells were collected, washed with FACS buffer, and $1 \times 10^6$ engineered T cells were stained by adding an antibody recognizing murine TCRβ constant region to detect the expression of recombinant TCR), thus, the T cells engineered by recombinant TCR were obtained.

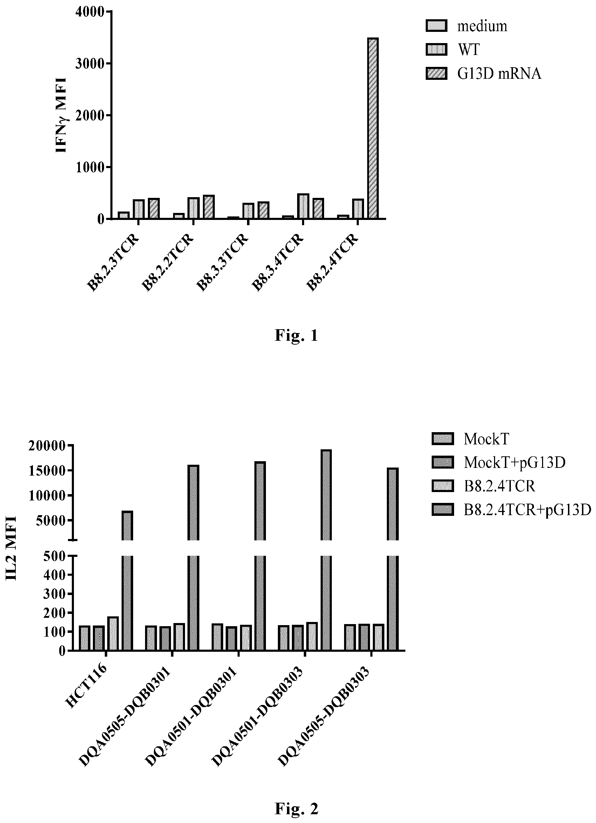

According to the electroporation method in "Example 3", the autologous LCL cells from Patient B8 were transiently transfected with KRAS$^{G13D}$ mRNA, and cultured overnight; the T cells engineered by recombinant TCR and the electroporated LCL cells were inoculated at a ratio of $1 \times 10^5 : 1 \times 10^5$ and co-cultured in a 96-well U-bottom plate, the specific release of IFNγ in the supernatant was detected, and the results were shown in Fig. 1. The screening results showed that the clone numbered as B8.2.4TCR could specifically recognize the KRAS G13D point mutation but did not recognize wild-type RAS. The sequences of B8.2.4 TCR were shown in the table below:

Table 3. Sequences of B8.2.4 TCR

| Sequence type | B8.2.4 TCR Vα region TRAV/DV4*01-J44*01 | B8.2.4 TCR Vβ region TRBV28*01-D1*01-11-5*01 |
|---|---|---|
| Variable region | SEQ ID NO: 4 | SEQ ID NO: 5 |
| CDR1 | SEQ ID NO: 6 | SEQ ID NO: 9 |
| CDR2 | SEQ ID NO: 7 | SEQ ID NO: 10 |
| CDR3 | SEQ ID NO: 8 | SEQ ID NO: 11 |

Example 6: Detection of B8.2.4 TCR HLA restriction

T cells expressing recombinant B8.2.4 TCR (hereinafter referred to as B8.2.4 TCR-T) were prepared according to the method described in Example 5, which comprised transducing the lentiviral vector containing a nucleotide sequence encoding the recombinant TCR into T cells from healthy donors, wherein the nucleotide sequences of the α chain (TRAVmCa) and β chain (TRBVmCb) of the recombinant TCR were set forth in SEQ ID NO: 49 and 50, respectively. HLA restriction of the B8.2.4 TCR was determined by the method described below.

HCT116 and Lovo cell lines (colorectal cancer) contained KRAS G13D heterozygous mutation; NCI-H1944 cell line (lung cancer) contained KRAS G13D heterozygous mutation; and their HLA-DQ types were shown in Table 4 below:

Table 4. HLA-DQ types of different cells

| Cell line | HLA-DQ | Alleles |
|---|---|---|
| HCT116 | DQA1 | 05:01/05:05 |
| | DQB1 | 02:01/03:19 |
| Lovo | DQA1 | 01:02/01:03 |
| | DQB1 | 06:03/06:04 |
| NCI-H 1944 | DQA1 | 03:01/05:05 |
| | DQB1 | 03:01/03:02 |

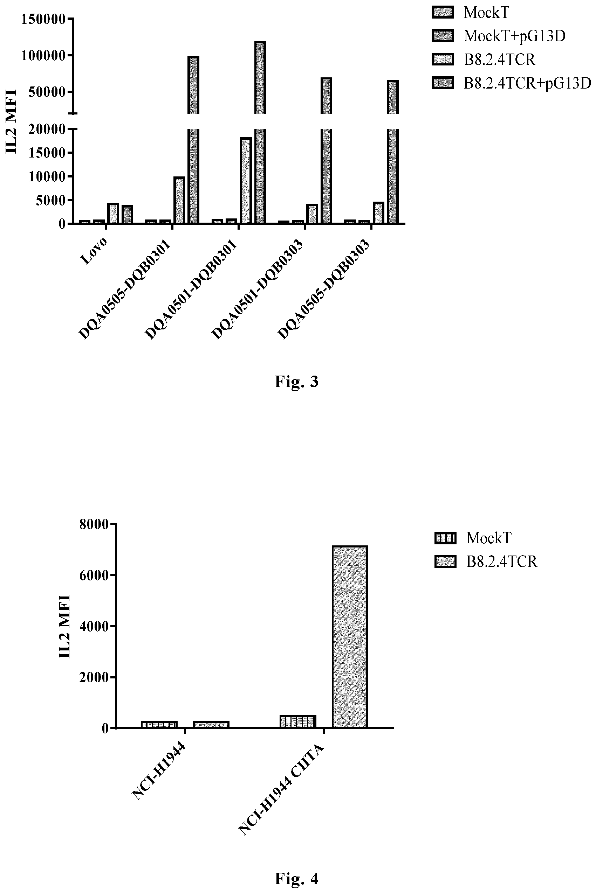

The CIITA gene (Genebank ID: 4261) was overexpressed in HCT116, Lovo, NCI-H1944 cells to construct SW620-CIITA or CFPAC1-CIITA cell lines, and HLA-DQA1*05:01/05:05 and HLA-DQB 1*03:01/03:03/03:19 were overexpressed to construct HLA-DQ genotyped cell lines (see, Table 5 for HLA-DQ combinations). The above cells were collected and resuspended in RPMI1640 medium, and added with 10 μg/ml RAS WT 23mer (its sequence was set forth in SEQ ID NO: 12) or G13D 23mer (its sequence was set forth in SEQ ID NO: 13) peptide (synthesized, added with DMSO to dissolve). After incubating at 37°C for 2 hours, washing twice with DPBS solution, the peptide-loaded antigen-presenting cells and the B8.2.4 TCR-T cells at a ratio of $2\times10^4$:$2\times10^4$ were co-cultured overnight in RPMI1640 medium containing 2% fetal bovine serum, the release of IL2 or IFNγ in the supernatant was measured, and the results were shown in Figs. 2 to 5.

Table 5. HLA-DQ combinations

| HLA-DQ combination | HLA-DQA1 | HLA-DQB1 |
|---|---|---|
| Combination 1 | DQA1*05:01 | DQB1*03:01 |
| Combination 2 | DQA1*05:01 | DQB1*03:03 |
| Combination 3 | DQA1*05:05 | DQB1*03:01 |
| Combination 4 | DQA1*05:05 | DQB1*03:03 |
| Combination 5 | DQA1*05:01 | DQB1*03:19 |
| Combination 6 | DQA1*05:05 | DQB1*03:19 |

The results showed that the B8.2.4 TCR could recognize the RAS G13D peptides exogenously presented by HCT116 cells (HLA-DQA1*05:01 or HLA-DQA1*05:05/DQB1*03:19) and HCT116-DQ (overexpressing the top four DQA1/DQB1 combinations as shown in Table 5), but did not recognize endogenously presented RAS G13D epitope. The B8.2.4 TCR could recognize the RAS G13D peptide exogenously presented by Lovo cells overexpressing HLA-DQ gene combinations (see, Table 5), and at the same time recognize the RAS G13D epitope endogenously presented by the HLA-DQ combination HLA-DQA1*05:01/05:05 and HLA-DQB1*0301. The B8.2.4 TCR could recognize the RAS G13D epitope endogenously presented by NCI-H1944 cells (see, Table 4 for HLA-DQ).

The HCT116 colorectal cancer cells could have a deletion or mutation in the antigen processing and presentation pathway, resulting in that endogenous RAS G13D epitope could not be loaded in HLA-DQ complex. In the Lovo colorectal cancer cells, overexpression of HLA-DQA1*05:01/05:05 combined with HLA-DQB1*0301 could effectively present endogenous RAS G13D epitope; while HLA-DQA1*05:01/05:05 combined with HLA-DQB1*0303 showed a low efficiency of endogenous presentation of RAS G13D epitope, but could exogenously present RAS G13D with high efficiency.

In summary, the results indicated that the B8.2.4 TCR could efficiently recognize the RAS G13D epitope presented by HLA-DQA1*05:01 or DQA1*05:05 in combination with HLA-DQB1*03:01, and could also recognize the RAS G13D epitope presented by HLA-DQA1*05 :01 or DQA1*05:05 in combination with HLA-DQB1*03:03 with a relatively lower efficiency.

Example 7: Determination of presenting epitope of RAS G13D

Peptides as shown in the following table were synthesized based on the peptide with length of 23 (SEQ ID NO: 13) containing the G13D mutation site and presented by autologous LCL cells, and the release of IFNγ was measured to screen the RAS G13D epitope recognized by B8.2.4 TCR.

Table 6. RAS G13D epitope screening table

| Peptide No. | Peptide length | Amino acid sequence |
|---|---|---|
| G13D-RT1 | 19 | KLVVVGAGDVGKSALTIQL |
| G13D-RT2 | 18 | KLVVVGAGDVGKSALTTQ |
| G13D-RT3 | 17 | KLVVVGAGDVGKSALTI |
| G13D-RT4 | 17 | YKLVVVGAGDVGKSAT T |
| G13D-RT5 | 16 | KLVVVGAGDVGKSALT |
| G13D-RT6 | 16 | YKLVVVGAGDVGKSAL |
| G13D-RT7 | 15 | KLVVVGAGDVGKSAT |

(continued)

| Peptide No. | Peptide length | Amino acid sequence |
|---|---|---|
| G13D-RT8 | 14 | LVVVGAGDVGKSAL |
| G13D-RT9 | 13 | VVVGAGDVGKSAL |
| G13D-RT10 | 12 | VVGAGDVGKSAL |
| G13D-RT11 | 11 | VGAGDVGKSAL |
| G13D-RT12 | 14 | KLVVVGAGDVGKSA |
| G13D-RT13 | 13 | KLVVVGAGDVGKS |
| G13D-RT14 | 12 | KLVVVGAGDVGK |
| G13D-RT15 | 11 | KLVVVGAGDVG |

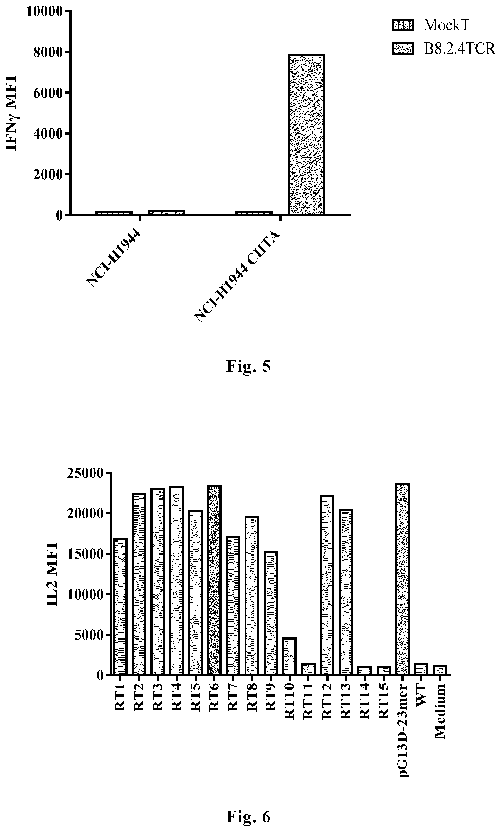

After the above peptides were synthesized, DMSO was added to dissolve them, the autologous LCL cells from Patient B8 were resuspended in RPMI1640 medium, added with the above peptides to a final concentration of 1 μg/ml, incubated for 2 hours, washed twice with DPBS solution, and resuspended in RPMI1640 medium with 2% fetal bovine serum to $2\times10^5$/ml; the antigen-presenting cells loaded with peptides and the B8.2.4 TCR-T cells at a ratio of $2\times10^4$:$2\times10^4$ were co-cultured overnight, and the release of IFNγ in supernatant was measured; the results were shown in Fig. 6.

The above results showed that the G13D-RT6 peptide (SEQ ID NO: 20) could most effectively induce the release of IL2 of B8.2.4 TCR-T, so the RAS G13D epitope presented by the HLA-DQ combinations (see Table 5) was G13D-RT6 peptide, wherein the core sequence of the RAS G13D epitope was VVVGAGDVGKS (SEQ ID NO: 14). The above results also indicated that the peptide comprising the amino acid residues at positions 7-17 of RAS G13D mutant could effectively activate T lymphocytes, thereby inducing an immune response against tumors with RAS G13D mutation.

Example 8: Determination of key amino acids involved in epitope presentation in RAS G13D epitope via alanine scanning

By performing one by one alanine replacement in the RAS G13D epitope peptide, the key amino acids involved in antigen presentation in the RAS G13D epitope could be screened out. The peptides after alanine mutation (mutated amino acid was underlined) were shown in the following table:

Table 7. Alanine scanning of RAS G13D epitope

| Peptide No. | Mutated amino acid | Amino acid sequence |
|---|---|---|
| G13D-RT6-A1 | pY4A | AKLVWGAGDVGKSAL |
| G13D-RT6-A2 | pK5A | YALWVGAGDVGKSAL |
| G13D-RT6-A3 | pL6A | YKAVVGAGDVGKSAL |
| G13D-RT6-A4 | pV7A | YKLAVVGAGDVGKSAL |
| G13D-RT6-A5 | pV8A | YKLVAVGAGDVGKSAL |
| G13D-RT6-A6 | pV9A | YKLVVAGAGDVGKSAL |
| G13D-RT6-A7 | pG10A | YKLWVAAGDVGKSAL |
| G13D-RT6-G8 | pA11G | YKLVWGGGDVGKSAL |
| G13D-RT6-A9 | pG12A | YKLVVVGAADVGKSAL |
| G13D-RT6-A11 | pV14A | YKLVVVGAGDAGKSAL |
| G13D-RT6-A12 | pG15A | YKLVVVGAGDVAKSAL |
| G13D-RT6-A13 | pK16A | YKLVVVGAGDVGASAL |
| G13D-RT6-A14 | pS17A | YKLWVGAGDVGKAAL |
| G13D-RT6-G15 | pA18G | YKLVWGAGDVGKSGL |
| G13D-RT6-A16 | pL19A | YKLWVGAGDVGKSAA |

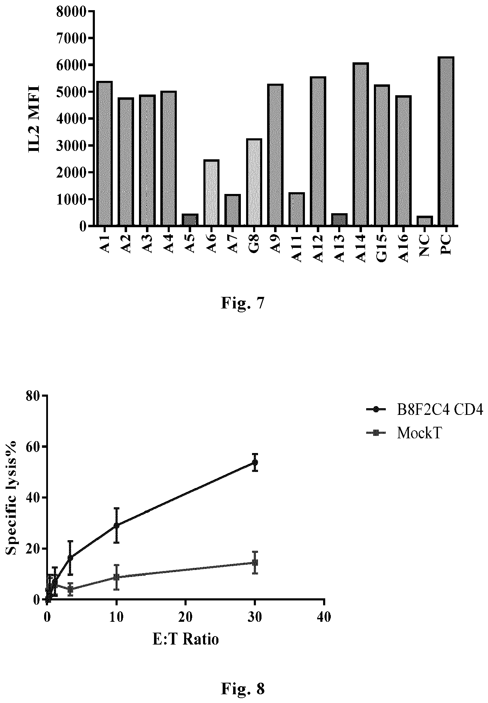

After the above peptides were synthesized, IFNγ or IL2 in the supernatant after the co-culture of antigen-presenting cells and the B8.2.4 TCR-T was measured according to the method in Example 7, and the results were shown in Fig. 7. The above results indicated that the amino acids at positions p8V, p10G, p14V, and p16K in the RAS G13D peptide were most important for the recognition of B8.2.4 TCR, while the amino acids at positions p9V and p11G were less important for the recognition of B8.2.4 TCR.

Example 9: Tumor cell lysis determination of B8.2.4 TCR-T

Lovo-CIITA-DQA1*05:01/DQB1*0301-Luc (expressing Firefly luciferase) cells were resuspended in RPMI1640 medium containing 2% FBS, inoculated in a 96-well plate at $2 \times 10^4$/well, and added with Mock-T and B8.2.4 TCR-CD4+T at E:T ratios of 10, 3, 1, 0.3 and 0.1; and a control without T cells (negative control well RLU) was set; after co-incubating for 24 hours, 100μl of One-Glo luciferase substrate was added to each well, and Luminescence (Relative light unit, RLU) was read.

$$\text{Killing rate} = 1 - (\text{Test well RLU} / \text{Negative control well RLU})$$

The results were shown in Fig. 8. The results showed that the B8.2.4 TCR-T could kill tumor cells in a dose-dependent manner, and was CD4-dependent.

Example 10: Functional avidity determination of B8.2.4 TCR

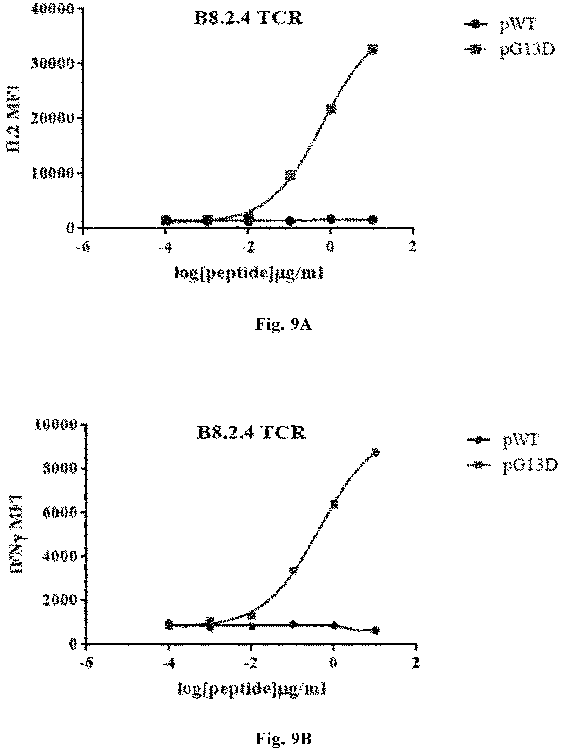

The autologous LCL cells from Patient B8 were loaded with RAS G13D-RT6 peptide (SEQ ID NO:20) and the corresponding wild-type peptide (SEQ ID NO:52) at different concentrations (10 μg/ml, 1 μg/ml, 0.1 μg/ml, 0.01μg/ml, 0.001μg/ml), respectively, cultured at 37°C for 2 hours, and washed twice with DPBS solution, and the antigen-presenting cells and the B8.2.4 TCR-T cells at ratio of $2 \times 10^4 : 2 \times 10^4$ were co-cultured overnight in RPMI1640 medium containing 2% fetal bovine serum, and the release of IL2 and IFNγ in the supernatant was measured. The results were shown in Figs. 9A to 9B. The results showed that B8.2.4 TCR-T could specifically recognize the RAS G13D mutant peptide within the tested concentration range, and could release IFNγ and IL2, in which it could still efficiently recognize the RAS G13D mutant peptide when the antigen peptide concentration was 0.01 μg/ml, indicating that the T cells expressing the TCR had high affinity and high specificity for the RAS G13D mutant.

Example 11. Affinity maturation of B8.2.4 TCR

According to the table below, point mutations were performed on B8.2.4TCR Vα and Vβ, and a lentiviral vector shuttle plasmid encoding TCR mutant was constructed, which is subjected to standard lentiviral vector package in 293T cells, and then the function screening of B8.2.4TCR mutant was carried out. The corresponding positions to be mutated were shown in Table 8, wherein the amino acid sites were determined according to the IMGT TCR numbering.

Table 8. Amino acid sites to be mutated

| Mutation region | Amino acid at mutation position |
|---|---|
| Vα-CDR3 | 97R |
| Vβ-CDR3 | 95Q, 96T, 97V, 98P |

TCR CDR3 mutant screening

The TCR mutant (having a mutation present in CDR3 region) lentiviral vector was transduced into Jurkat-NFAT-Luc cell line, and Lovo-DPA0301:DPB0501 cells loaded with KRAS G13D antigen (G13D-RT6, SEQ ID NO: 20) were used as antigen-presenting cells (referred to as APCs), $2 \times 10^4$ of the TCR-T and $2 \times 10^4$ of the antigen-loaded APCs were co-cultured for 16-24 hours, added with ONE Glo Luciferase to detect the fluorescent signal expression, then TCR Mut RLU/WT RLU (ratio of RLU signal value of TCR mutant to RLU signal value of wild-type B8.2.4TCR) was calculated, and the results were shown in Table 9.

Table 9. TCR Mut RLU/WT RLU of each mutant

| TCR Clone | TCR Mut RLU/WT RLU | mTCRβ% |
|---|---|---|
| α97R-A | 0.13 | 47.2% |
| α97R-C | 0.27 | 63.2% |
| α97R-D | 0.17 | 61.7% |
| α97R-E | 0.16 | 67.3% |
| α97R-F | 0.21 | 81% |
| α97R-G | 0.16 | 77.5% |
| α97R-H | 0.13 | 66.2% |
| α97R-I | 0.16 | 74.9% |
| α97R-K | 0.24 | 63.8% |
| α97R-L | 0.19 | 78% |
| α97R-M | 0.17 | 71.1% |
| α97R-N | 0.16 | 69.9% |
| α97R-P | 0.21 | 52.5% |
| α97R-Q | 0.16 | 69.1% |
| α97R-S | 0.22 | 52% |
| α97R-T | 0.23 | 73% |
| α97R-V | 0.16 | 76.7% |
| α97R-W | 0.18 | 80.2% |
| α97R-Y | 0.16 | 80% |
| β950-A | 0.86 | 74.9% |
| β95Q-C | 1.14 | 82.1% |
| β95Q-D | 0.60 | 77.1% |
| β95Q-E | 0.81 | 36.5% |
| β95Q-F | 0.34 | 80% |
| β95Q-G | 1.03 | 76.6% |
| β95Q-H | 0.68 | 69.2% |
| β95Q-I | 0.74 | 75.6% |
| β95Q-K | 0.30 | 73.7% |
| β95Q-L | 0.64 | 68.6% |
| β95Q-M | 0.99 | 82.1% |
| β95Q-N | 0.60 | 75.1% |
| β95Q-P | 0.21 | 76.8% |
| β95Q-R | 0.43 | 73.6% |
| β95Q-S | 0.61 | 77.3% |
| β95Q-T | 0.59 | 66.8% |
| β95Q-V | 0.76 | 62.7% |
| β95Q-W | 0.88 | 78.2% |
| β95Q-Y | 0.92 | 78.4% |

(continued)

| TCR Clone | TCR Mut RLU/WT RLU | mTCRβ% |
|---|---|---|
| β96T-A | 0.77 | 76.4% |
| β96T-C | 0.78 | 82.3% |
| β96T-D | 0.28 | 73.8% |
| β96T-E | 0.06 | 73.8% |
| β96T-F | 0.42 | 76.9% |
| β96T-G | 0.05 | 55.3% |
| β96T-H | 0.82 | 76.2% |
| β96T-I | 0.41 | 64.6% |
| β96T-K | 0.84 | 72% |
| p96T-L | 0.31 | 73.1% |
| β96T-M | 0.35 | 67.9% |
| β96T-N | 0.82 | 74.1% |
| β96T-P | 0.39 | 46.6% |
| β96T-R | 0.49 | 69.2% |
| β96T-Q | 0.57 | 81.5% |
| β96T-S | 0.80 | 61.9% |
| β96T-V | 0.97 | 54.5% |
| β96T-W | 0.64 | 67.4% |
| β96T-Y | 0.40 | 69.9% |
| β97V-A | 0.56 | 71.3% |
| β97V-C | 0.37 | 52.6% |
| β97V-D | 0.21 | 65.6% |
| β97V-E | 0.31 | 78.4% |
| β97V-F | 0.31 | 77.2% |
| β97V-G | 0.26 | 74.3% |
| β97V-H | 0.44 | 53.6% |
| β97V-I | 0.61 | 69.9% |
| β97V-K | 0.48 | 65.5% |
| β97V-L | 0.58 | 63.8% |
| β97V-M | 0.35 | 28% |
| β97V-N | 0.26 | 52.4% |
| β97V-P | 0.32 | 53.4% |
| β97V-K | 0.37 | 55.2% |
| β97V-Q | 0.30 | 74.5% |
| β97V-S | 0.78 | 72.2% |
| β97V-T | 0.98 | 64.7% |
| β97V-W | 0.52 | 74.5% |
| β97V-Y | 0.54 | 36.9% |

(continued)

| TCR Clone | TCR Mut RLU/WT RLU | mTCRβ% |
|-----------|--------------------|--------|
| β98P-A | 0.50 | 67.6% |
| β98P-C | 1.11 | 80.7% |
| β98P-D | 0.80 | 82% |
| β98P-E | 0.99 | 60.8% |
| β98P-F | 0.96 | 82.5% |
| β98P-G | 0.73 | 81.5% |
| β98P-H | 0.60 | 72.9% |
| β98P-I | 0.33 | 66.4% |
| β98P-K | 0.47 | 63.8% |
| β98P-L | 0.67 | 72.5% |
| β98P-M | 0.83 | 61.2% |
| β98P-N | 0.41 | 75.8% |
| β98P-R | 0.73 | 78% |
| β98P-Q | 0.45 | 67.8% |
| β98P-S | 0.94 | 76.3% |
| β98P-T | 0.47 | 54% |
| β98P-V | 0.63 | 77.2% |
| β98P-W | 0.81 | 81% |
| β98P-Y | 0.25 | 54% |

The above results showed that:

1) The results of mTCRβ expression detection showed that all TCR mutants could be expressed on the surface of Jurkat-NFAT-Luc cells;

2) In the B8.2.4TCR CDRα3 and CDRβ3 mutation regions, the amino acid position that had an important impact on TCR specificity was CDRα3-97R; and after the amino acid at the position was mutated, most mutants could not effectively recognize KRAS G13D antigen peptide presented by the antigen-presenting cells;

3) TCR CDRα3 and CDRβ3 mutants with activity (mutants that maintained about 60% or more the RLU signal value of the wild-type B8.2.4 TCR) were shown in Table 10, in which the preferred TCR mutants (mutants that maintained ≥80% the RLU signal value of the wild-type B8.2.4 TCR) were underlined.

Table 10. Active TCR CDRα3 and CDRβ3 mutants

| TCR region | Mutant amino acid position | Active TCR mutant |
|------------|---------------------------|-------------------|
| CDRα3 | 97R | None |
| CDRβ3 | 95Q | A, C, D, E, G, H, I, L, M, N, S, T, V, W, Y |
| | 96T | A, C, H, K, N, S, V, W |
| | 97V | I, S, T |
| | 98P | C, D, E, F, G, H, L, M, R, S, V, W |

Although the specific models of the present invention have been described in detail, those skilled in the art will understand that: according to all the teachings that have been disclosed, various modifications and changes can be made to the details, and these changes are all within the protection scope of the present invention. The full scope of the present invention is given by the appended claims and any equivalents thereof.

**Claims**

1. An isolated epitope peptide or variant thereof, wherein the epitope peptide consists of 11-30 (e.g., 30, 29, 28, 27, 26, 25, 24, 23, 22, 21, 20, 19, 18, 17, 16, 15, 14, 13, 12, or 11) contiguous amino acid residues of a RAS G13D mutant, and comprises the amino acid residues at positions 7-17 of the RAS G13D mutant;

   the variant differs from the epitope peptide from which it is derived only by a substitution of one or several (e.g., 1, 2 or 3) amino acid residues, and does not comprise an amino acid substitution at positions corresponding to the amino acid positions 8, 10, 13, 14 and 16 of the RAS G13D mutant, and retains a biological function of the epitope peptide from which it is derived;
   preferably, the epitope peptide consists of 11-25 (e.g., 11-20, 11-19, or 11-16) contiguous amino acid residues of the RAS G13D mutant.

2. The variant according to claim 1, which does not comprise an amino acid substitution at positions corresponding to the amino acid positions 8, 9, 10, 11, 13, 14 and 16 of the RAS G13D mutant, and retains a biological function of the epitope peptide from which it is derived.

3. The epitope peptide or variant thereof according to claim 1 or 2, wherein the epitope peptide or variant thereof can be presented by an MHC-II molecule, and the epitope peptide or variant thereof associated with the MHC-II molecule is capable of being recognized by a T cell, for example, recognized by an antigen-specific T cell receptor on the T cell;

   preferably, the MHC-II molecule is HLA-DQ;
   preferably, the HLA-DQ comprises one selected from the group consisting of HLA-DQB1*0301, HLA-DQB1 *0303, HLA-DQB1*0319, HLA-DQB1 *0201, HLA-DQB1 *0603, HLA-DQB1*0604, and HLA-DQB1 *0302; preferably, the HLA-DQ comprises one selected from the group consisting of HLA-DQB1*0301, HLA-DQB1 *0303, and HLA-DQB1*0319;
   preferably, the HLA-DQ comprises one selected from the group consisting of HLA-DQA1*0501, HLA-DQA1*0505, HLA-DQA1*0102, HLA-DQA1*0103, and HLA-DQA1*0301; preferably, the HLA-DQ comprises one selected from the group consisting of HLA-DQA1*0501 and HLA-DQA1 *0505;
   preferably, the HLA-DQ comprises one selected from the group consisting of HLA-DQB1*0301, HLA-DQB1*0319 or HLA-DQB1*0303, and further comprises one selected from the group consisting of HLA-DQA1*0501 or DQA1*0505.

4. The epitope peptide or variant thereof according to any one of claims 1-3, wherein the amino acid residues at positions 7-17 of the RAS G13D mutant have a sequence as set forth in SEQ ID NO: 14.

5. The epitope peptide or variant thereof according to any one of claims 1-4, wherein the epitope peptide comprises amino acid residues at positions 7-17, amino acid residues at positions 5-23, amino acid residues at positions 5-22, amino acid residues at positions 5-21, amino acid residues at positions 4-20, amino acid residues at positions 5-20, amino acid residues at positions 4-19, amino acid residues at positions 5-19, amino acid residues at positions 6-19, amino acid residues at positions 7-19, amino acid residues at positions 5-18, or amino acid residues at positions 5-17 of the RAS G13D mutant;

   preferably, the amino acid residues at positions 7-17, amino acid residues at positions 5-23, amino acid residues at positions 5-22, amino acid residues at positions 5-21, amino acid residues at positions 4-20, amino acid residues at positions 5-20, amino acid residues at positions 4-19, amino acid residues at positions 5-19, amino acid residues at positions 6-19, amino acid residues at positions 7-19, amino acid residues at positions 5-18, and amino acid residues at positions 5-17 of the RAS G13D mutant have the sequences as set forth in SEQ ID NOs: 14-23, 26-27, respectively;
   preferably, the epitope peptide comprises the amino acid residues at positions 4-19 of the RAS G13D mutant.

6. The epitope peptide or variant thereof according to any one of claims 1-5, wherein the RAS G13D mutant has a sequence set forth in SEQ ID NO: 51.

7. The epitope peptide or variant thereof according to any one of claims 1-6, wherein the epitope peptide comprises a sequence set forth in any one of SEQ ID NOs: 14-23, 26-27; the variant comprises a sequence selected from the group consisting of the following: (i) a sequence having a substitution, deletion or addition of one or several amino acids (e.g., a substitution, deletion or addition of 1, 2, 3, 4 or 5 amino acids) as compared to the sequence set forth in any one of SEQ ID NOs: 14-23, 26-27; (ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in any one of SEQ ID NOs: 14-23, 26-27.

8. An MHC-peptide complex, which comprises the epitope peptide or variant thereof according to any one of claims 1-7, and an MHC-II molecule bound to the epitope peptide or variant thereof;

preferably, the MHC-II molecule is HLA-DQ;
preferably, the HLA-DQ comprises one selected from the group consisting of HLA-DQB1*0301, HLA-DQB1 *0303, HLA-DQB1*0319, HLA-DQB1 *0201, HLA-DQB1 *0603, HLA-DQB1 *0604, and HLA-DQB1 *0302; preferably, the HLA-DQ comprises one selected from the group consisting of HLA-DQB1*0301, HLA-DQB1 *0303, and HLA-DQB1 *0319;
preferably, the HLA-DQ comprises one selected from the group consisting of HLA-DQA1*0501, HLA-DQA1*0505, HLA-DQA1*0102, HLA-DQA1*0103, and HLA-DQA1*0301; preferably, the HLA-DQ comprises one selected from the group consisting of HLA-DQA1*0501 and HLA-DQA1 *0505;
preferably, the HLA-DQ comprises one selected from the group consisting of HLA-DQB1*0301, HLA-DQB1*0319 or HLA-DQB1*0303, and further comprises one selected from the group consisting of HLA-DQA1*0501 or DQA1*0505.

9. An isolated T cell receptor or antigen-binding fragment thereof, which is capable of specifically recognizing the epitope peptide or variant thereof according to any one of claims 1-7 or the MHC-peptide complex according to claim 8;

preferably, the T cell receptor or antigen-binding fragment thereof is capable of recognizing the epitope peptide or variant thereof presented by an MHC-II molecule;
preferably, the MHC-II molecule is HLA-DQ;
preferably, the HLA-DQ comprises one selected from the group consisting of HLA-DQB1*0301, HLA-DQB1 *0303, HLA-DQB1 *0319, HLA-DQB1 *0201, HLA-DQB1 *0603, HLA-DQB1*0604, and HLA-DQB1*0302; preferably, the HLA-DQ comprises one selected from the group consisting of HLA-DQB1*0301, HLA-DQB1 *0303, and HLA-DQB1 *0319;
preferably, the HLA-DQ comprises one selected from the group consisting of HLA-DQA1*0501, HLA-DQA1*0505, HLA-DQA1*0102, HLA-DQA1*0103, and HLA-DQA1*0301; preferably, the HLA-DQ comprises one selected from the group consisting of HLA-DQA1*0501 and HLA-DQA1 *0505;
preferably, the HLA-DQ comprises one selected from the group consisting of HLA-DQB1*0301, HLA-DQB1*0319 or HLA-DQB1*0303, and further comprises one selected from the group consisting of HLA-DQA1*0501 or DQA1*0505;
preferably, the TCR is soluble or membrane-bound;
preferably, the TCR is a full length TCR, a soluble TCR or a single-chain TCR.

10. An isolated T cell receptor (TCR) or antigen-binding fragment thereof, which is capable of specifically recognizing a RAS G13D mutant, the TCR or antigen-binding fragment thereof comprising an $\alpha$ chain variable region (V$\alpha$) and/or a $\beta$ chain variable region (V$\beta$), wherein,

(a) the V$\alpha$ comprises CDR1$\alpha$, CDR2$\alpha$ and CDR3$\alpha$, wherein the CDR3$\alpha$ has a sequence set forth in SEQ ID NO: 8 or a sequence having a substitution, deletion or addition of one or several amino acids (e.g., a substitution, deletion or addition of 1, 2 or 3 amino acids) as compared thereto;
and/or,
(b) the V$\beta$ comprises CDR1$\beta$, CDR2$\beta$ and CDR3$\beta$, wherein the CDR3$\beta$ has a sequence set forth in any one of SEQ ID NOs: 11, 54-91 or a sequence having a substitution, deletion or addition of one or several amino acids (e.g., a substitution, deletion or addition of 1, 2 or 3 amino acids) as compared thereto;

preferably, the CDR3$\alpha$ does not comprise an amino acid substitution and deletion at position corresponding to amino acid position 5 of SEQ ID NO: 8;
preferably, the CDR1$\alpha$ has a sequence set forth in SEQ ID NO: 6 or a sequence having a substitution, deletion or addition of one or several amino acids (e.g., a substitution, deletion or addition of 1, 2 or 3 amino acids) as compared thereto;
preferably, the CDR2α has a sequence set forth in SEQ ID NO: 7 or a sequence having a substitution, deletion or addition of one or several amino acids (e.g., a substitution, deletion or addition of 1, 2 or 3 amino acids) as compared thereto;
preferably, the CDR1β has a sequence set forth in SEQ ID NO: 9 or a sequence having a substitution, deletion or addition of one or several amino acids (e.g., a substitution, deletion or addition of 1, 2 or 3 amino acids) as compared thereto;
preferably, the CDR2β has a sequence set forth in SEQ ID NO: 10 or a sequence having a substitution, deletion or addition of one or several amino acids (e.g., a substitution, deletion or addition of 1, 2 or 3 amino acids) as compared thereto;
preferably, the substitution is a conservative substitution;
preferably, the TCR is soluble or membrane-bound;
preferably, the TCR is a full length TCR, a soluble TCR or a single-chain TCR.

11. The TCR or antigen-binding fragment thereof according to claim 10, wherein the CDR3β has a sequence set forth in $ASSX_1X_2X_3X_4PQH$ (SEQ ID NO: 92);

wherein, $X_1$ is selected from the group consisting of Q, A, C, D, E, G, H, I, L, M, N, S, T, V, W or Y; $X_2$ is selected from the group consisting of T, A, C, H, K, N, S, V or W; $X_3$ is selected from the group consisting of V, I, S or T; $X_4$ is selected from the group consisting of P, C, D, E, F, G, H, L, M, R, S, V or W;
preferably, $X_1$ is selected from the group consisting of Q, A, C, E, G, M, W or Y; $X_2$ is selected from the group consisting of T, H, K, N, S or V; $X_3$ is selected from the group consisting of V or T; $X_4$ is selected from the group consisting of P, C, D, E, F, M, S or W.

12. The TCR or antigen-binding fragment thereof according to claim 10 or 11, wherein the CDR3β has a sequence set forth in any one of SEQ ID NOs: 11, 54-91;
preferably, the CDR3β has a sequence set forth in any one of SEQ ID NOs: 11, 54-55, 57-58, 62, 67-68, 71-75, 79-83, 87, 89, 91.

13. The TCR or antigen-binding fragment thereof according to any one of claims 10-12, wherein,

(a) the Vα further comprises FR1α, FR2α, FR3α and FR4α, wherein:

the FR1α has a sequence set forth in SEQ ID NO: 93 or a sequence having a substitution, deletion or addition of one or several amino acids (e.g., a substitution, deletion or addition of 1, 2 or 3 amino acids) as compared thereto;
the FR2α has a sequence set forth in SEQ ID NO: 94 or a sequence having a substitution, deletion or addition of one or several amino acids (e.g., a substitution, deletion or addition of 1, 2 or 3 amino acids) as compared thereto;
the FR3α has a sequence set forth in SEQ ID NO: 95 or a sequence having a substitution, deletion or addition of one or several amino acids (e.g., a substitution, deletion or addition of 1, 2 or 3 amino acids) as compared thereto;
the FR4α has a sequence set forth in SEQ ID NO: 96 or a sequence having a substitution, deletion or addition of one or several amino acids (e.g., a substitution, deletion or addition of 1, 2 or 3 amino acids) as compared thereto;
and/or,

(b) the Vβ further comprises FR1β, FR2β, FR3β and FR4β, wherein:

the FR1β has a sequence set forth in SEQ ID NO: 97 or a sequence having a substitution, deletion or addition of one or several amino acids (e.g., a substitution, deletion or addition of 1, 2 or 3 amino acids) as compared thereto;
the FR2β has a sequence set forth in SEQ ID NO: 98 or a sequence having a substitution, deletion or addition of one or several amino acids (e.g., a substitution, deletion or addition of 1, 2 or 3 amino acids) as compared thereto;
the FR3β has a sequence set forth in SEQ ID NO: 99 or a sequence having a substitution, deletion or addition of one or several amino acids (e.g., a substitution, deletion or addition of 1, 2 or 3 amino acids) as compared thereto;

the FR4β has a sequence set forth in SEQ ID NO: 100 or a sequence having a substitution, deletion or addition of one or several amino acids (e.g., a substitution, deletion or addition of 1, 2 or 3 amino acids) as compared thereto;
preferably, the substitution is a conservative substitution.

14. The TCR or antigen-binding fragment thereof according to any one of claims 10-13, wherein the Vα of the TCR or antigen-binding fragment thereof comprises a sequence set forth in SEQ ID NO: 4 or variant thereof, wherein the variant is selected from the group consisting of the following amino acid sequences:

(i) a sequence having a substitution, deletion or addition of one or several amino acids (e.g., a substitution, deletion or addition of 1, 2, 3, 4 or 5 amino acids) as compared to the sequence set forth in SEQ ID NO: 4; or
(ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in SEQ ID NO: 4;
preferably, the substitution described in (i) is a conservative substitution;
preferably, the variant does not comprise an amino acid substitution and deletion at position 97, in which the amino acid position is determined according to the IMGT TCR numbering system.

15. The TCR or antigen-binding fragment thereof according to any one of claims 10-14, wherein the Vβ of the TCR or antigen-binding fragment thereof comprises a sequence set forth in SEQ ID NO: 5 or variant thereof, wherein the variant is selected from the group consisting of the following amino acid sequences:

(i) a sequence having a substitution, deletion or addition of one or several amino acids (e.g., a substitution, deletion or addition of 1, 2, 3, 4 or 5 amino acids) as compared to the sequence set forth in SEQ ID NO: 5; or
(ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in SEQ ID NO: 5;
preferably, the substitution described in (i) is a conservative substitution;
preferably, the variant comprises one or several (e.g., 1, 2, 3 or 4) amino acid substitutions selected from the group consisting of the following, and the amino acid positions are determined according to the IMGT TCR numbering system: (1) substitution of amino acid at position 95 with A, C, D, E, G, H, I, L, M, N, S, T, V, W or Y; (2) substitution of amino acid at position 96 with A, C, H, K, N, S, V or W; (3) substitution of amino acid at position 97 with I, S or T; (4) substitution of amino acid at position 98 with C, D, E, F, G, H, L, M, R, S, V or W;
preferably, the variant comprises one or several (e.g., 1, 2, 3 or 4) amino acid substitutions selected from the group consisting of the following, and the amino acid positions are determined according to the IMGT TCR numbering system: (1) substitution of amino acid at position 95 with A, C, E, G, M, W or Y; (2) substitution of amino acid at position 96 with H, K, N, S or V; (3) substitution of amino acid at position 97 with T; (4) substitution of amino acid at position 98 with C, D, E, F, M, S or W.

16. The TCR or antigen-binding fragment thereof according to any one of claims 10-15, wherein the TCR or antigen-binding fragment thereof is capable of specifically recognizing the epitope peptide or variant thereof according to any one of claims 1-7 or the MHC-peptide complex according to claim 8;

preferably, the TCR or antigen-binding fragment thereof is capable of recognizing the epitope peptide or variant thereof presented by a MHC-II molecule;
preferably, the MHC-II molecule is HLA-DQ;
preferably, the HLA-DQ comprises one selected from the group consisting of HLA-DQB1*0301, HLA-DQB1*0303, HLA-DQB1*0319, HLA-DQB1*0201, HLA-DQB1*0603, HLA-DQB1*0604, HLA-DQB1*0302; preferably, the HLA-DQ comprises one selected from the group consisting of HLA-DQB1*0301, HLA-DQB1 *0303, and HLA-DQB1*0319;
preferably, the HLA-DQ comprises one selected from the group consisting of HLA-DQA1*0501, HLA-DQA1*0505, HLA-DQA1*0102, HLA-DQA1*0103, HLA-DQA1*0301; preferably, the HLA-DQ comprises one selected from the group consisting of HLA-DQA1*0501 and HLA-DQA1 *0505;
preferably, the HLA-DQ comprises one selected from the group consisting of HLA-DQB1*0301, HLA-DQB1*0319 or HLA-DQB1*0303, and further comprises one selected from the group consisting of HLA-DQA1*0501 or DQA1*0505;
preferably, a T cell expressing on its surface the TCR or antigen-binding fragment thereof is activated under co-cultivation with a second cell (e.g., APC) that displays the epitope peptide or variant thereof according to any one of claims 1-7.

17. A conjugate, which comprises the TCR or antigen-binding fragment thereof according to any one of claims 9-16 and an effector moiety conjugated thereto;

preferably, the effector moiety is selected from the group consisting of a therapeutic moiety, an immunoglobulin constant region (e.g. a human immunoglobulin constant region), or a detectable label;
preferably, the therapeutic moiety is selected from the group consisting of an immunopotentiator or a cytotoxic agent;
preferably, the immunopotentiator is selected from the group consisting of an immunostimulatory antibody (e.g., anti-CD3 antibody, anti-CD28 antibody, anti-CD40L (CD154) antibody, anti-41BB (CD137) antibody, anti-OX40 antibody, anti-GITR antibody or any combination thereof), or an immunostimulatory cytokine (e.g., IL-2, IL-3, IL-12, IL-15, IL-18, IFN-γ, IL-10, TGF-β, GM-CSF, or any combination thereof);
preferably, the cytotoxic agent is selected from the group consisting of an alkylating agent, a microtubule inhibitor, an anticancer antibiotic or an antimetabolite;
preferably, the TCR or antigen-binding fragment thereof is soluble.

18. A fusion protein, which comprises the TCR or antigen-binding fragment thereof according to any one of claims 9-16 and an additional peptide or protein;

preferably, the additional peptide or protein is selected from the group consisting of a therapeutic peptide or protein, an immunoglobulin constant region (e.g. a human immunoglobulin constant region), a detectable protein marker or a protein tag;
preferably, the therapeutic peptide or protein is selected from the group consisting of an immunostimulatory antibody (e.g., anti-CD3 antibody, anti-CD28 antibody, anti-CD40L (CD154) antibody, anti-41BB (CD137) antibody, anti-OX40 antibody, anti-GITR antibody or any combination thereof), an immunostimulatory cytokine (e.g., IL-2, IL-3, IL-12, IL-15, IL-18, IFN-γ, IL-10, TGF-β, GM-CSF, or any combination thereof), or a peptide or protein (e.g., thymidine kinase TK (TK/GCV), TRAIL, or FasL) that is toxic to a cell, capable of inhibiting cell proliferation, or capable of inducing cell apoptosis;
preferably, the TCR or antigen-binding fragment thereof is soluble.

19. An isolated nucleic acid molecule, which comprises a nucleotide sequence encoding the epitope peptide or variant thereof according to any one of claims 1-7, or comprises a nucleotide sequence encoding the TCR or antigen-binding fragment thereof according to any one of claims 9-16 or α-chain variable region and/or β-chain variable region thereof, or comprises a nucleotide sequence encoding the fusion protein according to claim 18.

20. A vector, which comprises the isolated nucleic acid molecule according to claim 19;

preferably, the vector comprises a nucleotide sequence encoding the epitope peptide or variant thereof according to any one of claims 1-7;
preferably, the vector comprises a nucleotide sequence encoding the TCR or antigen-binding fragment thereof according to any one of claims 9-16 or α-chain variable region and/or β-chain variable region thereof;
preferably, the vector comprises a nucleotide sequence encoding the fusion protein according to claim 18;
preferably, the vector is a viral vector, such as a lentiviral vector, a retroviral vector, an adenoviral vector, an adeno-associated viral vector or a baculoviral vector.

21. A host cell, which comprises the isolated nucleic acid molecule according to claim 19, or the vector according to claim 20;

preferably, the host cell comprises a nucleotide sequence encoding the epitope peptide or variant thereof according to any one of claims 1-7;
preferably, the host cell comprises a nucleotide sequence encoding the TCR or antigen-binding fragment thereof according to any one of claims 9-16 or α-chain variable region and/or β-chain variable region thereof;
preferably, the host cell comprises a nucleotide sequence encoding the fusion protein according to claim 18;
preferably, the host cells comprise *Escherichia coli,* yeast, insect cell, or mammalian cell.

22. A method for preparing the epitope peptide or variant thereof according to any one of claims 1-7, or the TCR or antigen-binding fragment thereof according to any one of claims 9-16, or the fusion protein according to claim 18, which comprises culturing the host cell according to claim 21 under conditions that allow protein expression, and recovering the epitope peptide or variant thereof, or the TCR or antigen-binding thereof, or the fusion protein, from a culture of the cultured host cell.

23. An engineered antigen-presenting cell (APC), which presents on its surface the epitope peptide or variant thereof according to any one of claims 1-7;

   preferably, the APC is selected from the group consisting of dendritic cell, monocyte, macrophage, lymphoblastoid cell (LCL), or any combination thereof;
   preferably, the APC is positive for HLA-DQB1*0301, positive for HLA-DQB1*0303, positive for HLA-DQB1*0319, positive for HLA-DQB1*0201, positive for HLA-DQB1*0603, positive for HLA-DQB 1*0604 or positive for HLA-DQB 1*0302; preferably, the APC is positive for HLA-DQB1*0301, positive for HLA-DQB1*0303 or positive for HLA-DQB1*0319;
   preferably, the APC is positive for HLA-DQA1*0501, positive for HLA-DQA1*0505, positive for HLA-DQA1*0102, positive for HLA-DQA1*0103 or positive for HLA-DQA1*0301; preferably, the APC is positive for HLA-DQA1*0501 or positive for HLA-DQA1*0505;
   preferably, the APC is positive for HLA-DQB1*0301, positive for HLA-DQB1*0319 or positive for HLA-DQB1*0303, and is further positive for HLA-DQA1*0501 or positive for HLA-DQA1*0505.

24. A method for preparing the engineered APC according to claim 23, comprising: (1) providing an APC from a subject; (2) contacting the APC with the epitope peptide or variant thereof according to any one of claims 1-7 in vitro, or introducing an expression vector comprising a nucleotide sequence encoding the epitope peptide or variant thereof according to any one of claims 1-7 into the APC, to obtain an APC presenting on its surface the epitope peptide or variant thereof.

25. An engineered immune cell, which expresses on its surface the TCR or antigen-binding fragment thereof according to any one of claims 9-16;

   preferably, the engineered immune cell comprises a nucleotide sequence encoding the TCR or antigen-binding fragment thereof according to any one of claims 9-16;
   preferably, the immune cell is a lymphocyte;
   preferably, the immune cell is selected from the group consisting of a T cell (e.g., αβT cell, γδT cell or iPSC-derived T cell), a tumor infiltrating lymphocyte (TIL), a natural killer (NK) cell, a natural killer T (NKT) cell, or any combination thereof.

26. A method for preparing the engineered immune cell according to claim 25, comprising: (1) providing an immune cell from a subject; (2) introducing the isolated nucleic acid molecule according to claim 19 or the vector according to claim 20 into the immune cell of step (1), the nucleic acid molecule or vector comprising a nucleotide sequence encoding the TCR or antigen-binding fragment thereof according to any one of claims 9-16, so as to obtain an immune cell expressing the TCR or antigen-binding fragments thereof;

   preferably, in step (1), the immune cell is subjected to pretreatment; the pretreatment comprises sorting, activation and/or proliferation of the immune cell;
   preferably, the pretreatment comprises contacting the immune cell with one or more selected from the group consisting of anti-CD3 antibody, anti-CD28 antibody, IL-2 and IL-15, thereby stimulating the immune cell and inducing its proliferation, and thereby producing a pretreated immune cell.

27. A pharmaceutical composition, which comprises the epitope peptide or variant thereof according to any one of claims 1-7, the MHC-peptide complex according to claim 8, a nucleic acid molecule or vector or host cell comprising a nucleotide sequence encoding the epitope peptide or variant thereof, or the engineered antigen-presenting cell (APC) according to claim 23; and a pharmaceutically acceptable carrier and/or excipient;

   preferably, the pharmaceutical composition is a tumor vaccine;
   preferably, the pharmaceutical composition comprises an adjuvant;
   preferably, the pharmaceutical composition further comprises an additional therapeutic agent, such as an antineoplastic agent or an immunopotentiator;
   preferably, the antineoplastic agent is selected from the group consisting of an alkylating agent, a mitotic inhibitor, an antitumor antibiotic, an antimetabolite, a topoisomerase inhibitor, a tyrosine kinase inhibitor, a radionuclide agent, a radiosensitizer, an anti-angiogenic agent, a cytokine, an immune checkpoint inhibitor (e.g., PD-1 antibody, PD-L1 antibody, CTLA-4 antibody, LAG-3 antibody, or TIM3 antibody);

preferably, the immunopotentiator is selected from the group consisting of an immunostimulatory antibody (e.g., anti-CD3 antibody, anti-CD28 antibody, anti-CD40L (CD154) antibody, anti-41BB (CD137) antibody, anti-OX40 antibody, anti-GITR antibody, or any combination thereof) or an immunostimulatory cytokine (e.g., IL-2, IL-3, IL-12, IL-15, IL-18, IFN-γ, IL-10, TGF-β, GM-CSF, or any combination thereof).

28. A pharmaceutical composition, which comprises the TCR or antigen-binding fragment thereof according to any one of claims 9-16, the conjugate according to claim 17, the fusion protein according to claim 18, a nucleic acid molecule or vector or host cell comprising a nucleotide sequence encoding the TCR or antigen-binding fragment or fusion protein thereof, or the engineered immune cell according to claim 25; and a pharmaceutically acceptable carrier and/or excipient;

preferably, the pharmaceutical composition further comprises an additional therapeutic agent, such as an antineoplastic agent or an immunopotentiator;
preferably, the antineoplastic agent is selected from the group consisting of an alkylating agent, a mitotic inhibitor, an antitumor antibiotic, an antimetabolite, a topoisomerase inhibitor, a tyrosine kinase inhibitor, a radionuclide agent, a radiosensitizer, an anti-angiogenic agent, a cytokine, an immune checkpoint inhibitor (e.g., PD-1 antibody, PD-L1 antibody, CTLA-4 antibody, LAG-3 antibody, or TIM3 antibody);
preferably, the immunopotentiator is selected from the group consisting of an immunostimulatory antibody (e.g., anti-CD3 antibody, anti-CD28 antibody, anti-CD40L (CD154) antibody, anti-41BB (CD137) antibody, anti-OX40 antibody, anti-GITR antibody, or any combination thereof) or an immunostimulatory cytokine (e.g., IL-2, IL-3, IL-12, IL-15, IL-18, IFN-γ, IL-10, TGF-β, GM-CSF, or any combination thereof).

29. Use of the epitope peptide or variant thereof according to any one of claims 1-7, the MHC-peptide complex according to claim 8, a nucleic acid molecule or vector or host cell comprising a nucleotide sequence encoding the epitope peptide or variant thereof, or the engineered antigen-presenting cell (APC) according to claim 23, or the pharmaceutical composition according to claim 27, in the manufacture of medicament, wherein the medicament is used for inducing an immune response against a tumor with a RAS G13D mutation in a subject, and/or preventing or treating a tumor with a RAS G13D mutation in a subject;

preferably, the tumor with RAS G13D mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia;
preferably, the subject is a human;
preferably, the subject is positive for HLA-DQB1*0301, positive for HLA-DQB1*0303, positive for HLA-DQB1*0319, positive for HLA-DQB1*0201, positive for HLA-DQB1*0603, positive for HLA-DQB1*0604 or positive for HLA-DQB1*0302; preferably, the subject is positive for HLA-DQB 1 *0301, positive for HLA-DQB 1 *0303 or positive for HLA-DQB 1 *0319;
preferably, the subject is positive for HLA-DQA1*0501, positive for HLA-DQA1*0505, positive for HLA-DQA1*0102, positive for HLA-DQA1*0103 or positive for HLA-DQA1*0301; preferably, the subject is positive for HLA-DQA1*0501 or positive for HLA-DQA1*0505;
preferably, the subject is positive for HLA-DQB 1*0301, positive for HLA-DQB1 *0319 or positive for HLA-DQB1*0303, and is further positive for HLA-DQA1*0501 or positive for HLA-DQA1*0505;
preferably, the epitope peptide or variant thereof, MHC-peptide complex, nucleic acid molecule or vector or host cell, engineered antigen presenting cell (APC), or pharmaceutical composition is administered in combination with an additional therapeutic agent, for example, simultaneously, separately or sequentially; preferably, the additional therapeutic agent is an immunopotentiator or antineoplastic agent.

30. Use of the TCR or antigen-binding fragment thereof according to any one of claims 9-16, the conjugate according to claim 17, the fusion protein according to claim 18, a nucleic acid molecule or vector or host cell comprising a nucleotide sequence encoding the TCR or antigen-binding fragment or fusion protein thereof, or the engineered immune cells according to claim 25, or the pharmaceutical composition according to claim 27, in the manufacture of a medicament, wherein the medicament is used for inducing an immune response against a tumor with a RAS G13D mutation in a subject, and/or preventing or treating a tumor with a RAS G13D mutation in a subject; wherein the nucleic acid molecule, vector or host cell comprises a nucleotide sequence encoding the TCR or antigen-binding fragment or fusion protein thereof;

preferably, the tumor with RAS G13D mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia;

preferably, the subject is a human;

preferably, the subject is positive for HLA-DQB1*0301, positive for HLA-DQB1*0303, positive for HLA-DQB1*0319, positive for HLA-DQB1*0201, positive for HLA-DQB1*0603, positive for HLA-DQB1*0604 or positive for HLA-DQB1*0302; preferably, the subject is positive for HLA-DQB 1 *0301, positive for HLA-DQB 1 *0303 or positive for HLA-DQB 1 *0319;

preferably, the subject is positive for HLA-DQA1*0501, positive for HLA-DQA1*0505, positive for HLA-DQA1*0102, positive for HLA-DQA1*0103 or positive for HLA-DQA1*0301; preferably, the subject is positive for HLA-DQA1*0501 or positive for HLA-DQA1*0505;

preferably, the subject is positive for HLA-DQB 1*0301, positive for HLA-DQB 1 *0319 or positive for HLA-DQB1*0303, and is further positive for HLA-DQA1*0501 or positive for HLA-DQA1*0505;

preferably, the TCR or antigen-binding fragment thereof, conjugate, fusion protein, nucleic acid molecule or vector or host cell, engineered immune cell, or pharmaceutical composition is administered in combination with an additional therapeutic agent, for example, simultaneously, separately or sequentially; preferably, the additional therapeutic agent is an immunopotentiator or antineoplastic agent.

31. A method for inducing an immune response in a subject against a tumor with a RAS G13D mutation, and/or preventing or treating a tumor with a RAS G13D mutation in a subject, the method comprising administrating to the subject in need thereof an effective amount of the epitope peptide or variant thereof according to any one of claims 1-7, the MHC-peptide complex according to claim 8, a nucleic acid molecule or vector or host cell comprising a nucleotide sequence encoding the epitope peptide or variant thereof, or the engineered antigen-presenting cell (APC) according to claim 23, or the pharmaceutical composition according to claim 27;

preferably, the tumor with RAS G13D mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia;

preferably, the subject is a human;

preferably, the subject is positive for HLA-DQB1*0301, positive for HLA-DQB1*0303, positive for HLA-DQB1*0319, positive for HLA-DQB1*0201, positive for HLA-DQB1*0603, positive for HLA-DQB1*0604 or positive for HLA-DQB1*0302; preferably, the subject is positive for HLA-DQB 1 *0301, positive for HLA-DQB 1 *0303 or positive for HLA-DQB 1 *0319;

preferably, the subject is positive for HLA-DQA1*0501, positive for HLA-DQA1*0505, positive for HLA-DQA1*0102, positive for HLA-DQA1*0103 or positive for HLA-DQA1*0301; preferably, the subject is positive for HLA-DQA1*0501 or positive for HLA-DQA1*0505;

preferably, the subject is positive for HLA-DQB 1*0301, positive for HLA-DQB 1 *0319 or positive for HLA-DQB1*0303, and is further positive for HLA-DQA1*0501 or positive for HLA-DQA1*0505;

preferably, the method further comprises administering to the subject an additional therapeutic agent, such as an immunopotentiator or an antineoplastic agent.

32. A method for inducing an immune response against a tumor with a RAS G13D mutation in a subject, and/or preventing or treating a tumor with a RAS G13D mutation in a subject, the method comprising administration to the subject in need thereof an effective amount of the TCR or antigen-binding fragment thereof according to any one of claims 9-16, the conjugate according to claim 17, the fusion protein according to claim 18, a nucleic acid molecule or vector or host cell comprising a nucleotide sequence encoding the TCR or antigen-binding fragment or fusion protein thereof, or the engineered immune cell according to claim 25, or the pharmaceutical composition according to claim 28;

preferably, the tumor with RAS G13D mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia;

preferably, the subject is a human;

preferably, the subject is positive for HLA-DQB1*0301, positive for HLA-DQB1*0303, positive for HLA-DQB1*0319, positive for HLA-DQB1*0201, positive for HLA-DQB1*0603, positive for HLA-DQB1*0604 or positive for HLA-DQB1*0302; preferably, the subject is positive for HLA-DQB 1 *0301, positive for HLA-DQB 1 *0303 or positive for HLA-DQB 1 *0319;

preferably, the subject is positive for HLA-DQA1*0501, positive for HLA-DQA1*0505, positive for HLA-DQA1*0102, positive for HLA-DQA1*0103 or positive for HLA-DQA1*0301; preferably, the subject is positive for HLA-DQA1*0501 or positive for HLA-DQA1*0505;

preferably, the subject is positive for HLA-DQB1*0301, positive for HLA-DQB 1 *0319 or positive for HLA-DQB1*0303, and is further positive for HLA-DQA1*0501 or positive for HLA-DQA1*0505;

preferably, the method further comprises administering to the subject an additional therapeutic agent, such as an immunopotentiator or an antineoplastic agent;

preferably, the method comprises: (1) providing an immune cell required by the subject; (2) introducing a nucleotide sequence encoding the TCR or antigen-binding fragment thereof according to any one of claims 9-16 into the immune cell of step (1) to obtain an immune cell expressing on its surface the TCR or antigen-binding fragment thereof; (3) administering the immune cell obtained in step (2) to the subject;

preferably, the immune cell is a lymphocyte;

preferably, the immune cell is selected from the group consisting of a T cell (e.g., $\alpha\beta$T cell, $\gamma\delta$T cell or iPSC-derived T cell), a tumor infiltrating lymphocyte (TIL), a natural killer (NK) cell, a natural killer T (NKT) cell, or any combination thereof.

Fig. 1

Fig. 2

Fig. 3

Fig. 4

Fig. 5

Fig. 6

Fig. 7

Fig. 8

Fig. 9A

Fig. 9B

## INTERNATIONAL SEARCH REPORT

International application No.

**PCT/CN2022/075005**

### A. CLASSIFICATION OF SUBJECT MATTER

C07K 14/82(2006.01)i; C07K 14/725(2006.01)i; C12N 5/10(2006.01)i; C12N 15/12(2006.01)i; A61K 38/17(2006.01)i; A61K 39/00(2006.01)i; A61P 35/00(2006.01)i

According to International Patent Classification (IPC) or to both national classification and IPC

### B. FIELDS SEARCHED

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, CNTXT, VCN, WPABS, WPABSC, DWPI, VEN, ENTXT, OETXT, CNKI, 万方, WANFANG, ISI_Web of Science, PubMed, Elsevier Science, 百度学术, BAIDU SCHOLAR, 中国专利生物序列检索系统, Chinese Patent Biological Sequence Retrieval System, STN, EMBL, GenBank; KRAS, RAS, K-RAS, G13D, 表位, 突变, MHC-II, MHCII, HLA, HLA-II, HLA-DQ, HLA-DP, HLA-DR, T细胞受体, TCR, APC, 抗原提呈细胞, 抗原递呈细胞, 抗原呈递细胞, T细胞, 淋巴细胞, 缀合物, MHC-肽复合物, 融合蛋白, 药物组合物, 肿瘤, 截短, mutant, mutation, truncation, T cell receptor, antigen presenting cell, T cell, lymphocyte, conjugate, MHC-peptide complex, fusion protein, pharmaceutical composition, tumor, cancer; 序列: SEQ ID NOs: 6-48, 51-100

### C. DOCUMENTS CONSIDERED TO BE RELEVANT

| Category* | Citation of document, with indication, where appropriate, of the relevant passages | Relevant to claim No. |
|---|---|---|
| X | CN 107922472 A (TARGOVAX AS) 17 April 2018 (2018-04-17)<br>see claims 1-25, and description, paragraphs 153-279 | 1-30 |
| X | WO 2020145222 A1 (KANAGAWA PREFECTURAL HOSPITAL ORGANIZATION et al.) 16 July 2020 (2020-07-16)<br>see claims 1-18, and description, paragraphs 17-43 | 1-30 |
| X | CN 105980403 A (TARGOVAX AS) 28 September 2016 (2016-09-28)<br>see claims 1-19 | 1-30 |
| X | CN 112118847 A (ELICIO THERAPEUTICS INC) 22 December 2020 (2020-12-22)<br>see claims 1-22 | 1-22, 27-30 |
| Y | CN 112118847 A (ELICIO THERAPEUTICS INC) 22 December 2020 (2020-12-22)<br>see claims 1-22 | 23-26 |

☑ Further documents are listed in the continuation of Box C. ☑ See patent family annex.

| | | |
|---|---|---|
| * | Special categories of cited documents: | |
| "A" | document defining the general state of the art which is not considered to be of particular relevance | "T" | later document published after the international filing date or priority date and not in conflict with the application but cited to understand the principle or theory underlying the invention |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 March 2022** | **26 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/075005** |

### C. DOCUMENTS CONSIDERED TO BE RELEVANT

| Category* | Citation of document, with indication, where appropriate, of the relevant passages | Relevant to claim No. |
|---|---|---|
| X | CN 110430894 A (MODERNATX INC.) 08 November 2019 (2019-11-08) see claims 1-189, and description, paragraphs 7-189 | 1-22, 27-30 |
| Y | CN 110430894 A (MODERNATX INC.) 08 November 2019 (2019-11-08) see claims 1-189, and description, paragraphs 7-189 | 23-26 |
| X | WO 2020123300 A2 (LILLY CO ELI) 18 June 2020 (2020-06-18) see claims 1-53 | 1-22, 27-30 |
| Y | WO 2020123300 A2 (LILLY CO ELI) 18 June 2020 (2020-06-18) see claims 1-53 | 23-26 |
| X | CN 110506107 A (ADVAXIS INC.) 26 November 2019 (2019-11-26) see claims 1-118 | 1-22, 27-30 |
| Y | CN 110506107 A (ADVAXIS INC.) 26 November 2019 (2019-11-26) see claims 1-188 | 23-26 |
| A | GJERTSEN, M. K. et al. "Cytotoxic CD4+ and CD8+ T lymphocytes, generated by mutant p21-ras (12Val) peptide vaccination of a patient, recognize 12Val-dependent nested epitopes present within the vaccine peptide and kill autologous tumour cells carrying this mutation" *Int J Cancer*, Vol. 72, No. 5, 04 September 1997 (1997-09-04), ISSN: 1097-0215, see entire document | 1-30 |

Form PCT/ISA/210 (second sheet) (January 2015)

56

**INTERNATIONAL SEARCH REPORT**

| International application No. |
| --- |
| **PCT/CN2022/075005** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
| --- |
| **PCT/CN2022/075005** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **31、32**
   because they relate to subject matter not required to be searched by this Authority, namely:

   [1] PCT Rule 39.1(iv)-a method for treatment of a human or animal body by surgery or therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/075005**

| Patent document cited in search report | | | Publication date (day/month/year) | Patent family member(s) | | | Publication date (day/month/year) |
|---|---|---|---|---|---|---|---|
| CN | 107922472 | A | 17 April 2018 | HK | 1248714 | A1 | 19 October 2018 |
| | | | | KR | 20180021072 | A | 28 February 2018 |
| | | | | RU | 2018101225 | A | 16 July 2019 |
| | | | | US | 2019374628 | A1 | 12 December 2019 |
| | | | | CA | 2989373 | A1 | 22 December 2016 |
| | | | | JP | 2018518177 | A | 12 July 2018 |
| | | | | CL | 2017003228 | A1 | 13 July 2018 |
| | | | | EP | 3310808 | A1 | 25 April 2018 |
| | | | | BR | 112017025728 | A2 | 07 August 2018 |
| | | | | WO | 2016202937 | A1 | 22 December 2016 |
| | | | | AU | 2016280770 | A1 | 04 January 2018 |
| | | | | IL | 256077 | D0 | 31 January 2018 |
| | | | | MX | 2017015927 | A | 18 April 2018 |
| WO | 2020145222 | A1 | 16 July 2020 | None | | | |
| CN | 105980403 | A | 28 September 2016 | CL | 2018000238 | A1 | 22 June 2018 |
| | | | | IL | 246007 | D0 | 31 July 2016 |
| | | | | CA | 2933126 | A1 | 18 June 2015 |
| | | | | RU | 2016127327 | A | 23 January 2018 |
| | | | | AU | 2019201937 | A1 | 11 April 2019 |
| | | | | MX | 2016007429 | A | 11 November 2016 |
| | | | | RS | 57623 | B1 | 30 November 2018 |
| | | | | BR | 112016013138 | A2 | 16 January 2018 |
| | | | | CL | 2016001405 | A1 | 24 March 2017 |
| | | | | EP | 3357505 | A1 | 08 August 2018 |
| | | | | PL | 3079715 | T3 | 30 November 2018 |
| | | | | JP | 2017502081 | A | 19 January 2017 |
| | | | | US | 2017326218 | A1 | 16 November 2017 |
| | | | | US | 2016331820 | A1 | 17 November 2016 |
| | | | | SG | 10201811172 P | A | 30 January 2019 |
| | | | | EP | 3369432 | A1 | 05 September 2018 |
| | | | | CY | 1120578 | T1 | 10 July 2019 |
| | | | | LT | 3079715 | T | 27 August 2018 |
| | | | | US | 2017189515 | A1 | 06 July 2017 |
| | | | | WO | 2015086590 | A2 | 18 June 2015 |
| | | | | HU | E039840 | T2 | 28 February 2019 |
| | | | | KR | 20160097290 | A | 17 August 2016 |
| | | | | JP | 2020023525 | A | 13 February 2020 |
| | | | | SI | 3079715 | T1 | 31 August 2018 |
| | | | | PT | 3079715 | T | 02 August 2018 |
| | | | | AU | 2014363643 | A1 | 14 July 2016 |
| | | | | HR | P20181213 | T1 | 19 October 2018 |
| | | | | US | 2018021419 | A1 | 25 January 2018 |
| | | | | CL | 2018000226 | A1 | 22 June 2018 |
| | | | | ES | 2682038 | T3 | 18 September 2018 |
| | | | | SG | 11201604644 Q | A | 28 July 2016 |
| | | | | EP | 3079715 | A2 | 19 October 2016 |
| | | | | EP | 3363458 | A2 | 22 August 2018 |
| | | | | CL | 2018000232 | A1 | 22 June 2018 |
| | | | | EP | 3363457 | A1 | 22 August 2018 |
| | | | | DK | 3079715 | T3 | 17 September 2018 |

Form PCT/ISA/210 (patent family annex) (January 2015)

**INTERNATIONAL SEARCH REPORT**
Information on patent family members

International application No.

**PCT/CN2022/075005**

| Patent document cited in search report | | | Publication date (day/month/year) | Patent family member(s) | | | Publication date (day/month/year) |
|---|---|---|---|---|---|---|---|
| CN | 112118847 | A | 22 December 2020 | MA | 52435 | A | 06 January 2021 |
| | | | | SG | 11202008433 Q | A | 29 September 2020 |
| | | | | US | 2021060149 | A1 | 04 March 2021 |
| | | | | CA | 3092679 | A1 | 06 September 2019 |
| | | | | KR | 20200141994 | A | 21 December 2020 |
| | | | | JP | 2021517471 | A | 26 July 2021 |
| | | | | EP | 3758713 | A1 | 06 January 2021 |
| | | | | IL | 277100 | D0 | 29 October 2020 |
| | | | | AU | 2019226586 | A1 | 15 October 2020 |
| | | | | BR | 112020017645 | A2 | 29 December 2020 |
| | | | | WO | 2019169332 | A1 | 06 September 2019 |
| CN | 110430894 | A | 08 November 2019 | BR | 112019015797 | A2 | 17 March 2020 |
| | | | | CA | 3051252 | A1 | 09 August 2018 |
| | | | | US | 2019351039 | A1 | 21 November 2019 |
| | | | | US | 2021128721 | A1 | 06 May 2021 |
| | | | | KR | 20190110612 | A | 30 September 2019 |
| | | | | CO | 2019009234 | A2 | 30 August 2019 |
| | | | | SG | 11201906969 P | A | 27 August 2019 |
| | | | | WO | 2018144775 | A1 | 09 August 2018 |
| | | | | JP | 2020514321 | A | 21 May 2020 |
| | | | | IL | 268361 | D0 | 26 September 2019 |
| | | | | MX | 2019009070 | A | 30 October 2019 |
| | | | | AU | 2018214556 | A1 | 15 August 2019 |
| | | | | US | 2019175727 | A1 | 13 June 2019 |
| | | | | CL | 2019002134 | A1 | 22 November 2019 |
| WO | 2020123300 | A2 | 18 June 2020 | EP | 3893909 | A2 | 20 October 2021 |
| | | | | JP | 2022511977 | A | 01 February 2022 |
| | | | | CA | 3122068 | A1 | 18 June 2020 |
| | | | | TW | 202039534 | A | 01 November 2020 |
| | | | | CN | 113631177 | A | 09 November 2021 |
| | | | | AU | 2019395319 | A1 | 24 June 2021 |
| | | | | IL | 283795 | D0 | 29 July 2021 |
| CN | 110506107 | A | 26 November 2019 | CA | 3035591 | A1 | 07 June 2018 |
| | | | | SG | 11201901979 S | A | 29 April 2019 |
| | | | | US | 2019322714 | A1 | 24 October 2019 |
| | | | | MX | 2019005685 | A | 04 September 2019 |
| | | | | KR | 20190082850 | A | 10 July 2019 |
| | | | | IL | 265156 | D0 | 30 May 2019 |
| | | | | JP | 2020513737 | A | 21 May 2020 |
| | | | | WO | 2018102584 | A1 | 07 June 2018 |
| | | | | AU | 2017367642 | A1 | 30 May 2019 |
| | | | | EP | 3548623 | A1 | 09 October 2019 |
| | | | | SG | 10202105561 P | A | 29 July 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003020763 A
- WO 2004033685 A
- WO 2011044186 A
- US 8361794 B
- US 8906383 B

### Non-patent literature cited in the description

- *Front Oncol.,* 2014, vol. 4, 378
- **LEFRANC ; LEFRANC.** T Cell Receptor Facts Book. Academic Press, 2001
- **LEFRANC.** *The Immunologist,* 1999, vol. 7, 132-136
- **LEFRANC et al.** *Nucleic Acids Res,* 1999, vol. 27, 209-212
- **LEFRANC.** T Cell Receptor Facts Book. Academic Press, 2001
- **LEFRANC et al.** *Dev Comp Immunol,* 2003, vol. 27 (1), 55-77
- **KABAT et al.** *Sequences of proteins of immunological interest,* 1991
- **MALMQVIST M.** *Nature,* 1993, vol. 361, 186-187
- **DAVIES et al.** *Annual Rev Biochem,* 1990, vol. 59, 439-473
- **NEEDLEMAN et al.** *J. Mol. Biol.,* 1970, vol. 48, 443-453
- **E. MEYERS ; W. MILLER.** *Comput. Appl Biosci.,* 1988, vol. 4, 11-17
- **NEEDLEMAN ; WUNSCH.** *J Mol Biol.,* 1970, vol. 48, 444-453
- **BRUMMELL et al.** *Biochem.,* 1993, vol. 32, 1180-1187
- **KOBAYASHI et al.** *Protein Eng.,* 1999, vol. 12 (10), 879-884
- **BURKS et al.** *Proc. Natl Acad. Set USA,* 1997, vol. 94, 412-417
- Immunology-A Synthesis. Sinauer Associates, 1991
- Remington's Pharmaceutical Sciences. Mack Publishing Company, 1995
- **J. SAMBROOK et al.** Molecular Cloning: A Laboratory Manual. Cold Spring Harbor Laboratory Press, 1989
- **F. M. AUSUBEL et al.** Short protocols in Molecular Biology. John Wiley & Sons, Inc, 1995